(19) Europäisches Patentamt
European Patent Office
Office européen des brevets

(11) Publication number: 0 167 935 B1

# (12) EUROPEAN PATENT SPECIFICATION

(45) Date of publication of patent specification: 30.08.89

(51) Int. Cl.[4]: C 07 H 15/252, C 12 P 19/56, A 61 K 31/70 // (C12R1/465, C12P19:56)

(21) Application number: 85107940.0

(22) Date of filing: 26.06.85

## (54) Anthracycline derivatives, a process for the production thereof, a pharmaceutical composition containing the same and their use as medicaments.

(30) Priority: 03.07.84 JP 137486/84

(43) Date of publication of application: 15.01.86 Bulletin 86/03

(45) Publication of the grant of the patent: 30.08.89 Bulletin 89/35

(84) Designated Contracting States: AT BE CH DE FR GB IT LI LU NL SE

(56) References cited:
EP-A-0 022 574
EP-A-0 110 155
EP-A-0 131 181
EP-A-0 131 942
JOURNAL OF ANTIBIOTICS, vol. 36, no. 8, August 1983, pages 1080-1083. T. UCHIDA et al.: "New antitumor antibiotics, ditrisarubicins A, B and C"
CHEMICAL ABSTRACTS, vol. 101, no. 23, December 3, 1984, page 489, ref. no. 209080z, Columbus, Ohio, US & JP A 5982395 (MICROBIOCHEMICAL RESEARCH FOUNDATION) 12-05-1984

(73) Proprietor: MICROBIAL CHEMISTRY RESEARCH FOUNDATION
14-23, Kamiosaki 3 Chome Shinagawa-ku
Tokyo 141 (JP)

(72) Inventor: Umezawa, Hamao, Prof.
23, Toyotama-kita 4-chome
Nerima-ku Tokyo (JP)
Inventor: Takeuchi, Tomio
1-11, Higashigotanda 5-chome
Shinagawa-ku Tokyo (JP)
Inventor: Hamada, Masa
26, Naito-cho 1-chome
Shinjuku-ku Tokyo (JP)
Inventor: Naganawa, Hiroshi
17, Denenchofu-honcho 3-chome
Ohta-ku Tokyo (JP)
Inventor: Sawa, Tsutomu
6-7, Ryosei 4-chome
Ayase-city Kanagawa Prefecture (JP)
Inventor: Uchida, Takeshi
1-102, 22-18, Kishiya 4-chome Tsurumi-ku
Yokohama-city Kanagawa Prefecture (JP)
Inventor: Imoto, Masaya
18-14-101, Tokiwa 7-chome
Urawa-city Saitama Prefecture (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 167 935 B1

(56) References cited:
JOURNAL OF ANTIBIOTICS, vol. 38, no. 6, June 1985, pages 795-798, Japan antibiotics, Res. Ass., Tokyo, JP. T. UCHIDA et al.:; "New anthracycline antibiotics: serirubicin and 1-hydroxyseriribucin"

(74) Representative: Becker, Heinrich Karl Engelbert, Dr. et al
HOECHST AKTIENGESELLSCHAFT Central Patent Department P.O. Box 80 03 20
D-6230 Frankfurt am Main 80 (DE)

## Description

The present invention relates to novel anthracycline compounds, a process for producing the same, and use of the same.

Anthracycline compounds are important as cancer-control anti-biotics and several types of the compounds have already been proposed.

The physiological activities of chemical substances largely depend on their chemical structures and anthracycline compounds are not an exception. It could be said that there exists a constant demand for developing new anthracycline compounds having unconventional types of, or substituents on, the aglycone and sugar portions.

The invention provides, therefore, anthracycline compounds of the formula (I) shown below or acid addition salts thereof:

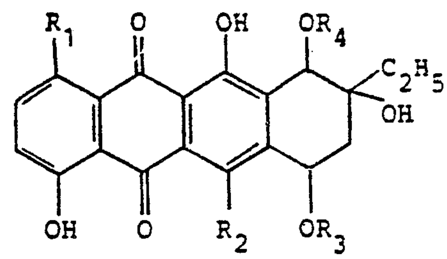

(I)

wherein $R_1$, $R_2$, $R_3$ and $R_4$ represent any one of the combinations (1) to (8) and (11) listed in the following table:

| No. | Compound | | $R_1$ | $R_2$ | $R_3$ | $R_4$ |
|---|---|---|---|---|---|---|
| (1) | ditrisarubicin | D | H | OH | (I) | (V) |
| (2) | ditrisarubicin | E | H | OH | (II) | (II) |
| (3) | ditrisarubicin | F | H | OH | (I) | (VI) |
| (4) | ditrisarubicin | G | H | OH | (I) | (III) |
| (5) | ditrisarubicin | CR | H | OH | (IV) | (I) |
| (6) | ditrisarubicin | GR | H | OH | (III) | (I) |
| (7) | ditrisarubicin | G/de-Ro | H | OH | (I) | (VII) |
| (8) | ditrisarubicin | GR/de-Ro | H | OH | (VII) | (I) |
| (9) | ditrisarubicin (not claimed) | B/10de-dF-CB | H | OH | (I) | (VIII) |
| (10) | ditrisarubicin (not claimed) | B/7de-dF-CB | H | OH | (VIII) | (I) |
| (11) | 1-hydroxyserirubicin | | OH | H | (I) | (I) |

wherein the symbols for $R_3$ and $R_4$ represent the following sugar chains:

(I) RN-dF-CB

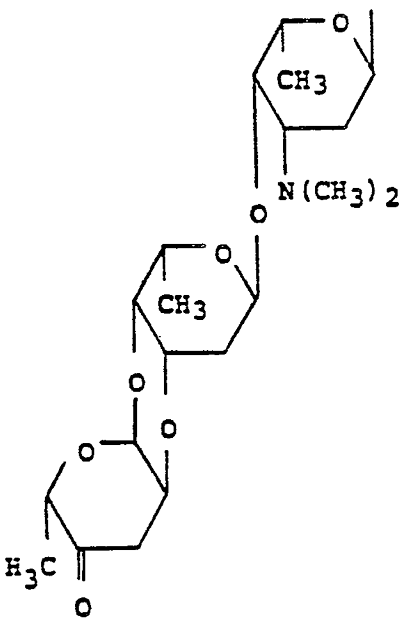

(II) RN-dF-CA

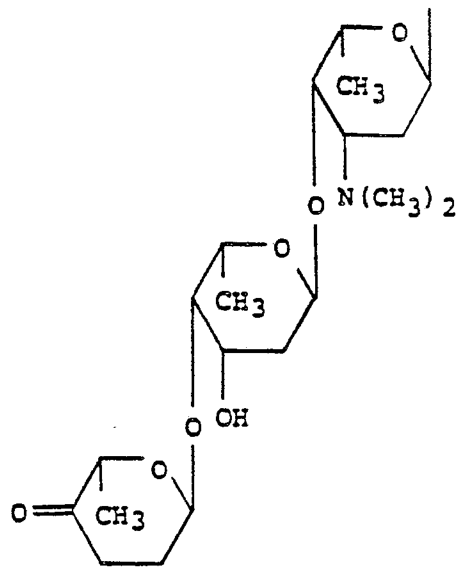

(III) RN-dF-Ro

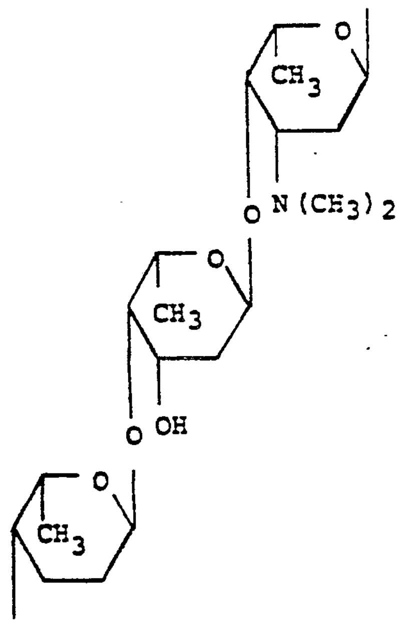

(IV) RN-Ro-Ac

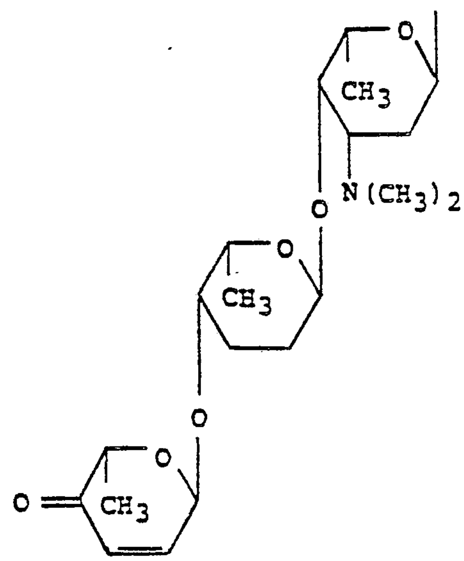

(V) RN-Ro-CA

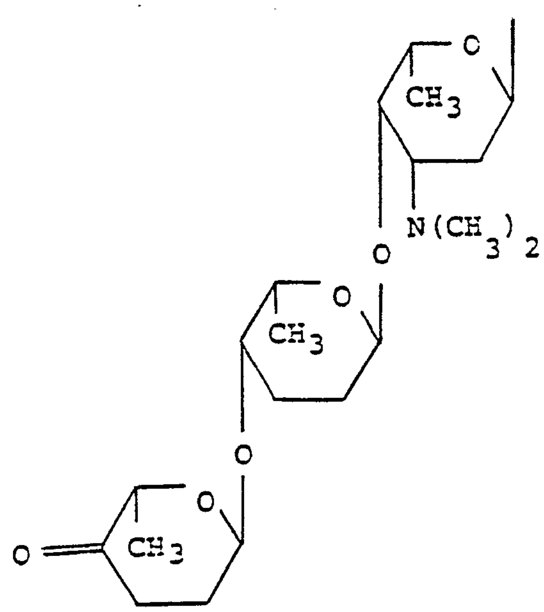

(VI) RN-Ro-Ro

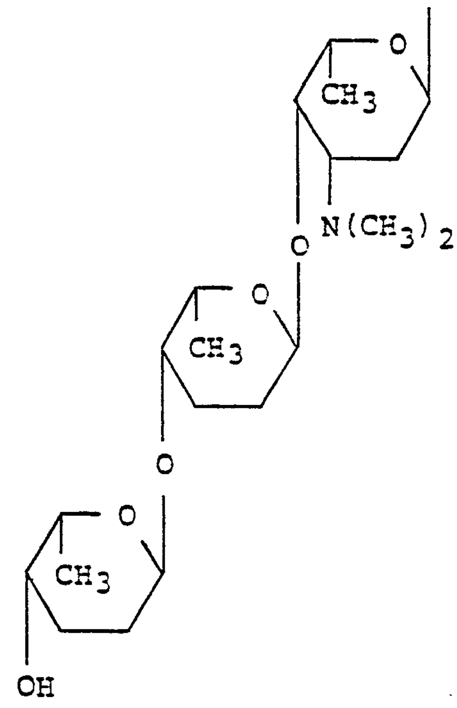

(VII) RN-dF

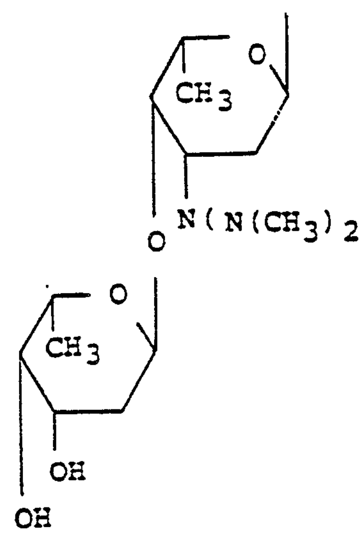

(VIII) RN

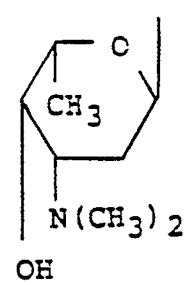

(RN: rhodosamine, dF: 2-deoxyfucose, CB: cinerulose B, CA: cinerulose A, Ac: aculose, Ro: rhodinose).

Similar compounds having the aglycone of formula (I) where $R_1$ = H and $R_2$ = OH are known from EP—A—110155 and from EP—A—131181 and EP—A—131942 (State-of-the-art under Art. 54(3) and (4)), EP—A—110155 describes compounds of formula (I) where $R_3$ represents sugar chain (I) and $R_4$ represents sugar chains (I), (II) or (IV). EP—A—131181 describes compounds where $R_3$ and $R_4$ represent sugar chains (I), (II), (III), (IV) or (VI) in various combinations. EP—A—131942 describes compounds where $R_3$ and $R_4$ represent sugar chains (I) and (VIII) or (VIII) and (I) respectively.

The anthracycline compounds according to the present invention have the chemical structure represented by formula (I). They consist of nine types according to the types of substituents $R_1$ to $R_4$ in formula (I) and how these substituents are combined.

The anthracycline compounds of the present invention have a dimethylamino group in the sugar portion and hence are capable of forming acid addition salts. Acids with which the compounds react to form addition salts include hydrohalogenic acids such as hydrochloric acid, as well as sulfuric acid and tartaric acid.

The chemical structure of the anthracycline compounds according to the present invention were determined by the methods shown in the Experimental Examples given later in this specification.

3) Physicochemical properties

Some of the physicochemical properties of the anthrocycline compounds according to the present invention are shown below.

TABLE 1—1

| | | Ditrisarubicin D (1) | Ditrisarubicin E (2) | Ditrisarubicin F (3) | Ditrisarubicin G (4) |
|---|---|---|---|---|---|
| 1 | Appearance | red powder | red powder | red powder | red powder |
| 2 | Elemental analysis (%) | | | | |
| | (1) Found | C: 63.21 H: 6.89 N: 2.71 | C: 60.63 H: 7.31 N: 2.52 | C: 62.21 H: 7.02 N: 2.51 | C: 60.39 H: 7.26 N: 2.21 |
| | (2) Calculated | C: 61.74 H: 7.08 N: 2.40 | C: 60.80 H: 7.14 N: 2.36 | C: 61.63 H: 7.24 N: 2.39 | C: 60.80 H: 7.14 N: 2.36 O: 29.69 |
| | | (for $C_{60}H_{82}N_2O_{21}$) | (for $C_{60}H_{84}N_2O_{22}$) | (for $C_{60}H_{84}N_2O_{21}$) | (for $C_{60}H_{84}N_2O_{22}$) |
| 3 | Molecular weight (1) FD-MS m/z | 1167 $(m+H)^+$ | 1185 $(M+H)^+$ | 1169 $(M+H)^+$ | 1185 $(M+H)^+$ |
| | (2) Calcd. | 1167.3 | 1185.3 | 1169.3 | 11.85.3 |
| 4 | mp (°C) | 139 ~ 143 | 147 ~ 152 | 178 ~ 181 | 110 ~ 112 |
| 5 | $[\alpha]_D^{25}$ Specific rotation | +136° | +48° | +161° | +132° |
| | | (C 0.05 in $CHCl_3$) | (C 0.05 in $CHCl_3$) | (C 0.05 in $CHCl_3$) | (C 0.05 in $CHCl_3$) |
| 6 | UV absorption spectrum | (in acidic methanol) | (in acidic methanol) | (in acidic methanol) | (in acidic methanol) |
| | $\lambda$ max nm ($E^{1\%}_{cm}$) | 254 (240), 290 (69) 495 (130) $530_s$ (87) | 254 (215), 292 (66) 495 (124) $530_s$ (81) | 254 (227), 290 (68) 495 (126) $530_s$ (83) | 254 (219), 290 (67) 494 (126) $530_s$ (83) |
| | | (in alkaline methanol) | (in alkaline methanol) | (in alkaline methanol) | (in alkaline methanol) |
| | | 241 (343), 295 (64) 568 (143), 607 (127) | 241 (308), 298 (59) 568 (135), 607 (121) | 241 (334), 298 (64) 568 (140), 607 (123) | 242 (325), 298 (62) 565 (138), 605 (125) |

TABLE 1—1 (continued)

| | | Ditrisarubicin D | Ditrisarubicin E | Ditrisarubicin F | Ditrisarubicin G |
|---|---|---|---|---|---|
| 7 | IR absorption spectrum (KBr tab.) | Fig. 1 | Fig. 3 | Fig. 5 | Fig. 8 |
| 8 | NMR spectrum | Fig. 2<br>250 MHz (in $CDCl_3$) | Fig. 4<br>250 MHz (in $CDCl_3$) | Fig. 6<br>250 MHz (in $CDCl_3$) | Fig. 9<br>250 MHz (in $CDCl_3$) |
| 9 | TLC *1<br>Rf value<br>$CHCl_3$:$CH_3OH$:<br>conc. $NH_3$ water | 0.31<br>100:5:0.1 | 0.27<br>100:5:0.1 | 0.25<br>100:5:0.1 | 0.22<br>100:5:0.1 |
| 10 | HPLC *2<br>$t_R$ value<br>acetonitrile:<br>0.45% aq.<br>phosphoric acid | 9.5 min<br>35/65 | 3.8 min<br>35/65 | 6.0 min<br>35/65 | 3.4 min<br>35/65 |
| 11 | Solubility<br>(1) Soluble | chloroform, ethyl acetate, methanol, acetonitrile, pyridine, dimethyl sulfoxide | chloroform, ethyl acetate, methanol, acetonitrile, pyridine, dimethyl sulfoxide | chloroform, ethyl acetate, methanol, acetonitrile, pyridine, dimethyl sulfoxide | chloroform, ethyl acetate, methanol, acetonitrile, pyridine, dimethyl sulfoxide |
| | (2) Sparingly soluble | water, petroleum ether, n-hexane | water, petroleum ether, n-hexane | water, petroleum ether, n-hexane | water, petroleum ether, n-hexane |

*1 On silica gel plate 60 $F_{254}$ (product of Merck Co.)
*2 Using Nucleosil $5C_{18}$ (Macherey-Nagel, Germany) in 4.6φ × 250 mm column.

TABLE 1—2

| | | Ditrisarubicin CR (5) | Ditrisarubicin GR (6) | Ditrisarubicin G/de-Ro (7) | Ditrisarubicin GR/De-Ro (8) |
|---|---|---|---|---|---|
| 1 | Appearance | red powder | red powder | red powder | red powder |
| 2 | Elemental analysis (%) (1) Found | C: 61.05 H: 7.13 N: 2.38 | C: 60.44 H: 7.41 N: 2.48 | C: 60.61 H: 6.82 N: 2.37 | C: 60.39 H: 6.78 N: 2.83 |
| | (2) Calculated | C: 61.84 H: 6.92 N: 2.40 | C: 60.80 H: 7.14 N: 2.36 | C: 60.55 H: 6.96 N: 2.61 | C: 60.55 H: 6.96 N: 2.61 |
| | | (for $C_{60}H_{80}N_2O_{21}$) | (for $C_{60}H_{84}N_2O_{22}$) | (for $C_{54}H_{74}N_2O_{20}$) | (for $C_{54}H_{74}N_2O_{20}$) |
| 3 | Molecular weight (1) FD-MS m/z | 1165 $(m+H)^+$ | 1184 $(m+H)^+$ | 1071 $(m+H)^+$ | 1071 $(m+H)^+$ |
| | (2) Calcd. | 1165.3 | 1185.3 | 1071.2 | 1071.2 |
| 4 | mp (°C) | 182 ~ 184 | 168 ~ 170 | 191 ~ 195 | 192 ~ 194 |
| 5 | $[\alpha]_D^{25}$ Specific rotation | +199° | +167° | +176° | +194° |
| | | (C 0.06 in $CHCl_3$) | (C 0.1 in $CHCl_3$) | (C 0.05 in $CHCl_3$) | (C 0.05 in $CHCl_3$) |
| 6 | UV absorption spectrum | (in acidic methanol) | (in acidic methanol) | (in acidic methanol) | (in acidic methanol) |
| | λ max nm ($E_{cm}^{1\%}$) | (265), 290 (92) 495 (125) $530_s$ (76) | 254 (219), 290 (67) 494 (120) $530_s$ (83) | 254 (256), 290 (75) 495 (142) $530_s$ (93) | 254 (256), 290 (72) 495 (136) $530_s$ (90) |
| | | (in alkaline methanol) | (in alkaline methanol) | (in alkaline methanol) | (in alkaline methanol) |
| | | 240 (410), 295 (85) 566 (134), 606 (119) | 240 (325), 298 (62) 565 (132), 605 (118) | 242 (374), 300 (67) 565 (159), 605 (141) | 241 (370), 300 (67) 565 (151), 605 (133) |

TABLE 1—2 (continued)

| | | Ditrisarubicin CR (5) | Ditrisarubicin GR (6) | Ditrisarubicin G/de-Ro (7) | Ditrisarubicin GR/de-Ro (8) |
|---|---|---|---|---|---|
| 7 | IR absorption spectrum (KBr tab.) | Fig. 11 | Fig. 13 | Fig. 15 | Fig. 17 |
| 8 | NMR spectrum | Fig. 12<br>400 MHz (in $CDCl_3$) | Fig. 14<br>400 MHz (in $CDCl_3$) | Fig. 16<br>400 MHz (in $CDCl_3$) | Fig. 18<br>400 MHz (in $CDCl_3$) |
| 9 | TLC<br>$CHCl_3$:$CH_3OH$:<br>conc.$NH_3$ water | 0.37<br>100:5:0.1 | 0.21<br>100:5:0.1 | 0.38<br>100:10:0.05 | 0.35<br>100:10:0.5 |
| 10 | HPLC *2<br>$t_R$ value<br>acetonitrile: 0.45% phosphoric acid | 11.5 min<br>35/65 | 3.9 min<br>35/65 | 3.0 min<br>35/65 | 3.8 min<br>35/65 |
| 11 | Solubility<br>(1) Soluble | chloroform, ethyl acetate, methanol, acetonitrile, pyridine, dimethyl sulfoxide | chloroform, ethyl acetate, methanol, acetonitrile, pyridine, dimethyl sulfoxide | chloroform, ethyl acetate, methanol, acetonitrile, pyridine, dimethyl sulfoxide | chloroform, ethyl acetate, methanol, acetonitrile, pyridine, dimethyl sulfoxide |
| | (2) Sparingly soluble | water, petroleum ether, n-hexane | water, petroleum ether, n-hexane | water, petroleum ether, n-hexane | water, petroleum ether, n-hexane |

*1, *2: See the footnotes to Table 1-1.

TABLE 1—3

| | | Ditrisarubicin B/10de-dF-CB (9) | Ditrisarubicin B/7de-dF-CB (10) | 1-hydroxy serirubicin |
|---|---|---|---|---|
| 1 | Appearance | red powder | red powder | red powder |
| 2 | Elemental analysis (%) | | | |
| | (1) Found | C: 61.03 H: 6.39 N: 2.64 | C: 61.13 H: 7.03 N: 2.84 | C: 60.83 H: 7.12 N: 2.27 |
| | (2) Calculated | C: 61.27 H: 6.86 N: 2.98 | C: 61.27 H: 6.86 N: 2.98 | C: 61.01 H: 6.83 N: 2.37 |
| | | (for $C_{48}H_{64}N_2O_{17}$) | (for $C_{48}H_{64}N_2O_{17}$) | (for $C_{60}H_{80}N_2O_{22}$) |
| 3 | Molecular weight | | | |
| | (1) FD-MS m/z | 941 $(M+H)^+$ | 941 $(M+H)^+$ | 1181 $(M+H)^+$ |
| | (2) Calcd. | 941.0 | 941.0 | 1181.3 |
| 4 | mp (°C) | 191 ~ 195 | 189 ~ 192 | 189 ~ 192 |
| 5 | $[\alpha]_D^{25}$ Specific rotation | +291.2° | +345.5° | +1° (±2) |
| | | (C 0.05 in $CHCl_3$) | (C 0.05 in $CHCl_3$) | (C 0.1 in $CHCl_3$) |
| 6 | UV absorption spectrum | (in acidic methanol) | (in acidic methanol) | (in acidic methanol) |
| | λ max nm ($E^{1\%}_{cm}$) | 255 (270), 292 (81) 495 (153), $530_s$ (107) | 255 (284), 292 (85) 496 (165), $530_s$ (108) | 256 (190), 290 (68) 493 (119), $513_s$ (89) |
| | | (in alkaline methanol) | (in alkaline methanol) | (in alkaline methanol) |
| | | 241 (439), 298 (78) 568 (179), 610 (153) | 240 (425), 298 (73) 568 (176), 610 (159) | 238 (373), 290 (69) 565 (145), 606 (139) |

TABLE 1—3 (continued)

| | | Ditrisarubicin B/10de-dF-CB (9) | Ditrisarubicin B/7de-dF-CB (10) | 1-hydroxyserirubicin (11) |
|---|---|---|---|---|
| 7 | IR absorption spectrum (KBr tab.) | Fig. 19 | Fig. 21 | Fig. 24 |
| 8 | NMR spectrum | Fig. 20<br>400 MHz (in $CDDl_3$) | Fig. 22<br>400 MHz (in $CDCl_3$) | Fig. 25<br>400 MHz (in $CDCl_3$) |
| 9 | TLC *1 Rf value<br>$CHCl_3$:$CH_3OH$:<br>conc.$NH_3$ water | 0.30<br>100:10:0.05 | 0.23<br>100:10:0.05 | 0.41<br>100:5:0.1 |
| 10 | HPLC *2<br>$t_R$ value<br>acetonitrile:<br>0.45%<br>phosphoric acid | 1.25 min<br>25/75 | 20.8 min<br>25/75 | 6.3 min<br>35/65 |
| 11 | Solubility<br>(1) Soluble | chloroform, ethyl acetate, methanol, acetonitrile, pyridine, dimethyl sulfoxide | chloroform, ethyl acetate, methanol, acetonitrile, pyridine, dimethyl sulfoxide | chloroform, ethyl acetate, methanol, acetonitrile, pyridine, dimethyl sulfoxide |
| | (2) Sparingly soluble | water, petroleum ether, n-hexane | water, petroleum ether, n-hexane | water, petroleum ether, n-hexane |

*1, *2: See the footnotes to Table 1—1.

At present, the anthracycline compounds of the present invention can only be prepared by cultivating microorganisms. However, they could be prepared by chemical or microbiological modifications of related compounds, or even by total synthesis from such related compounds.

If the anthracycline compounds of the present invention are prepared by cultivation of microorganisms, the strain to be used is selected from among the microorganisms of the genus *Streptomyces* which are capable of producing the anthracycline compounds of the present invention. For example, the strain *Streptomyces cyaneus* MG-344-hF 49 has the ability to produce the anthracycline compounds of the present invention. Other suitable strains may be isolated from the nature by techniques conventionally used in the isolation of anti-biotics producing microorganisms. Strain *S. cyaneus* MG 344-hF 49 and other microorganisms capable of producing the anthracycline compounds of the present invention could be exposed to radio-active radiation or given other treatments for the purpose of enhancing their ability to produce the anthracycline compounds.

The strain MG 344-hF 49 has the following characteristics:

*Streptomyces cyaneus* MG 344-hF 49 is an actinomycetes that was isolated in August 1980 from the soil in the premises of the Institute of Microbial Chemistry. This strain was deposited with the Fermentation Research Institute, Agency of Industrial Science and Technology on June 28, 1982 and given the number FERM BP-314.

(1) Mycological Properties

A. Morphology

Under microscopic observation, strain MG 344-hF 49 forms spiny or spiral aerial mycellia from branched substrate mycellia, but no whirl is observed. Each mature spore chain consists of at least 10 spores. The spore size is roughly in the range of 0.4—0.6 × 0.8—1.0 µm, and the spore surface is spiny.

B. Growth on various media

The designations in brackets used in the color indication given in the following description are in accordance with Color Harmony Manual published by Container Corporation of America.

(a) Ciniclose-nitrate agar medium (cultured at 27°C)
A white thin film of aerial mycellia adheres to a growth of dull purplish red [9 le, Raspberry]. Purplish soluble pigment.

(b) Glucose-asparagine agar medium (cultured at 27°C)
Light bluish gray [18 ec, Lt Aqua] aerial mycellia adhere to a yellowish red [6 la, Lt Coral Red — 6 pc, paprika] growth. Reddish soluble pigment.

(c) Glycerine-asparagine agar medium (ISP-medium 5: cultured at 27°C)
Aerial mycellia having colors in the range of from white thru gray white to light bluish gray adhere to a grayish purple [9 lg, Rose Plum] growth. No soluble pigment observed.

(d) Starch-inorganic salt agar medium (ISP-medium 4: cultured at 27°C)
Aerial mycellia having colors in the range of from white to grayish blue green [19 ie, Turquoise Green] adhere to a pale pink growth. Soluble pigment with a pink tinge.

(e) Tyrosine agar medium (ISO-medium 7: cultured at 27°C)
Aerial mycellia having colors in the range of from gray white to light bluish gray adhere to a growth ranging in color from light brown to dark brown. Soluble pigment with a light brownish tinge.

(f) Nutrient agar medium (cultured at 27°C)
Aerial mycellia having a light purplish gray color adhere to a grayish red purple [8 le, Rose Wine] growth. A brown soluble pigment was produced.

(g) Yeast-malt agar medium (ISP medium 2: cultured at 27°C)
Aerial mycellia having colors in the range of from white thru purplish white to bluish white adhere to a grayish red purple [9 ne, Raspberry] growth. No soluble pigment observed.

(h) Oatmeal agar medium (ISP-medium 3: cultured at 27°C)
Aerial mycellia having colors in the range of bluish gray to grayish blue green [21, li, Dk Jade Gray] adhere to a growth having colors ranging from pale pink to dull purple [10 pc, Fuchsia Purple]. Soluble pigment with a red tinge.

(i) Glycerine-nitrate agar medium (cultured at 27°C)
White aerial mycellia slightly adhere to a grayish purple [9 le, Raspberry] growth. Soluble pigment with a purplish tinge.

(j) Starch agar medium (cultured at 27°C)
Growth with a dull grayish red purple color [7 1/2 le, Rose Wine]. No aerial mycellia. Soluble pigment with a purplish tinge.

(k) Calcium malate agar medium (cultured at 27°C)
A thin film of white aerial mycellia adhere to a pale purple growth. Soluble pigment with a purplish tinge.

(l) Cellulose (synthetic solution added with filter paper, cultured at 27°C).
No growth.

(m) Gelatin stab culture
With simple gelatin medium (cultured at 20°C), white aerial mycellia slightly adhere to a pale yellow growth. Brown soluble pigment is produced. With glucose-peptone-gelatin medium (cultured at 27°C), pink white aerial mycellia adhere to a colorless to pale yellow growth. Dark brown soluble pigment is produced.

(n) Skim milk (cultured at 37°C)
White aerial mycellia adhere slightly to a pink to grayish red growth. Soluble pigment with a brown tinge.

(2) Physiological Properties
A. General description
a. Growth temperature range
Tests on glucose-asparagine agar at 20°C, 24°C, 27°C, 30°C, 37°C and 50°C showed that the strain grew at all temperatures except for 50°C. Optimum temperature woild approximately be in the range of 30—37°C.

b. Liquefaction of gelatin (15% simple gelatin: cultured at 20°C, glucose-peptone-gelatin: cultured at 27°C)
With the simple gelatin, liquefaction started approximately on the fifth day of the cultivation. With the

glucose-peptone-gelatin, no liquefaction took place even when two weeks passed after the start of the cultivation, and weak liquefaction was observed approximately on the third week. The degree of liquefaction was from moderate to weak with the simple gelatin, and weak with the glucose-peptone-gelatin.

c. Hydrolysis of starch (cultured at 27°C on either starch-inorganic agar medium or starch agar medium)
Starch hydrolysis was found to occur on about the third day of the cultivation, and its degree was from moderate to weak.

d. Coagulation and peptonization of skim milk (cultivation on skim milk at 37°C)
Coagulation started to occur on about the third day of the cultivation, and on about the seventh day, coagulation was completed and peptonization started. The degree of peptonization was moderate.

e. Formation of melamine-like pigment (cultured at 27°C on either Tryptone-yeast-broth (ISP-medium 1), peptone-yeast-iron agar (ISP-medium 6) or tyrosine agar (ISO-medium 7))
Pigment formation was observed on each medium.

f. Utilization of carbon sources (cultured at 27°C on Pridham-Gottlieb agar medium (ISP-medium 9))
The strain grew by utilizing any of the following carbon sources: L-arabinose, D-xylose, D-glucose, D-fructose, sucrose, inositol, L-rhamnose, raffinose and D-mannitol.

g. Dissolution of calcium malate (calcium malate agar: cultured at 27°C)
Calcium malate was found to dissolve on the periphery of the growth approximately on the seventh day of the cultivation, but its degree was rather weak.
h. Reduction of nitrate (aqueous peptone containing 1.0% potassium nitrate (ISP-medium 8): cultured at 27°C)
Positive.

B. Conclusion and Identification of New Strain
To summarize: Strain MG 344-hF 49 belongs to the genus *Streptomyces* and 2,6-diaminopimelic acid contained in the cell wall is of the L,L-type. No sporandia observed. Aerial mycellia form a spiral but not a whirl. The spore surace is spiny. With various media, aerial mycellia having colors in the range of from white thru light bluish gray to grayish blue green adhere to a growth ranging in color from grayish red purple to yellowish red. Purplish or reddish soluble pigment formed. The reverse side of the growth serves as a pH indicator, and its color changes from purplish to bluish purple or blue upon addition of 1N NaOH. Melamine-like pigment is positive and the proteolytic power is from moderate to weak. The degree of starch hydrolysis is also from moderate to weak.

The screening of the known strains on the basis of these data show that *Streptomyces cyaneus* would be most closely related to strain MG 344-hF 49 (International Journal of Systematic Bacteriology, *22,* p. 290, 1972 (Reference 1), Waksman; The Actinomycetes, *2,* p. 199, 1961 (Reference 2), Bergey's Manual of Determinative Bacteriology, 7th ed., p. 757, 1957 and supra, 8th ed., p. 822, 1974 (Reference 3)).

The properties of strain MG 344-hF 49 are compared in the following table with those of *Streptomyces Cyaneus* listed in the literature cited.

TABLE 2

| | MG 344-hF 49 | *Streptomyces cyaneus* ISP 5108 |
|---|---|---|
| Form of aerial mycellia | spiral | spiral |
| Spore surface | spiny | spiny |
| Color of aerial mycellia | white — light bluish gray — grayish blue green | pale blue — bluish gray |
| Color of growth | pale pink — grayish red purple | dark grayish blue — dark grayish purple |
| Soluble pigment | from (−) to purplish, sometimes reddish | from (−) to purplish or bluish |
| Formation of melamine-like pigment | + | + |
| Starch hydrolysis | + moderate to weak | + weak* |
| Milk coagulation | + | + |
| Milk peptonization | + | + |
| Gelatin liquefaction | + moderate to weak | + strong* |
| Reduction of nitrate | + | −* |
| Utilization of carbon sources | | |
| D-glucose | + | + |
| L-arabinose | + | + |
| D-xylose | + | + |
| D-fructose | + | + |
| Sucrose | + | + |
| Inositol | + | + |
| L-rhamnose | + | + |
| Raffinose | + | + |
| D-mannitol | + | + |
| pH indicator | + | + |

*According to References 2 and 3.

As shown in Table 2, strain MG 344-hF 49 and *Streptomyces cyaneus* are substantially the same in their properties except for the reduction of nitrate. However, the reduction of nitrate is not considered a reliable parameter for identifying a certain microorganism as an Actinomyces and cannot be used to distinguish between the two strains.

Therefore, strain MG 344-hF 49 is considered to be most closely related to *Streptomyces cyaneus*, and for this reason, the present inventors identified strain MG 344-hF 49 as *Streptomyces cyaneus* MG 344-hF 49.

The anthracycline compounds of the present invention can be produced by aerobically cultivating on a suitable medium the microorganism of the genus *Streptomyces* that are capable of producing the anthracycline compounds of the present invention, and by subsequently collecting the final compound from the culture.

The medium used may contain any of the nutrient sources that can be utilized by the microorganism capable of producing the anthracycline compounds of the present invention. Specific examples of the nutrient sources include carbon sources such as glycerine, glucose, sucrose, maltose, dextrin, starch, as well as fats and oils; nitrogen sources such as organic substances like soybean meal, cotton seed oil cake, meat extract, peptone, dry yeast, yeast extract and corn steep liquor, as well as inorganic substances like ammonium salts and nitrate salts typified by ammonium sulfate, sodium nitrate and ammonium chloride. If necessary, inorganic salts such as sodium chloride, potassium chloride, phosphate salts and heavy metal salts may be added. In order to inhibit foaming that may occur during fermentation, a suitable defoamer such as silicone may be optionally added according to the conventional technique.

As in the case of common practice in the production of antibiotics, aerobic liquid submerged culture is the most appropriate method for cultivating the anthracycline producing microorganism. The cultivation temperature is suitably in the range of 20 to 35°C, with 25—30°C being particularly preferred. Whether this method depends on shake culture or aerated spinner culture, the yield of the anthracycline compound of the present invention reaches a maximum in three to seven days.

In this manner, a culture having the anthracycline compound of the present invention accumulated therein can be obtained. Part of the anthracycline compound may exist in the cultured cells, but the greater part of it is contained in the culture filtrate.

Any method that suits the specific purpose of the present invention may be used to recover the desired anthracycline compound from the thusly obtained culture. One technique is based on the principles of extraction; stated specifically, the anthracycline compound in the culture filtrate may be extracted with a water-immiscible ditrisarubicin dissolving solvent (see the literature cited above), such as ethyl acetate, butyl acetate, chloroform or butanol (high extraction efficiency is achieved if the culture filtrate is neutral to weakly basic), and if the anthracycline compound is within the cultured cells, the latter may be collected by filtration, centrifugation or any other suitable method, and the desired compound may be recovered by treating the collected cells with ethyl acetate, chloroform, methanol, ethanol, butanol, acetone, methyl ethyl ketone, aqueous hydrochloric acid, aqueous acetic acid, or the like. The culture may be directly subjected to such extraction treatments without separating the cells. The cells may be subjected to extraction after homogenization. Another possible extraction technique is the countercurrent distribution method.

Another method for recovering the anthracycline compounds of the present invention from the culture is based on the principles of adsorption. An already liquid substance such as the culture filtrate which contains the anthracycline compound of the present invention, or the extract obtained by the extraction techniques shown above is subjected to column chromatography, liquid chromatography or other adsorption techniques using a suitable adsorbent such as activated carbon, alumina, silica gel or "Sephadex® LH 20" (Pharmacia Co.); the adsorbate is subsequently eluted to recover the desired anthracycline compound of the present invention. The thusly obtained solution of the anthracycline compound of the present invention is concentrated to dryness under vacuum, thereby giving a crude sample of the end anthracycline compound as a red powder.

The crude sample is purified by repeating the necessary times the extraction and adsorption procedures shown above, which may be combined as required. If necessary, recrystallization may be performed. For example, column chromatography using an adsorbent such as silica gel, Sephadex LH 20®, a weakly acidic ion exchange resin or Diaion HP 20® (product of Mitsubishi Chemicals Industries Limited), or a gel filtering agent may be performed in combination with liquid chromatography (countercurrent distribution), high-pressure liquid chromatography or thin-layer chromatography using a suitable solvent. Stated more specifically, a crude powder of the anthracycline compound of the present invention is dissolved in a small amount of chloroform, and the solution is passed through a silica gel column. By elution with a suitable solvent, effluents containing the respective effective components are obtained. The desired active fractions are collected and concentrated under vacuum. By subsequent thin-layer chromatography, each of the end components can be obtained as a substantially pure product. For further purification, high-pressure liquid chromatography or crystallization from a suitable solvent may be performed.

The anthracycline compounds according to the present invention have the activity of inhibiting the growth of leukemic cells and the anti-tumor activity, so that are useful as medicines.

## Determination of Chemical Structure

I. Determining the structures of ditrisarubicins

An outline for the procedure of determining the structures of ditrisarubicin D, E, F, G, CR, GB, G/de-Ro, GR/de-Ro, B/10 de-dF-CB and B/7 de-dF-CB (hereunder collectively referred to as DTR-X) is shown in Fig. 26, wherein RN represents rhodosamine and R1, R2, R3, R4, R5 and R6 respective sugars. In the following description, the symbols in Fig. 26 are used to identify the respective compounds.

DTR-X (1) was completely hydrolyzed by heating in 0.1N HCl at 85°C for 30 minutes. In such case, red aglycone (2) and respective sugars were obtained. Based on the Rf value with TLC on silica gel, UV absorption spectra, mass spectrum (m/z 386 $(M)^+$) and $^1$H-NMR spectrum, (2) was identified as β-rhodomycinone.

The liquid hydrolyzates were neutralized with silver carbonate, developed on silica gel TLC with a

mixture of n-butanol/acetic acid/water (4:1:1) and subjected to color reaction with p-anisaldehyde. The Rf values and color changes were used as identification means. The constituents sugars of DTR-X (1) and the parent peaks obtained in FD-mass spectra are shown in Table 3, wherein ++, + and − represent color changes from the result obtained with the hydrolyzate of control ditrisarubicin B (DTR-B).

TABLE 3

| DTR-X (1) | FD — MS m/z | Constituent sugars* | | | | |
|---|---|---|---|---|---|---|
| | | rhodosamine | 2-deoxyfucose | rhodinose | cinerulose | cinerulose |
| DTR-D | 1167 $(M+H)^+$ | ++ | + | + | + | + |
| DTR-E | 1185 $(M+H)^+$ | ++ | ++ | − | ++ | − |
| DTR-F | 1169 $(M+H)^+$ | ++ | + | ++ | − | ++ |
| DTR-G | 1185 $(M+H)^+$ | ++ | ++ | + | − | + |
| DTR-CR | 1165 $(M+H)^+$ | ++ | + | + | − | ++** |
| DTR-GR | 1184 $(M)^+$ | ++ | ++ | + | − | + |
| DTR-G/de-Ro | 1071 $(M+H)^+$ | ++ | ++ | − | − | + |
| DTR-Gr/de-Ro | 1071 $(M+H)^+$ | ++ | ++ | − | − | + |
| DRT-B/10de-dF-CB | 941 $(M+H)^+$ | ++ | + | − | − | + |
| DTR-B/7de-dF-CB | 941 $(M+H)^+$ | ++ | + | − | − | + |
| DTR-B | 1181 $(M+H)^+$ | ++ | ++ | − | − | ++ |

*: ++, + and − represent respective color changes.
**: $^1$H-NMR spectrum shows that DTR-CR has a molecule each of aculose and cinerulose B.

When (*1*) was heated in 0.1N HCl at 70°C for 20 minutes, compound (3) resulted in each case. Upon complete hydrolysis, (3) gave β-rhodomycinone (2) and rhodosamine. Compound (3) gave a parent peak at m/z 700 $(M)^+$ in FD-mass spectrum. Based on this information taken together with the results of $^1$H-NMR spectrum, compound (3) was found to be β-rhodomycine II having a molecule of rhodosamine bonded to each of 7- and 10-positions (Tetrahedron Lett., 1969, 415—419, 1969). It therefore became clear that all variants of DTR-X (1) has β-rhodomycine II in their partial structure.

When compound (*1*) was reduced catalytically at ordinary temperature and pressure for 10—30 minutes in the presence of 5% $Pd/BaSO_3$, compound (*4*) was obtained.

When compound (*4*) was completely hydrolyzed in 0.1N HCl, red aglycone (*5*) and various sugars formed in each case. Based on the rf value with TLC on silica gel, UV absorption spectra, mass spectrum (m/z 370 $(M)^+$) and $^1$H-NMR specvtrum, (*5*) was identified as γ-rhodomycinone. Identification of the sugars was made on the basis of Rf values on silica gel TLC and color changes. The constituent sugars of the reduction decomposition product (4) of DTR-X (*1*) (the product is hereunder referred to as DTR-X/$H_2$) and the parent peaks in FD-mass spectra therefor are shown in Table 4.

TABLE 4

| DTR-X/$H_2$ (4) | FD — MS m/z | Constituent sugars* | | | | |
|---|---|---|---|---|---|---|
| | | rhodosamine | 2-deoxyfucose | rhodinose | cinerulose | cinerulose |
| DTR-D/$H_2$ | 754 $(M+H)^+$ | + | – | + | + | – |
| DTR-E/$H_2$ | 770 $(M+H)^+$ | + | + | – | + | – |
| DTR-F/$H_2$ | 756 $(M+H)^+$ | + | – | ++ | – | – |
| DTR-G/$H_2$ | 772 $(M+H)^+$ | + | + | + | – | – |
| DTR-CR/$H_2$ | 768 $(M+H)^+$ | + | + | – | – | + |
| DTR-GR/$H_2$ | 768 $(M+H)^+)$ | + | + | – | – | + |
| DTR-G/de-Ro/$H_2$ | 658 $(M+H)^+$ | + | + | – | – | – |
| DTR-Gr/de-Ro/$H_2$ | 768 $(M+H)^+$ | + | + | – | – | + |
| DRT-B/10de-dF-CB/$H_2$ | 527 $(M)^+$ | + | – | – | – | – |
| DTR-B/7de-dF-CB/$H_2$ | 768 $(M+H)^+$ | + | + | – | – | + |

*: ++, + and – represent respective color changes.

When DTR-X/$H_2$ (4) was partially hydrolyzed in 0.1N HCl by heating at 70°C for 10 minutes, compound (6) resulted in each case. Upon complete hydrolysis, (6) gave γ-rhodonomycine (5) and rhodosamine, with a parent peak appearing at m/z 527 $(M)^+$ in FD-mass spectrum. Analysis by $^1$H-NMR spectrum suggested a structure wherein a molecule of rhodosamine was bonded to (5). Based on these data, compound (6) was identified by γ-rhodomycine I (Naturwissenshaften, *48*, 717, 1961). It is therefore concluded that each variant of (4) contains γ-rhodomycine I in its partial structure.

Figs. 27 to 33 are $^1$H-NMR charts for DTR-D/$H_2$, E/$H_2$, F/$H_2$, G/$H_2$, CR/$H_2$, G/de-Ro/$H_2$ and B/10 de-dF-CB/$H_2$. Corresponding $^1$H-NMR spectra for DTR-GR/$H_2$, GR/de-Ro/$H_2$ and B/7 de-dF-CB/$H_2$ are omitted since they are the same as that for DTR-CR/$H_2$. These data show that DTR-X/$H_2$ (4) variants are compounds wherein the sugar chains listed in Table 5 are bonded to γ-rhodomycinone (5) at 10-position.

The data in Tables 3 and 5, as well as the $^1$H-NMR spectrum charts in Figs. 2, 4, 6, 9, 12, 14, 16, 18, 20 and 22 support the conclusion that the respective variants of DTR-X(1) have the structures shown in Table 6.

TABLE 5

| DTR-X/H (4) | Sugar chains* |
|---|---|
| DTR-D/$H_2$ | (V) |
| DTR-E/$H_2$ | (II) |
| DTR-F/$H_2$ | (VI) |
| DTR-G/$H_2$ | (III) |
| DTR-CR/$H_2$ | (I) |
| DTR-GR/$H_2$ | (I) |
| DTR-G/de-Ro/$H_2$ | (VII) |
| DTR-GR/de-Ro/$H_2$ | (I) |
| DTR-B/10de-dF-CB/$H_2$ | (VIII) |
| DTR-B/7de-dF-CB/$H_2$ | (I) |

*For the meanings of the respective symbols, see Fig. 36.

TABLE 6

Structures of respective ditrisarubicines

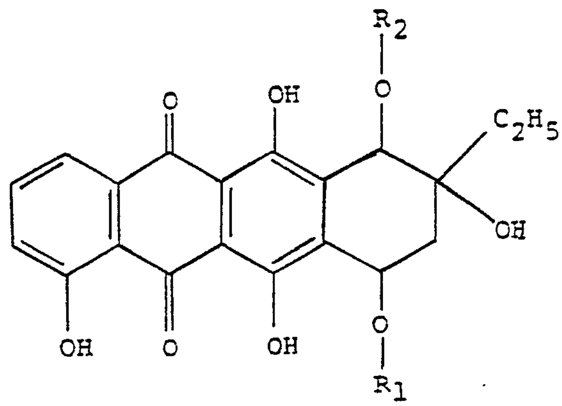

| Compound | Sugar chains | |
|---|---|---|
| | $R_1$ | $R_2$ |
| (*1*) DTR-D | (I) | (V) |
| (*2*) DTR-E | (II) | (II) |
| (*3*) DTR-F | (I) | (VI) |
| (*4*) DTR-G | (I) | (III) |
| (*5*) DTR-CR | (IV) | (I) |
| (*6*) DTR-GR | (III) | (I) |
| (*7*) DTR-G/de-Ro | (I) | (VII) |
| (*8*) DTR-GR/de-Ro | (VII) | (I) |
| (*9*) DTR-B/10de-dF-CB | (I) | (VIII) |
| (*10*) DTR-B/7de-dF-CB | (VIII) | (I) |

*For the meanings of the respective symbols, see Fig. 36.

(II) Determining the structure of 1-hydroxyserirubicin

When 1-hydroxyserirubicin (hereunder abbreviated as 1-HSR) was heated in 0.1N HCl at 85°C for 30 minutes, red orange aglycon and three sugars were produced. Based on the Rf value on silica gel TLC, mass spectrum (m/z 386 $(M)^+$) and $^1$H-NMR spectrum (Fig. 34), the aglycone was identified as $\alpha_2$-rhodomycinone (chem. Ber., *101,* 1341—1348, 1968). The sugars as hydrolyzates were developed on silica gel TLC with a mixture of n-butanol/acetic acid/water (4:1:1) and subjected to color reaction with anisaldehyde-sulfuric acid. Based on the Rf values and resulting color changes, the respective sugars were found to be rhodosamine, 2-deoxyfucose and cinerulose B. Since 1-HSR gave a parent peak at m/z 1181 $(M+H)^+$ in FD-mass spectrum, said compound is believed to have two molecules each of rhodosamine, 2-deoxyfucose and cinerulose bonded to $\alpha_2$-rhodomycinone.

Table 7 classifies the chemical shifts in $^{13}$C-NMR spectrum charts for the sugar chains in 1-HSR, as compared with the data for ditrisarubicin B (J. Antibiotics, *36,* 1080—1083, 1983) and serirubicin. The data suggest that the 1-HSR compounds tested have identical sugar chains. Fig. 25 shows $^1$H-NMR spectra for 1-HSR. This spectral analysis also supports the fact that 1-HSR compounds have the structures shown in Fig. 35.

TABLE 7

| Carbon | ditrisarubicin | serirubicin | 1-hydroxyserirubicin |
|---|---|---|---|
| 1′, 1′′′′ | 97.4, 101.9 | 96.6, 100.2 | 96.3, 100.2 |
| 2′, 2′′′′ | 29.4, 29.8 | 29.6 × 2 | 29.6 × 2 |
| 3′, 3′′′′ | 61.4 × 2 | 61.4, 61.6 | 61.4, 61.5 |
| 4′, 4′′′′ | 74.3, 74.6 | 74.4, 74.6 | 74.3, 74.5 |
| 5′, 5′′′′ | 68.3, 68.5 | 68.3 68.5 | 68.3, 68.4 |
| 6′, 6′′′′ | 17.9, 18.1 | 17.9, 18.1 | 18.0, 18.1 |
| $NMe_2 \times 2$ | 43.2, 43.3 | 43.28, 43.31 | 43.29, 43.33 |
| 1′′, 1′′′′′ | 99.1 × 2 | 99.2 × 2 | 99.1 × 2 |
| 2′′, 2′′′′′ | 26.9, 27.0 | 26.9, 27.0 | 26.9, 27.0 |
| 3′′, 3′′′′′ | 67.3, 67.4 | 67.28, 67.35 | 67.2, 67.3 |
| 4′′, 4′′′′′ | 67.0 × 2 | 67.0 × 2 | 66.9 × 2 |
| 5′′, 5′′′′′ | 65.3, 65.4 | 65.3, 65.4 | 65.2, 65.3 |
| 6′′, 6′′′′′ | 16.0 × 2 | 16.0 × 2 | 15.96, 16.00 |
| 1′′′, 1′′′′′′ | 91.6 × 2 | 91.6 × 2 | 91.6 × 2 |
| 2′′′, 2′′′′′′ | 63.0, 63.1 | 63.0, 63.1 | 62.97, 63.04 |
| 3′′′, 3′′′′′′ | 39.7, 39.8 | 39.7, 39.8 | 39.70, 39.75 |
| 4′′′, 4′′′′′′ | 208.1 × 2 | 208.1 × 2 | 208.1 × 2 |
| 5′′′, 5′′′′′′ | 77.88, 77.94 | 77.88, 77.94 | 77.88, 77.94 |
| 6′′′, 6′′′′′′ | 16.1, 16.2 | 16.1, 16.2 | 16.16, 16.20 |

| | $R_1$ | $R_2$ |
|---|---|---|
| ditrisarubicin B | H | OH |
| serirubicin | H | H |
| 1-hydroxyserirubicin | OH | H |

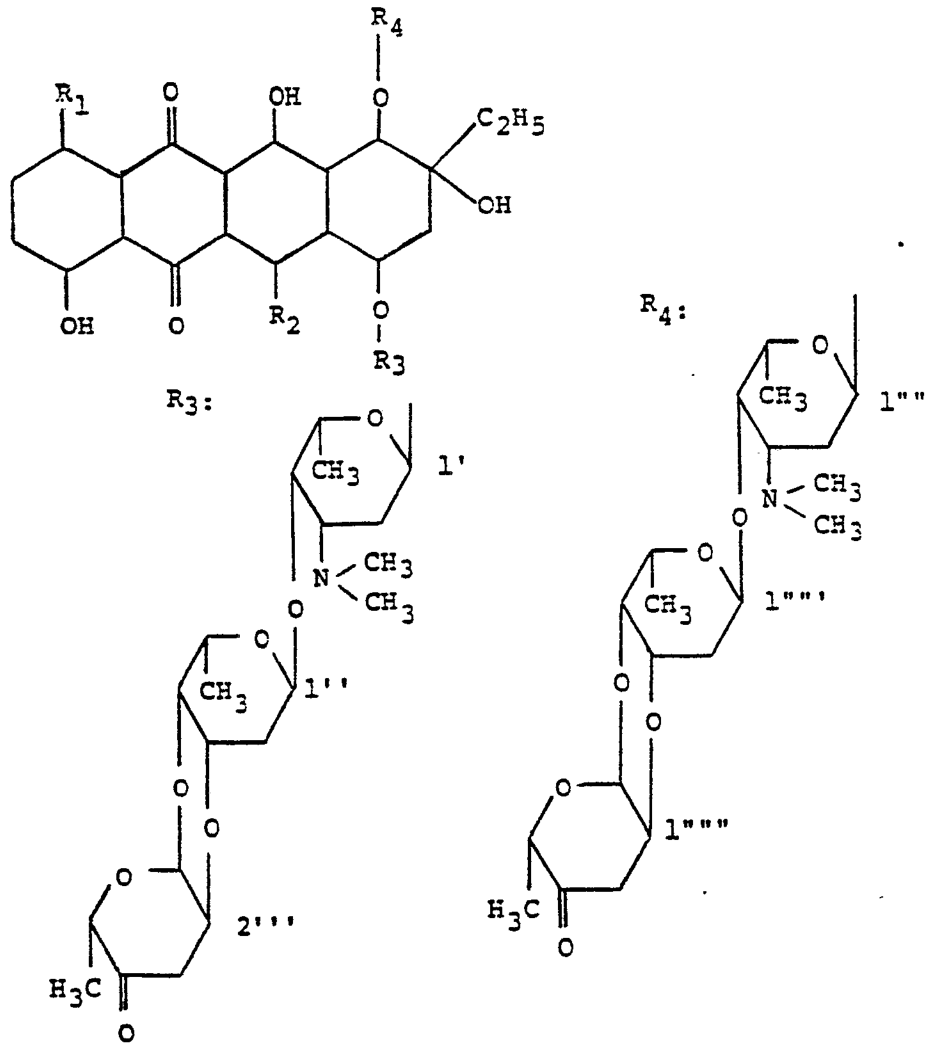

## Biological Activity

(1) Activity of inhibiting the growth of cultures leukemic cells P 388.

The anthracycline compounds according to the present invention inhibited the growth of the cultured cells in very low doses. The following table shows the concentrations ($IC_{50}$) of the compounds of the present invention that could achieve 50% inhibition of the growth of a two-day culture of leukemic cells P 388 as compared with the untreated group.

TABLE 8

| Compound | $IC_{50}$ (μm/ml) |
|---|---|
| DTR-D | 0.0040 |
| DTR-E | 0.0070 |
| DTR-F | 0.0038 |
| DTR-G | 0.0045 |
| DTR-CR | 0.0040 |
| DTR-GR | 0.0042 |
| DTR-G/de-Ro | 0.012 |
| DTR-GR/de-Ro | 0.020 |
| DTR-B/10de-dF-CB (not claimed) | 0.009 |
| DTR-B/7de-dF-CB (not claimed) | 0.021 |
| 1-HSR | 0.012 |

(2) Anti-tumor effect for mice bearing Leukemia L 1210

A group of $CDF_1$ mice were intraperitoneally transplanted with suspensions of leukemic cells L 1210 ($1 \times 10^5$ cells/mouse). Immediately after the transplantation, 0.25 ml each of the test compounds was injected peritoneally into the mice for 10 days. Observation was made for 30 days, and the percent survival for each test compound was determined, with 100 representing the number of days which the control mice group administered physiological saline survived. The results are shown in Table 9.

TABLE 9

| Dose (mg/kg/day) | Percent survival (%) | | | | |
|---|---|---|---|---|---|
| | DTR-D | DTR-E | DTR-F | DTR-G | 1-HSR |
| 1 | — | — | — | — | Toxin 114 |
| 0.5 | — | — | — | — | 139 |
| 0.25 | 131 | 106 | Toxin 133 | Toxin 125 | 145 |
| 0.125 | 114 | 156 | 152 | 138 | 151 |
| 0.0625 | 142 | 163 | 133 | 163 | 127 |
| 0.0313 | 125 | 119 | 127 | 117 | 108 |
| 0.0156 | 102 | 102 | 120 | 111 | — |
| 0.0078 | 97 | 102 | 101 | 109 | — |

## Production of the Compounds of the Present Invention

### Example 1

(1) Preparation of seed culture

A medium having the following composition was used.

| | |
|---|---|
| Galactose | 2% |
| Dextrin | 2% |
| Bacto-soyton® (Difco Lab.) | 1% |
| Corn steep liquor | 0.5% |
| Calcium carbonate | 0.1% |
| pH before sterilization | 7.4 |

A 100-ml portion of this medium was charged into a 500-ml conical flask and sterilized. The sterilized medium was inoculated with a portion of *Streptomyces cyaneus* MG 344-hF 49 taken from a slant culture with a platinum rod. The inoculant was subjected to shake culture at 27°C for 72 hrs.

(2) Fermentation

A medium having the following composition was used.

| | |
|---|---|
| Dextrin | 3% |
| Glucose | 0.3% |
| Toast-soya (trade name for soybean meal produced by Nissin-Seiyu Co.) | 2% |
| Cobalt chloride | 0.12 g/l |
| Calcium carbonate | 0.3% |

Fifteen liters of this medium was charged into a 30-liter jar fermenter, sterilized and inoculated with 500 ml of the seed culture prepared in (1). The inoculant was cultured at 27°C for 90 hrs at 150 rpm while air was supplied at a rate of 15 liters/min.

(3) Collection of ditrisarubicins

The culture obtained in (2) was filtered and the pH of the filtrate was adjusted to 8.0. The so adjusted filtrate was subjected to extraction with 10 liters of butyl acetate, and the top layer of the extract was concentrated to give 20 g of an oil. The oil was dissolved in a small amount of chloroform. The solution was passed through a column packed with 100 g ("Kiesel Gel 60" of Merck Co.) and subjected to gradient elution with a mixture of chloroform and methanol at varying chloroform to methanol ratios. The zones containing ditrisarubicin D, E, F, G and GR were concentrated to produce 230 mg of the ditrisarubicins as a red powder.

### Example 2

A 100-mg portion of the crude red powder obtained in Example 1 was dissolved in a small amount of chloroform and the solution was spotted on ten thin layers of silica gel ("Kiesel Gel 60 $F_{254}$" of Merck Co.) that were 0.25 mm thick and 20 × 20 cm large. After development with a mixture of chloroform, methanol and conc. aqueous ammonia (100:5:0.02), separate zones for ditrisarubicin D, E, F, G and GR were collected and eluted from the silica gel with a chloroform/methanol (10:1) mixture. The so-obtained fractions were concentrated and purified by HPLC. Separation was conducted on a column (20 φ × 300 mm) of "Nucleosil $5C_{18}$" (Nagel Co.) with a solvent system of acetonitrile/0.45% aq. phosphoric acid.

The respective eluates were neutralized with sodium bicarbonate and extracted with chloroform. The chloroform layer was concentrated to dryness. The samples dissolved in chloroform/methanol (1:1) mixture were put on columns (2.0 φ × 28 cm) of "Cephadex LH-20" that had been equilibrated with the same solvent mixture. Upon elution with the same solvent mixture and concentration under vacuum to dryness, ditrisarubicin D, E, F, G and GR were obtained in respective amounts of 32.0 mg, 31.5 mg, 30.5 mg, 65.5 mg and 67.5 mg. Each of these samples was found to consist of a single component upon analysis by TLC on silica gel plate and high-pressure liquid chromatography using "Nucleosil $5C_{18}$".

Example 3

Ditrisarubicin B (50 mg) was dissolved in 5 ml 0.1N HCl and the solution was left to stand at 70°C for 15 minutes. The solution was put on a column packed with 10 g of silica gel ("Kiesel Gel 50" of Merck Co.), which was washed with 50 ml of chloroform/methanol (100:1) and eluted with chloroform/methanol (100:5). The resulting eluates were concentrated and spotted on 10 thin layers of silica gel ("Kiesel Gel 60 $F_{254}$" of Merck Co.) that were 0.25 mm thick and 20 × 20 cm large. After development with a mixture of chloroform, methanol and conc. aqueous ammonia (100:10:0.05), the separating ditrisarubicin B/7 de-dF-CB and B/10 de-dF-CB were collected and eluted with a chloroform/methanol (1:1) mixture. The so-obtained fractions were concentrated and purified by HPLC. Separation was conducted on a column (20 ϕ × 300 mm) of "Nucleosil $5C_{18}$" (Nagel Co.) with a solvent system of acetonitrile/0.45% aq. phosphoric acid.

The respective eluates were neutralized with sodium bicarbonate and extracted with chloroform. The chloroform layer was concentrated to dryness. The samples dissolved in a mixture of chloroform/methanol (1:1) were put on columns (2.0 ϕ × 28 cm) of "Cephadex LH-20" that had been equilibrated with the same solvent mixture. Upon elution with the same solvent mixture and concentration under vacuum to dryness, ditrisarubicin B/7 de-dF-CB and B/10 de-dF-CB were obtained in respective amounts of 3.2 mg and 4.7 mg. Each of these samples was found to consist of a single component upon analysis by TLC on silica gel plate and high-pressure liquid chromatography using "Nucleosil $5C_{18}$".

Example 4

Fifty milligrams of ditrisarubicin G and GR was reacted with 0.1N HCl at 30°C for 20 minutes. After extraction, the extracts were subjected to silica gel chromatography. After removing DTR-G or DTR-GR with $CHCl_3$/MeOH (100:3) mixture, elution was conducted with $CHCl_3$/MeOH (100:5) and the respective eluates were concentrated under vacuum to dryness. Solutions of the residues were passed through a column of "Cephadex LH-20" and eluted with $CHCl_3$/MeOH (1:1) mixture to obtain DTR-G/de-Ro and DTR-GR/de-Ro in respective amounts of 25 mg and 15 mg. Upon analysis by TLC and HPLC, each of the samples thusly obtained was found to consist of a single component.

Example 5

Eight hundred mg of a mixture of DTR-B, C, CR, serirubicin (SR) and 1-hydroxyserirubicin (HSR) was dissolved in a small amount of chloroform, and the resulting solution was spotted on 80 thin layers of silica gel (Kiesel Gel 60 $F_{254}$" of Merck Co.) that were 0.25 mm thick and 20 × 20 cm larger. After development with a mixture of chloroform, methanol and conc. aqueous ammonia (100:5:0.02), zones for DTR-C, SR and HSR were collected and eluted with a chloroform/methanol (10:1) mixture. After concentration to dryness, solutions of the residues were spotted on 20 thin layers of the same silica gel and developed with a mixture of ethyl acetate, methanol and conc. aqueous ammonia (25:1:0.08). The spots were collected, eluted, concentrated and subjected to another TLC on three thin layers of the same silica gel, followed by elution with a mixture of chloroform, methanol and conc. aqueous ammonia (100:5:0.02) in order to remove the small amount of residual DTR-B. After concentrating the eluated to dryness, an HSR fraction was obtained by column chromatography on "Cephadex LH-20" (eluant: MeOH) and concentrated under vacuum to dryness so as to obtain 13.7 mg of HSR as a red orange powder. The thusly obtained sample was found to consist of a single component upon analysis by TLC and HPLC (reverse phase).

Brief Description of the Drawings

Figs. 1 to 25 are IR, NMR and UV spectra charts for compounds (*1*) to (*11*), as specified below:

| Fig. No. | Spectrum | Compound |
|---|---|---|
| 1 | IR | *(1)* |
| 2 | NMR | *(1)* |
| 3 | IR | *(2)* |
| 4 | NMR | *(2)* |
| 5 | IR | *(3)* |
| 6 | NMR | *(3)* |
| 7 | UV | *(4)* |
| 8 | IR | *(4)* |
| 9 | NMR | *(4)* |
| 10 | UV | *(5)* |
| 11 | IR | *(5)* |
| 12 | NMR | *(5)* |
| 13 | IR | *(6)* |
| 14 | NMR | *(6)* |
| 15 | IR | *(7)* |
| 16 | NMR | *(7)* |
| 17 | IR | *(8)* |
| 18 | NMR | *(8)* |
| 19 | IR | *(9)* |
| 20 | NMR | *(9)* |
| 21 | IR | *(10)* |
| 22 | NMR | *(10)* |
| 23 | UV | *(11)* |
| 24 | IR | *(11)* |
| 25 | NMR | *(11)* |

Fig. 26 is a flowsheet showing an outline for the procedures of determining the structures of the anthracycline compounds of the present invention.

Figs. 27 to 34 are NMR spectrum charts for the decomposition products obtained during the process for determining the structures of the anthracycline compounds of the present invention.

Fig. 35 shows the structures of compound *(11)*, and Fig. 36 shows the structures of the respective sugars in that compound.

**Claims for the Contracting States: BE CH DE FR GB IT LI LU NL SE**

1. A compound of the formula I or an acid addition salt thereof

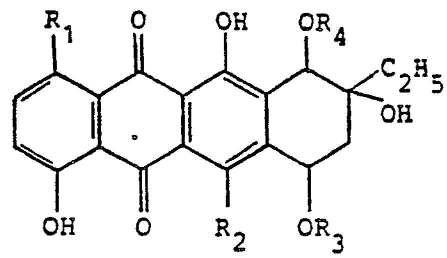

(I)

wherein $R_1$ is hydrogen, $R_2$ is hydroxy and $R_3$ and $R_4$ represent any of the following eight sugar combinations:

| $R_3$ | $R_4$ |
|---|---|
| (I) | (V) |
| (II) | (II) |
| (I) | (VI) |
| (I) | (III) |
| (IV) | (I) |
| (III) | (I) |
| (I) | (VII) |
| (VII) | (I) |

or $R_1$ is hydroxy, $R_2$ is hydrogen and $R_3$ and $R_4$ represent each the sugar combination (I), wherein the symbols (I) to (VII) represent the following sugar chains:

(I) RN-dF-CB

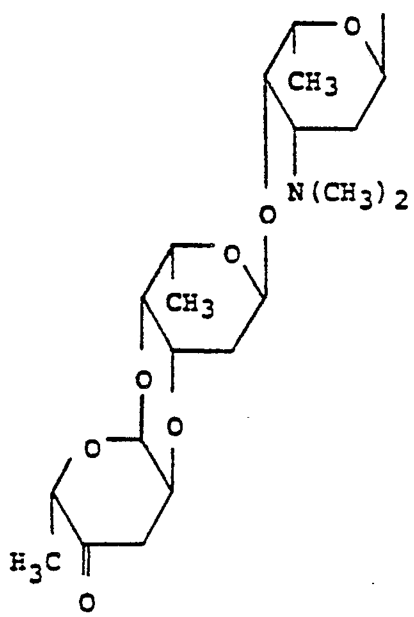

(II) RN-dF-CA

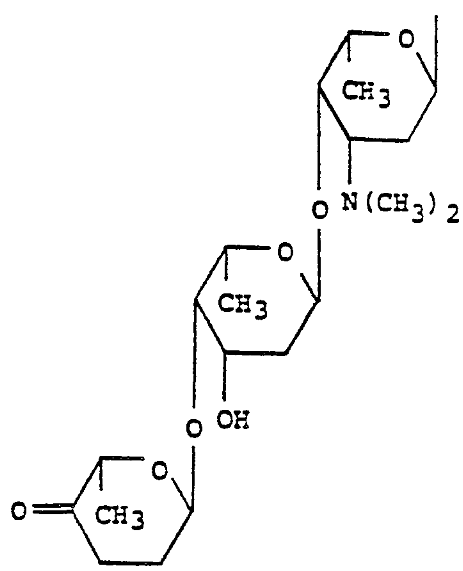

(III) RN-dF-Ro

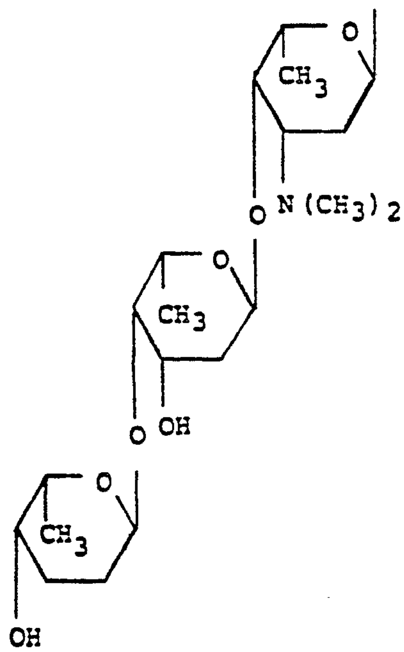

(IV) RN-Ro-Ac

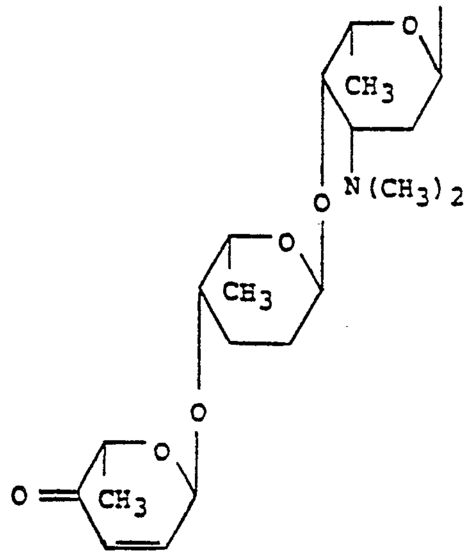

(V) RN-Ro-CA

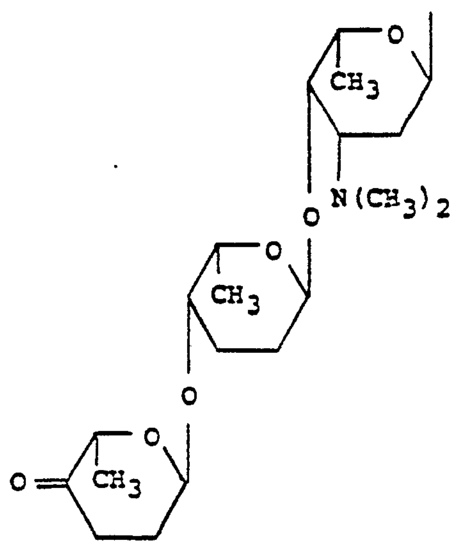

(VI) RN-Ro-Ro

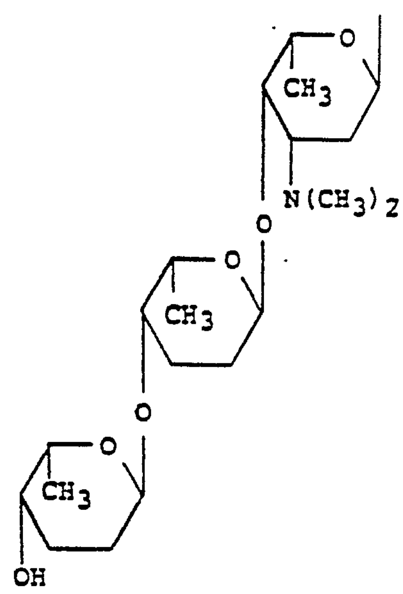

(VII) RN-dF

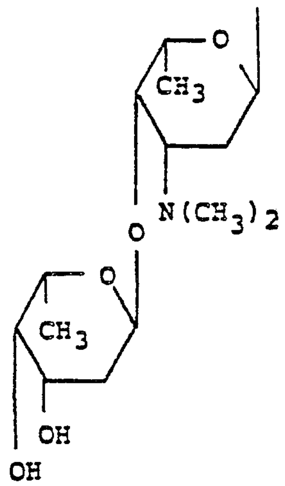

(RN: rhodasamine, dF: 2-deoxyfucose, CB: cinerulose B, CA: cinerulose A, Ac: aculose, Ro: rhodinose).

2. A process for the preparation of compounds of the formula I or an acid addition salt thereof

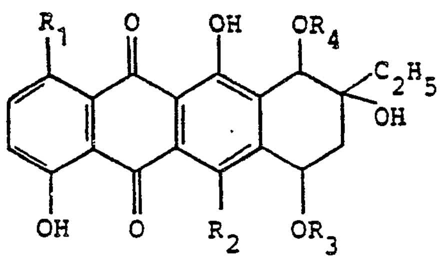

(I)

wherein $R_1$ is hydrogen, $R_2$ is hydroxy and $R_3$ and $R_4$ represent any of the following eight sugar combinations:

| $R_3$ | $R_4$ |
|---|---|
| (I) | (V) |
| (II) | (II) |
| (I) | (VI) |
| (I) | (III) |
| (IV) | (I) |
| (III) | (I) |
| (I) | (VII) |
| (VII) | (I) |

or $R_1$ is hydroxy, $R_2$ is hydrogen and $R_3$ and $R_4$ represent each the sugar combination (I), wherein the symbols (I) to (VII) represent the following sugar chains:

(I) RN-dF-CB

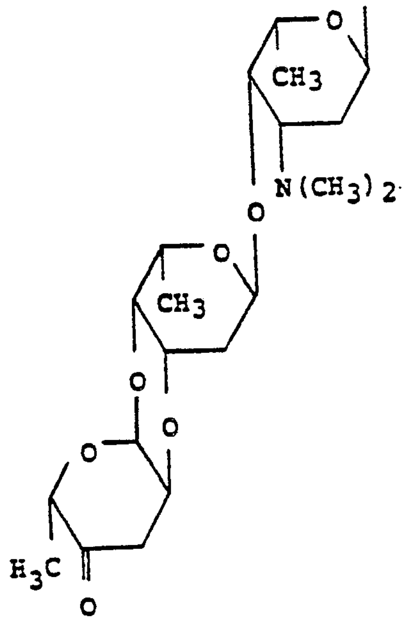

(II) RN-dF-CA

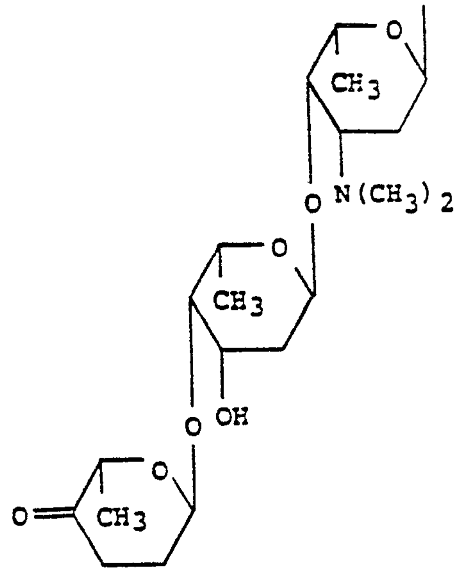

(III) RN-dF-Ro

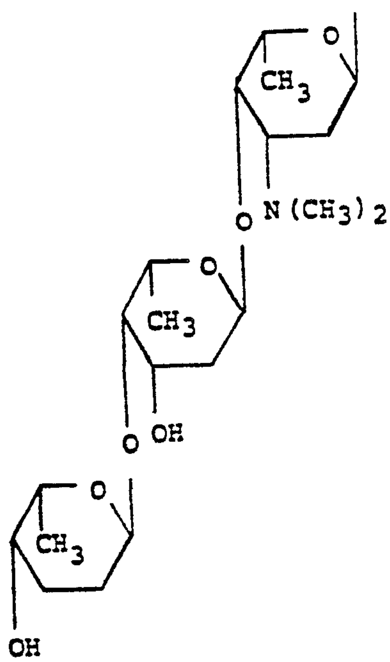

(IV) RN-Ro-Ac

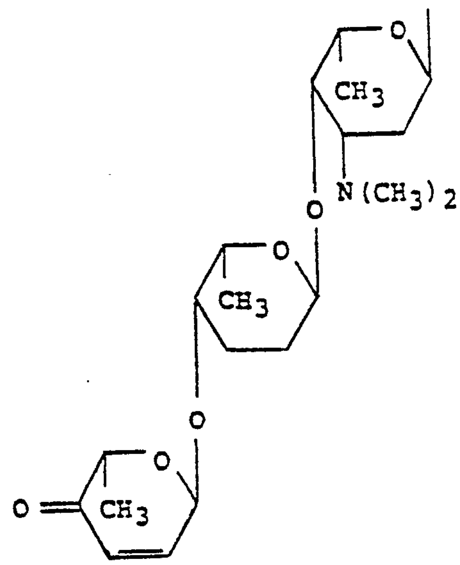

(V) RN-Ro-CA

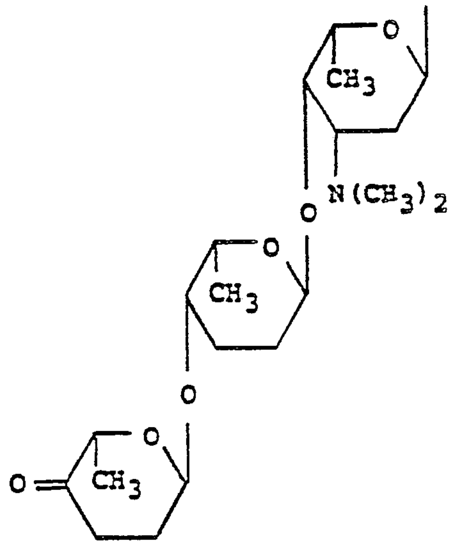

(VI) RN-Ro-Ro

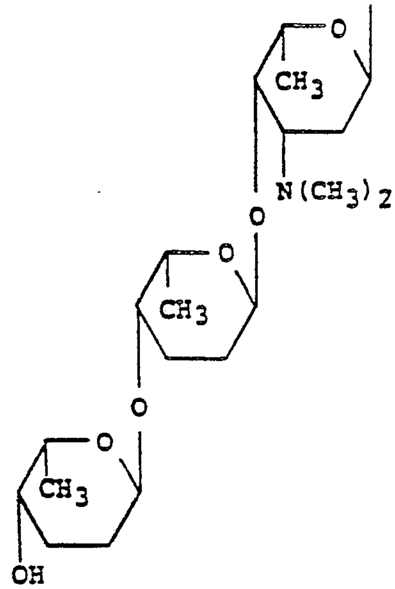

(VII) RN-dF

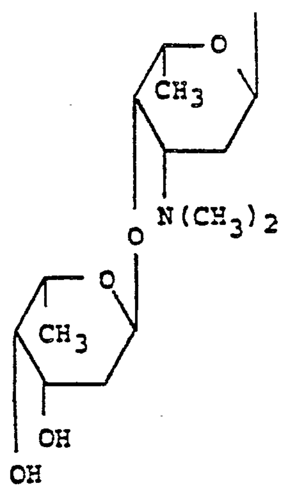

(RN: rhodasamine, dF: 2-deoxyfucose, CB: cinerulose B, CA: cinerulose A, Ac: aculose, Ro: rhodinose).

which comprises cultivating a strain of the genus Streptomyces being capable of producing the anthracycline compounds of the present invention, in a suitable culture medium under aerobic conditions and subsequently recovering the compounds of the formula I from the cultured medium.

3. The process as claimed in claim 2 wherein the culture medium is a liquid submerged culture consisting of nutritional carbon sources, nitrogen sources and optionally inorganic salts and/or antifoaming agents at a temperature from 20 to 35°C and the produced compounds are recovered from the cultured medium by usual extraction or adsorption methods.

4. The process as defined in claims 2 and 3 wherein the strain *Streptomyces cyaneus* MG 344—hF 49 (FERM BP—314) was used.

5. A pharmaceutical composition containing as its active ingredient a compound of the formula I or an acid addition salt thereof as defined in claim 1 in association with a pharmaceutically acceptable carrier.

6. The use of a compound of the formula I or an acid addition salt thereof as defined in claim 1 for the preparation of a medicament having anti-tumor activities.

**Claims for the Contracting State: AT**

1. A process for the preparation of compounds of the formula I or an acid addition salt thereof

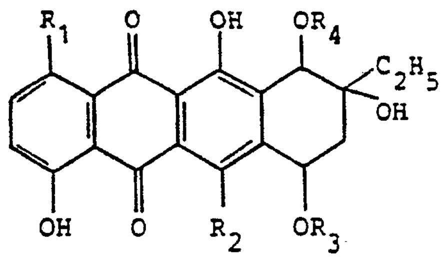

(I)

wherein $R_1$ is hydrogen, $R_2$ is hydroxy and $R_3$ and $R_4$ represent any of the following eight sugar combinations:

| $R_3$ | $R_4$ |
|---|---|
| (I) | (V) |
| (II) | (II) |
| (I) | (VI) |
| (I) | (III) |
| (IV) | (I) |
| (III) | (I) |
| (I) | (VII) |
| (VII) | (I) |

or $R_1$ is hydroxy, $R_2$ is hydrogen and $R_3$ and $R_4$ represent each the sugar combination (I), wherein the symbols (I) to (VII) represent the following sugar chains:

(I) RN-dF-CB

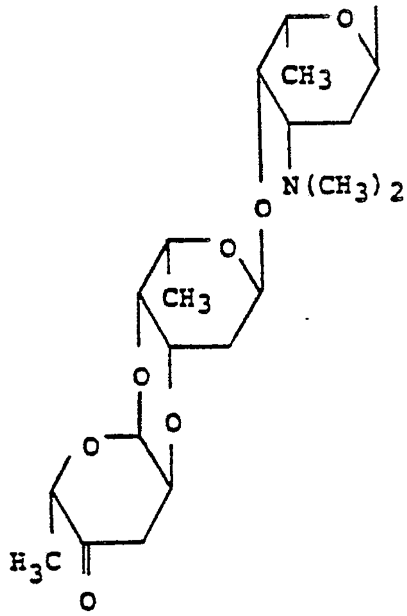

(II) RN-dF-CA

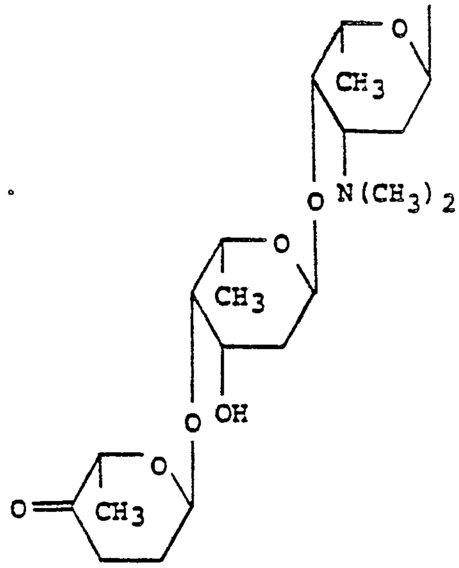

(III) RN-dF-Ro

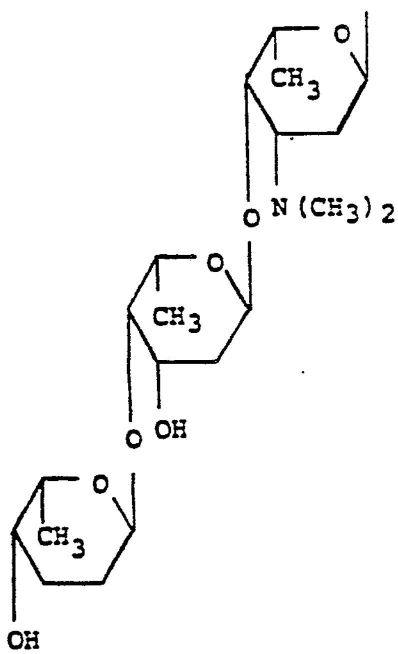

(IV) RN-Ro-Ac

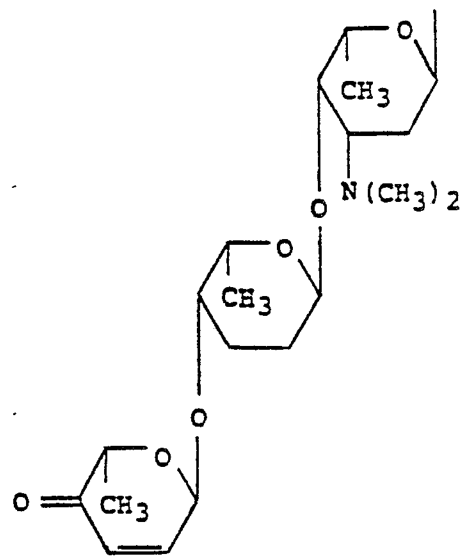

(V) RN-Ro-CA

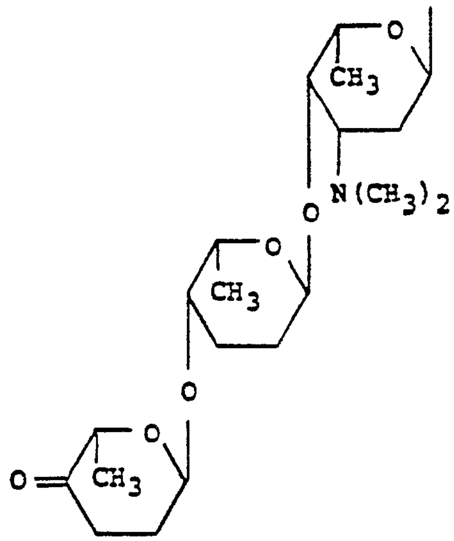

(VI) RN-Ro-Ro

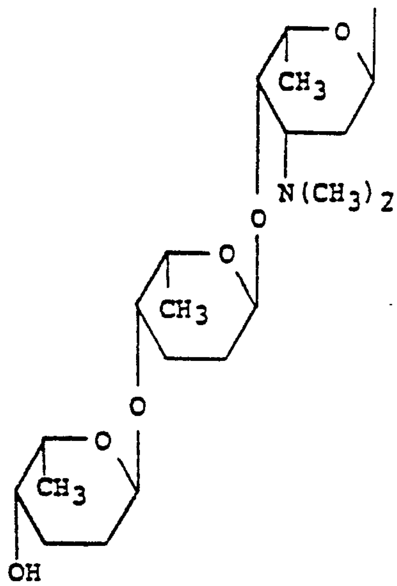

(VII) RN-dF

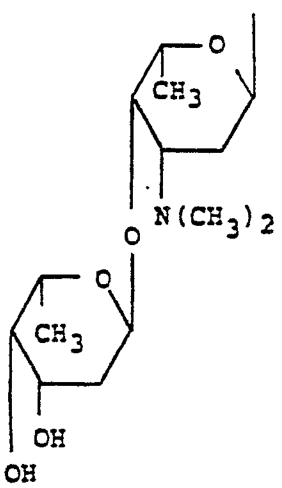

(RN: rhodasamine, dF: 2-deoxyfucose, CB: cinerulose B, CA: cinerulose A, Ac: aculose, Ro: rhodinose).

which comprises cultivating a strain of the genus Streptomyces being capable of producing the anthracycline compounds of the present invention, in a suitable culture medium under aerobic conditions and subsequently recovering the compounds of the formula I from the cultured medium.

2. The process as claimed in claim 1 wherein the culture medium is a liquid submerged culture consisting of nutritional carbon sources, nitrogen sources and optionally inorganic salts and/or antifoaming agents at a temperature from 20 to 35°C and the produced compounds are recovered from the cultured medium by usual extraction or adsorption methods.

3. The process as defined in claims 1 and 2 wherein the strain *Streptomyces cyaneus* MG 344—hF 49 (FERM BP—314) was used.

**Patentansprüche für die Vertragsstaaten: BE CH DE FR GB IT LI LU NL SE**

1. Eine Verbindung der Formel I oder eines ihrer Säureadditionssalze,

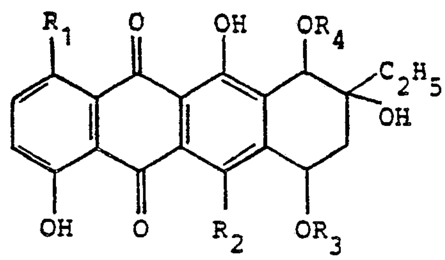

(I)

worin $R_1$ Wasserstoff, $R_2$ Hydroxyl bedeutet und $R_3$ und $R_4$ irgendeine der folgenden 8 Zuckerkombinationen darstellen:

| $R_3$ | $R_4$ |
|---|---|
| (I) | (V) |
| (II) | (II) |
| (I) | (VI) |
| (I) | (III) |
| (IV) | (I) |
| (III) | (I) |
| (I) | (VII) |
| (VII) | (I) |

oder $R_1$ Hydroxyl und $R_2$ Wasserstoff bedeutet und $R_3$ und $R_4$ beide die Zuckerkombination (I) darstellen, worin die Symbole (I) bis (VII) die folgenden Zuckerketten darstellen:

(I) RN-dF-CB

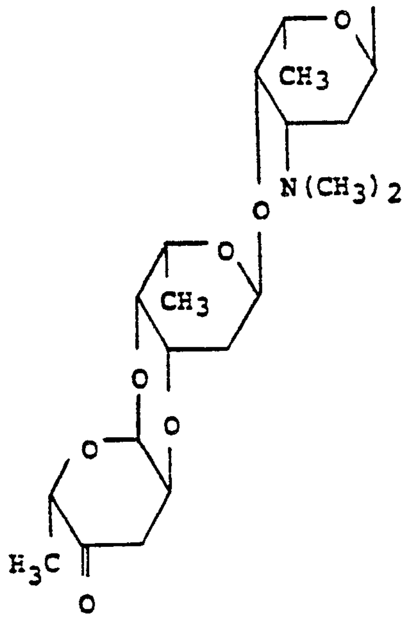

(II) RN-dF-CA

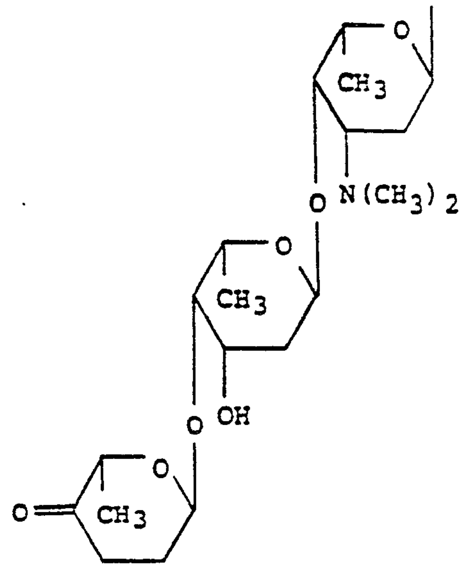

(III) RN-dF-Ro

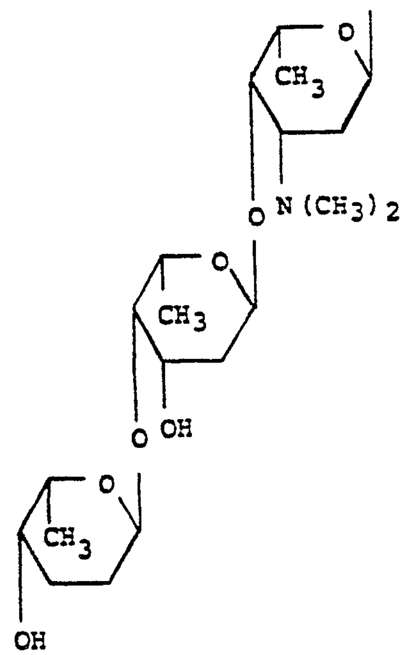

(IV) RN-Ro-Ac

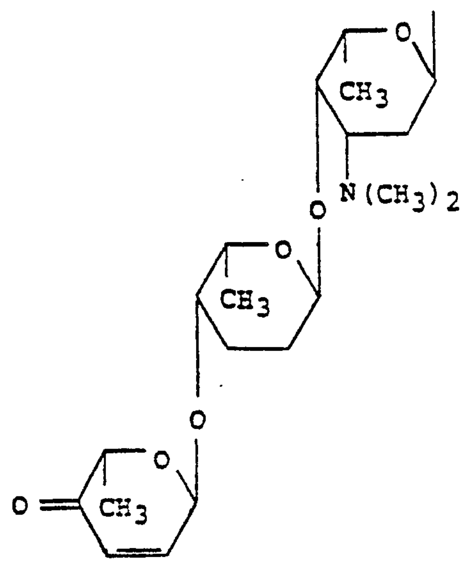

(V) RN-Ro-CA

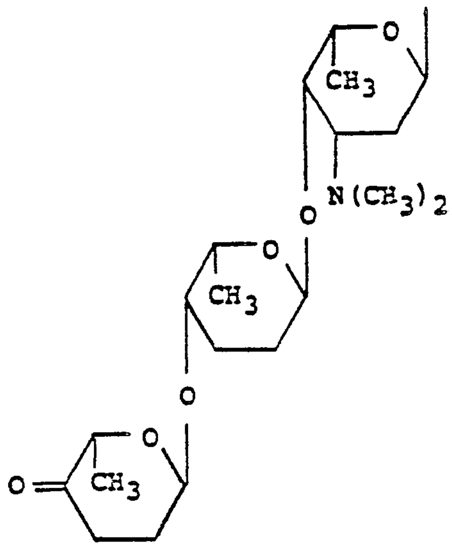

(VI) RN-Ro-Ro

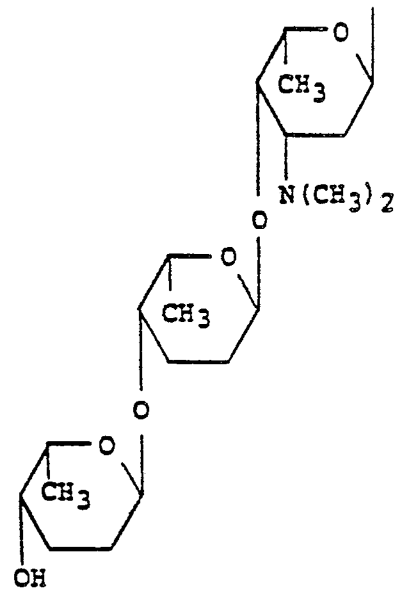

(VII) RN-dF

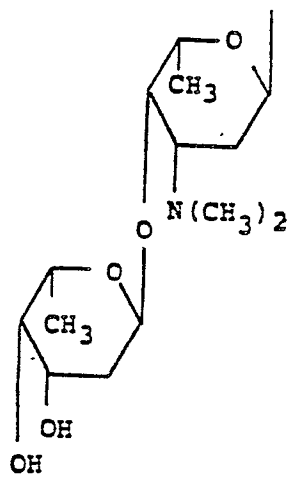

(RN: Rhodasamin, dF: 2-Desoxyfucose, CB: Cinerulose B, CA: Cinerulose A, Ac: Aculose, Ro: Rhodinose).

2. Ein Verfahren zur Herstellung von Verbindungen der Formel I oder eines ihrer Säureadditionssalze,

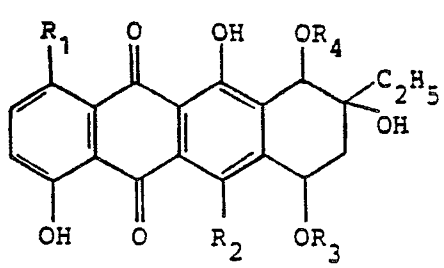

(I)

worin $R_1$ Wasserstoff, $R_2$ Hydroxyl bedeutet und $R_3$ und $R_4$ irgendeine der folgenden 8 Zuckerkombinationen darstellen:

| $R_3$ | $R_4$ |
|---|---|
| (I) | (V) |
| (II) | (II) |
| (I) | (VI) |
| (I) | (III) |
| (IV) | (I) |
| (III) | (I) |
| (I) | (VII) |
| (VII) | (I) |

oder $R_1$ Hydroxyl und $R_2$ Wasserstoff bedeutet und $R_3$ und $R_4$ beide die Zuckerkombination (I) darstellen, worin die Symbole (I) bis (VII) die folgenden Zuckerketten darstellen:

(I) RN-dF-CB

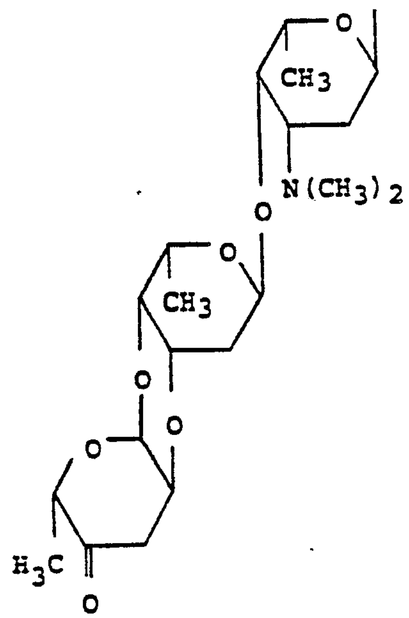

(II) RN-dF-CA

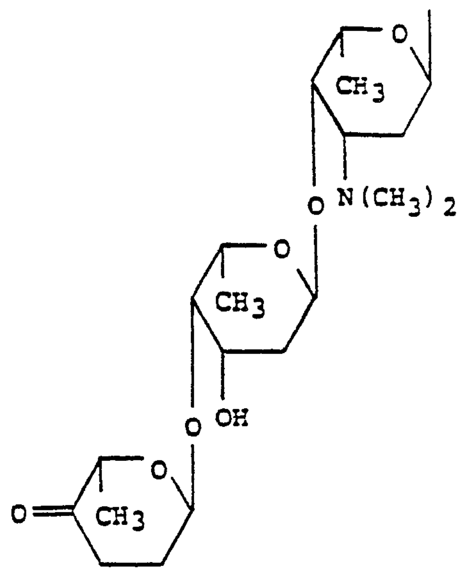

(III) RN-dF-Ro

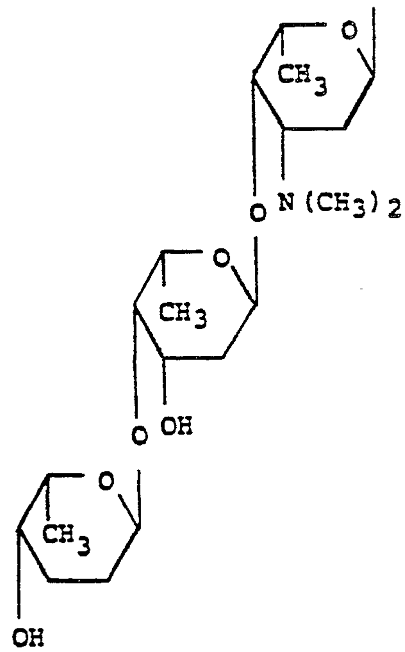

(IV) RN-Ro-Ac

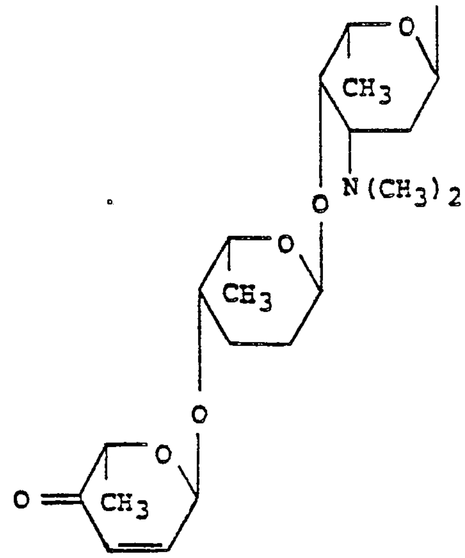

(V) RN-Ro-CA

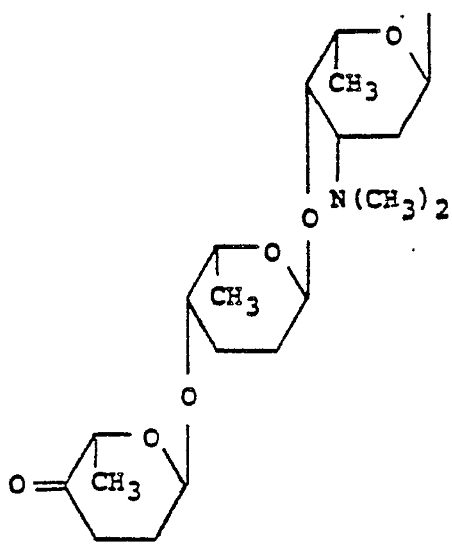

(VI) RN-Ro-Ro

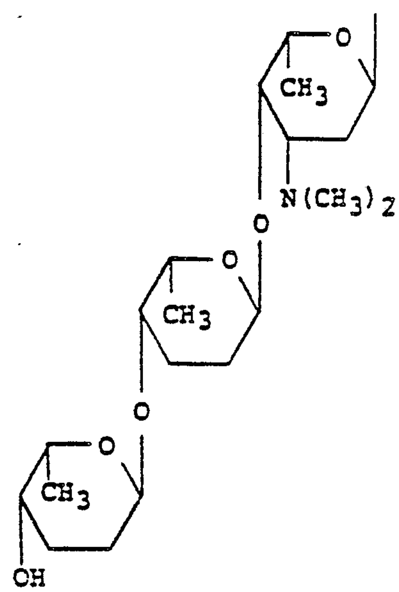

(VII) RN-dF

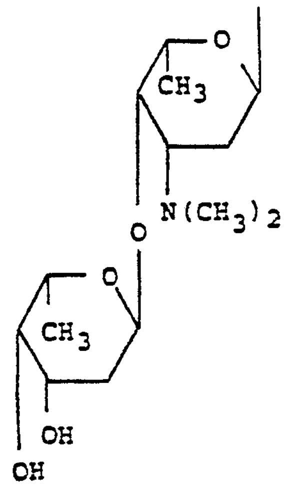

(RN: Rhodasamin, dF: 2-Desoxyfucose, CB: Cinerulose B, CA: Cinerulose A, Ac: Aculose, Ro: Rhodinose).

gekennzeichnet durch die Zucht eines Stammes der Gattung Streptomyces, der zur Bildung der Anthracyclinverbindungen gemäß der vorliegenden Erfindung geeignet ist, in einem geeigneten Nährboden unter aeroben Bedingungen und die darauffolgende Wiedergewinnung der Verbindungen der Formel I aus dem Nährboden, in dem die Zucht erfolgte.

3. Das wie in Anspruch 2 beanspruchte Verfahren, worin der Nährboden eine Flüssigsubmerskultur ist, die aus Kohlenstoffnahrungsquellen, Stickstoffquellen und wahlweise anorganischen Salzen und/oder Antischaummitteln besteht, bei einer Temperatur von 20 bis 35°C, und die erzeugten Verbindung aus dem Nährboden, in dem die Zucht erfolgte mit üblichen Extraktions- oder Adsorptionsmethoden wiedergewonnen wird.

4. Das wie in den Ansprüchen 2 und definierte Verfahren, worin der Stamm *Streptomyces cyaneus* MG 344-hF 49 (FERM BP—314) verwendet wurde.

5. Eine pharmazeutische Zusammensetzung, die als Wirkstoff eine Verbindung der Formel I oder eines ihrer Säureadditionssalze, wie in Anspruch 1 definiert, zusammen mit einem pharmazeutisch unbedenklichen Träger enthält.

6. Die Verwendung einer Verbindung der Formel I oder eines ihrer Säureadditionssalze, wie in Anspruch 1 definiert, für die Herstellung eines Medikaments mit antitumoriellen Wirkungen.

**Patentansprüche für den Vertragsstaat: AT**

1. Ein Verfahren zur Herstellung von Verbindung der Formel I oder eines ihrer Säureadditionssalze,

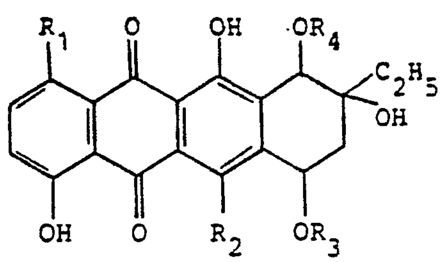

(I)

worin $R_1$ Wasserstoff, $R_2$ Hydroxyl bedeutet und $R_3$ und $R_4$ irgendeine der folgenden 8 Zuckerkombinationen darstellen:

| $R_3$ | $R_4$ |
|---|---|
| (I) | (V) |
| (II) | (II) |
| (I) | (VI) |
| (I) | (III) |
| (IV) | (I) |
| (III) | (I) |
| (I) | (VII) |
| (VII) | (I) |

oder $R_1$ Hydroxyl und $R_2$ Wasserstoff bedeutet und $R_3$ und $R_4$ beide die Zuckerkombination (I) darstellen, worin die Symbole (I) bis (VII) die folgenden Zuckerketten darstellen:

(I) RN-dF-CB

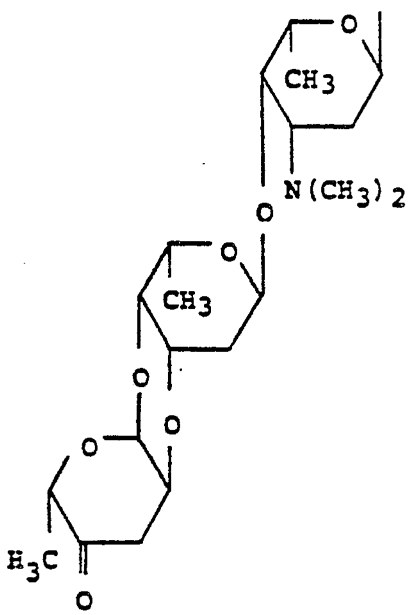

(II) RN-dF-CA

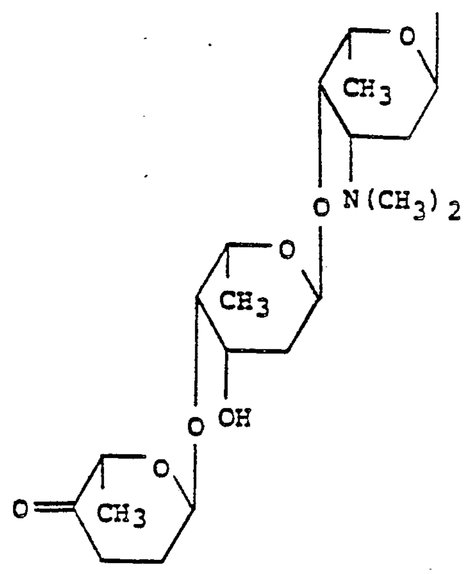

(III) RN-dF-Ro

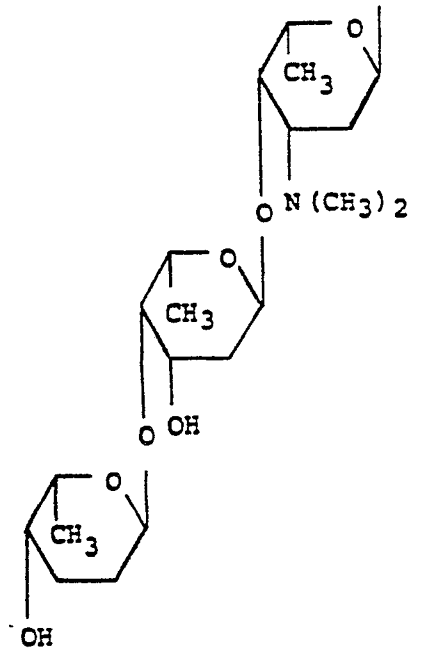

(IV) RN-Ro-Ac

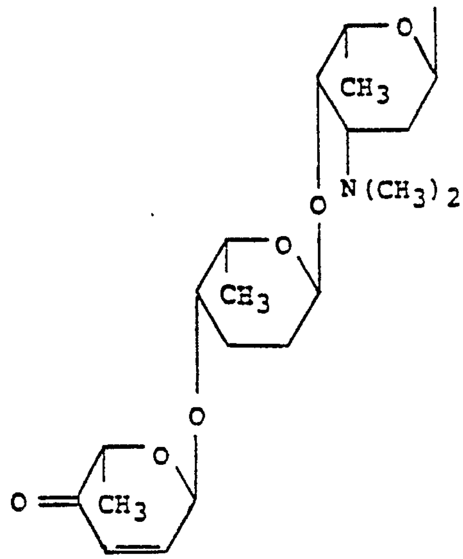

(V) RN-Ro-CA

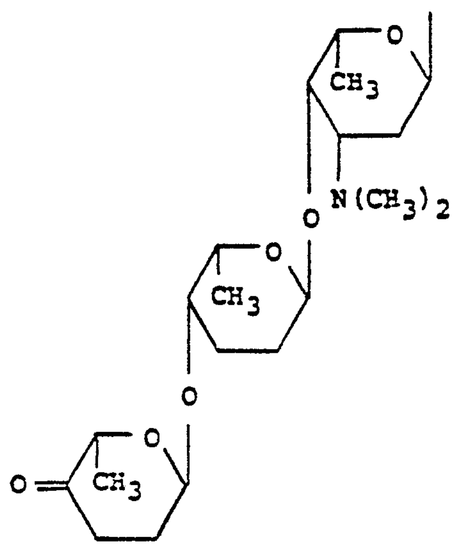

(VI) RN-Ro-Ro

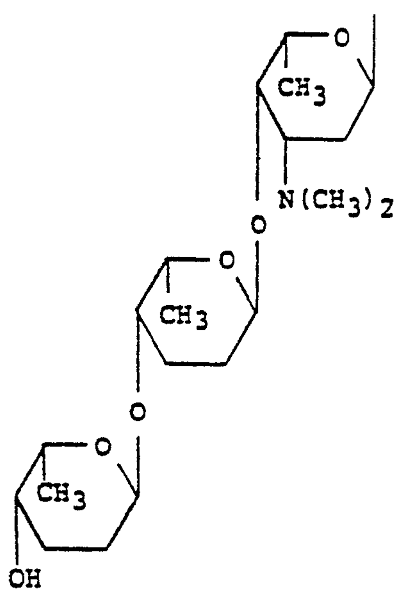

(VII) RN-dF

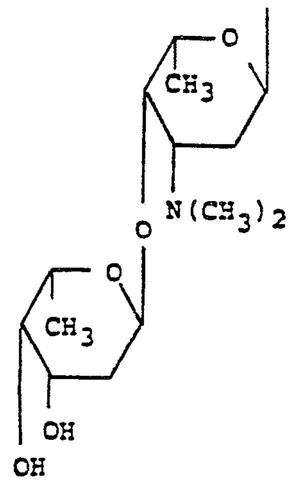

(RN: Rhodasamin, dF: 2-Desoxyfucose, CB: Cinerulose B, CA: Cinerulose A, Ac: Aculose, Ro: Rhodinose).

gekennzeichnet durch die Zucht eines Stammes der Gattung Streptomyces, der zur Bildung der Anthracyclinverbindungen gemäß der vorliegenden Erfindung geeignet ist, in einem geeigneten Nährboden unter aeroben Bedingungen und die darauffolgende Wiedergewinnung der Verbindungen der Formel I aus dem Nährboden, in dem die Zucht erfolgte.

2. Das wie in Anspruch 1 beanspruchte Verfahren, worin der Nährboden eine Flüssigsubmerskultur ist, die aus Kohlenstoffnahrungsquellen, Stickstoffquellen und wahlweise anorganischen Salzen und/oder Antischaummitteln besteht, bei einer Temperatur von 20 bis 35°C, und die erzeugten Verbindungen aus dem Nährboden, in dem die Zucht erfolgte mit üblichen Extraktions- oder Adsorptionsmethoden wiedergewonnen wird.

3. Das wie in den Ansprüchen 1 und 2 definierte Verfahren, worin der Stamm *Streptomyces cyaneus* MG 344-hF 49 (FERM BP—314) verwendet wurde.

**Revendications pour les Etats contractants: BE CH DE FR GB IT LI LU NL SE**

1. Composé de formule I, ou un sel d'addition d'acide des celui-ci

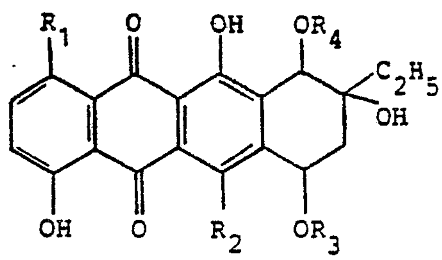

(I)

où $R_1$ est un hydrogène, $R_2$ est un hydroxy et $R_3$ et $R_4$ représentent l'une quelconque des huit combinaisons de sucres suivantes:

| $R_3$ | $R_4$ |
|---|---|
| (I) | (V) |
| (II) | (II) |
| (I) | (VI) |
| (I) | (III) |
| (IV) | (I) |
| (III) | (I) |
| (I) | (VII) |
| (VII) | (I) |

ou $R_1$ est un hydroxy, $R_2$ est un hydrogène et $R_3$ et $R_4$ représentent chacun la combinaison de sucres (I) où les symboles (I) à (VII) représentent les chaînes de sucres suivantes:

(I) RN-dF-CB

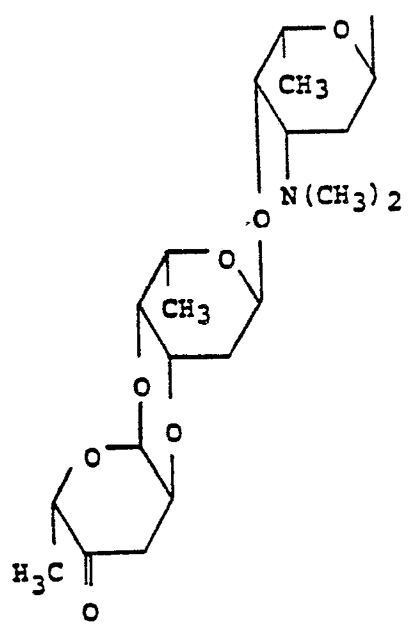

(II) RN-dF-CA

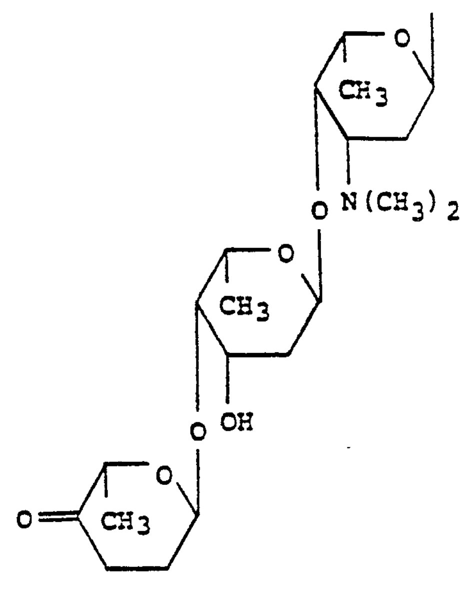

(III) RN-dF-Ro

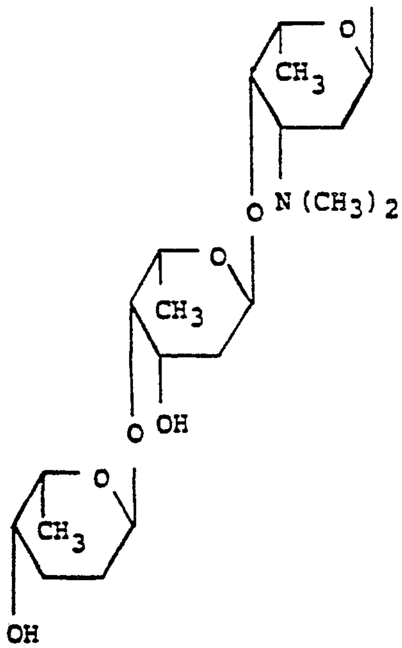

(IV) RN-Ro-Ac

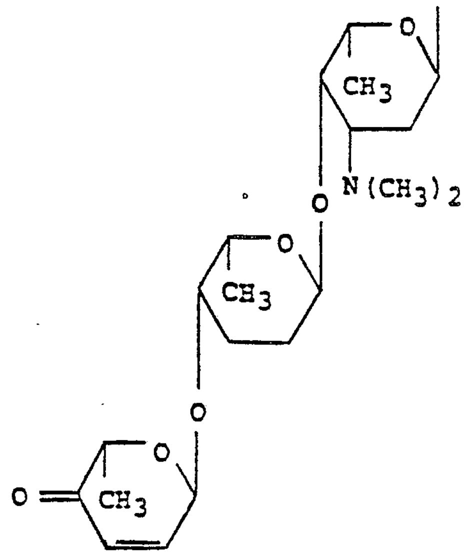

(V) RN-Ro-CA

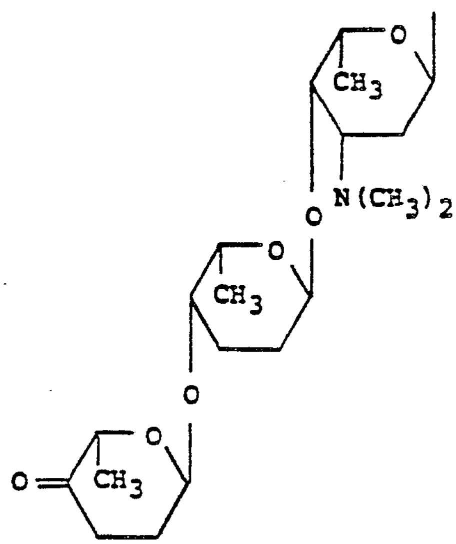

(VI) RN-Ro-Ro

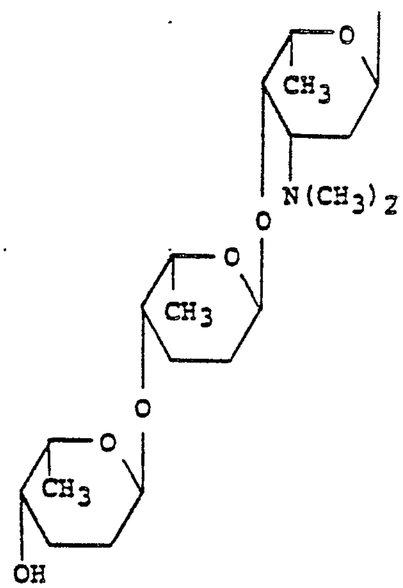

(VII) RN-dF

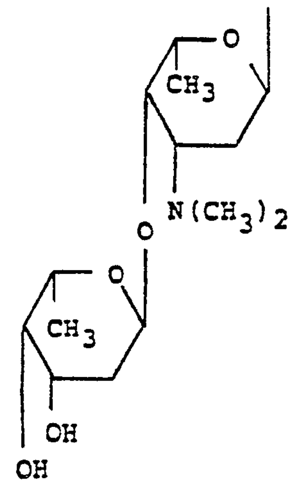

(RN: rhodosamine, dF: 2-désoxyfucose, CB: cinérulose B, CA: cinérulose A, Ac: aculose, Ro: rhodinose).

2. Procédé pour préparer les composés de formule I ou leurs sels d'additions d'acides

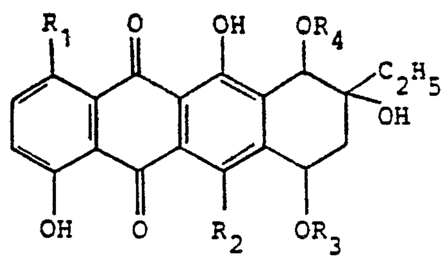

(I)

où $R_1$ est un hydrogène, $R_2$ est un hydroxy et $R_3$ et $R_4$ représentent l'une quelconque des huit combinaisons de sucres suivantes:

| $R_3$ | $R_4$ |
|---|---|
| (I) | (V) |
| (II) | (II) |
| (I) | (VI) |
| (I) | (III) |
| (IV) | (I) |
| (III) | (I) |
| (I) | (VII) |
| (VII) | (I) |

ou $R_1$ est un hydroxy, $R_2$ est un hydrogène et $R_3$ et $R_4$ représentent chacun la combinaison de sucres (I) où les symboles (I) à (VII) représentent les chaînes de sucres suivantes:

(I) RN-dF-CB

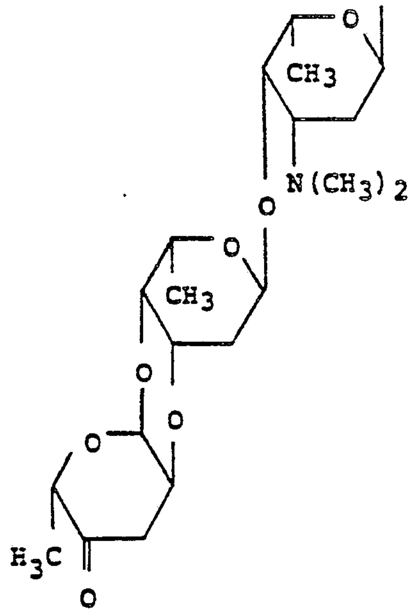

(II) RN-dF-CA

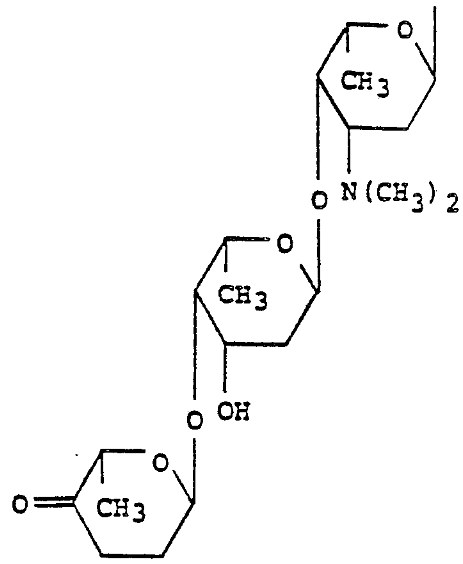

(III) RN-dF-Ro

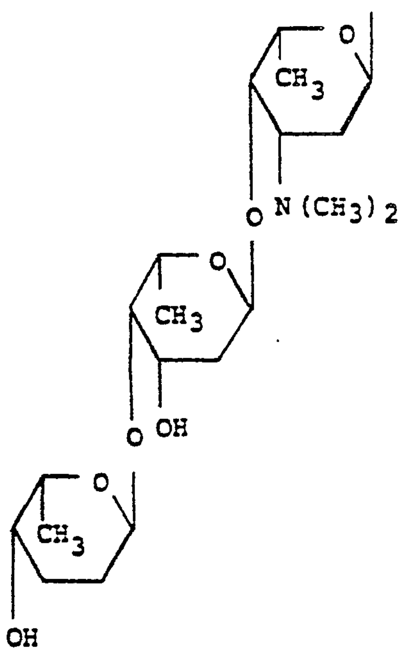

(IV) RN-Ro-Ac

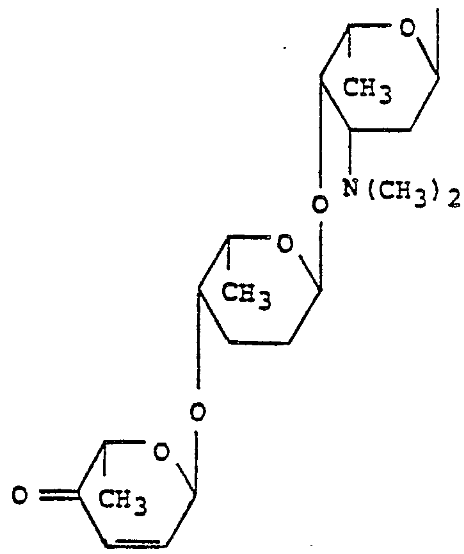

(V) RN-Ro-CA

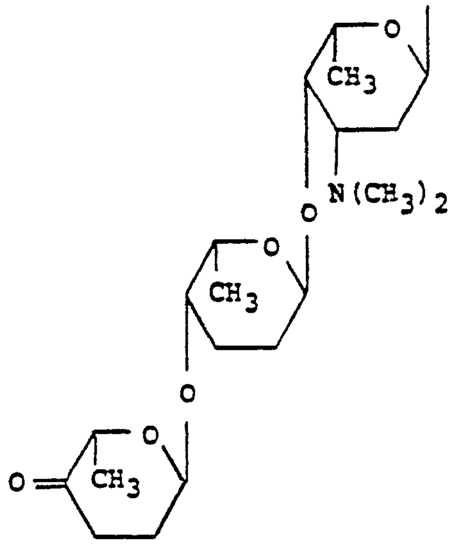

(VI) RN-Ro-Ro

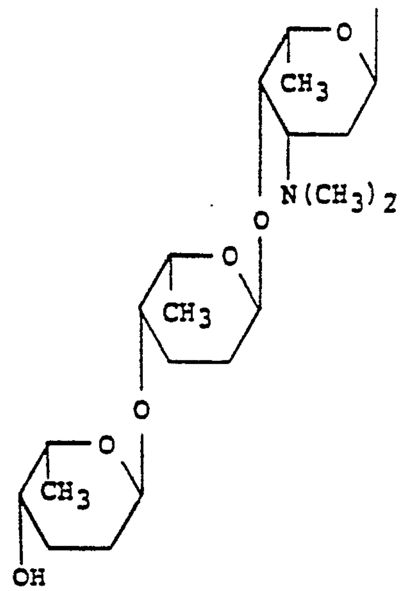

(VII) RN-dF

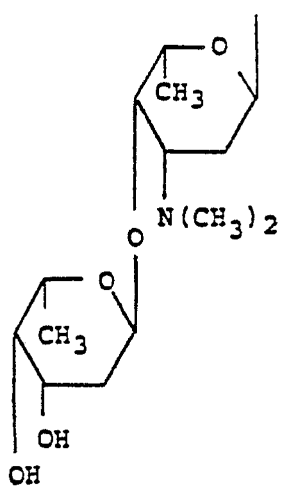

(RN: rhodosamine, dF: 2-désoxyfucose, CB: cinérulose B, CA: cinérulose A, Ac: aculose, Ro: rhodinose).

qui comprend la culture d'une souche du genre Streptomyces capable de produire les dérivés d'anthracycline de la présente invention dans un milieu de culture approprié en conditions aérobies, puis la récupération des composés de formule I à partir du milieu de culture.

3. Procédé selon la revendiction 2, dans lequel le milieu de culture est un milieu de culture submergé liquide constitué de sources nutritives de carbone, de sources d'azote et facultativement de sels minéraux et/ou d'agents antimousses à une température de 20 à 35°C, les composés produits étant récupérés à partir du milieu de culture selon des procédés habituels d'extraction ou d'adsorption

4. Procédé selon l'une des revendications 2 et 3, dans lequel on utilise la souche *Streptomyces cyaneus* MG 344-hF (FERM BP—314).

5. Composition pharmaceutique contenant comme ingrédient actif un composé de formule I ou un sel d'addition d'acide de celui-ci comme défini dans la revendication 1 en association avec un véhicule pharmaceutiquement acceptable.

6. Utilisation d'un composé de formule I ou d'un de ses sels d'addition d'acides comme défini dans la revendication 1 pour la préparation d'un médicament ayant des activités antitumorales.

**Revendications pour l'Etat contractant: AT**

1. Procédé pour préparer les composés de formule I ou un de leurs sels d'addition d'acides

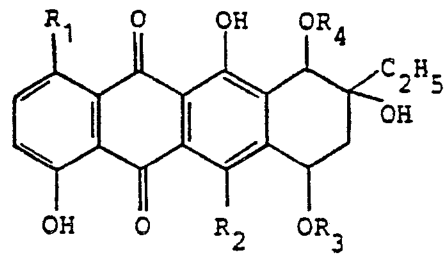

(I)

où $R_1$ est un hydrogène, $R_2$ est un hydroxy et $R_3$ et $R_4$ représentent l'une quelconque des huit combinaisons de sucres suivantes:

| $R_3$ | $R_4$ |
|---|---|
| (I) | (V) |
| (II) | (II) |
| (I) | (VI) |
| (I) | (III) |
| (IV) | (I) |
| (III) | (I) |
| (I) | (VII) |
| (VII) | (I) |

ou $R_1$ est un hydroxy, $R_2$ est un hydrogène et $R_3$ et $R_4$ représentent chacun la combinaison de sucres (I) où les symboles (I) à (VII) représentent les chaînes de sucres suivantes:

(I) RN-dF-CB

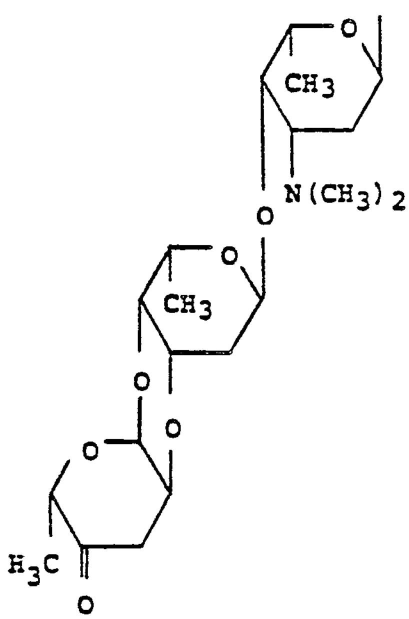

(II) RN-dF-CA

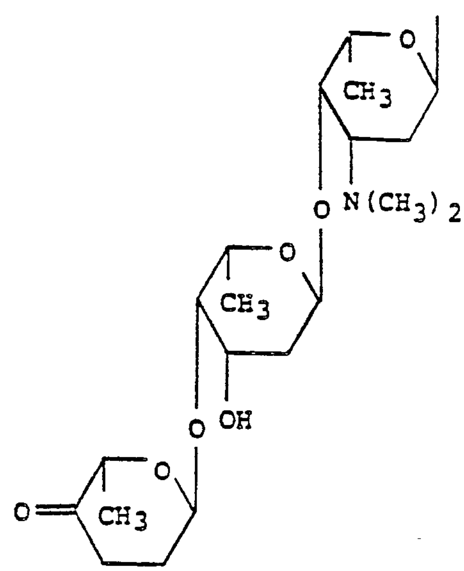

(III) RN-dF-Ro

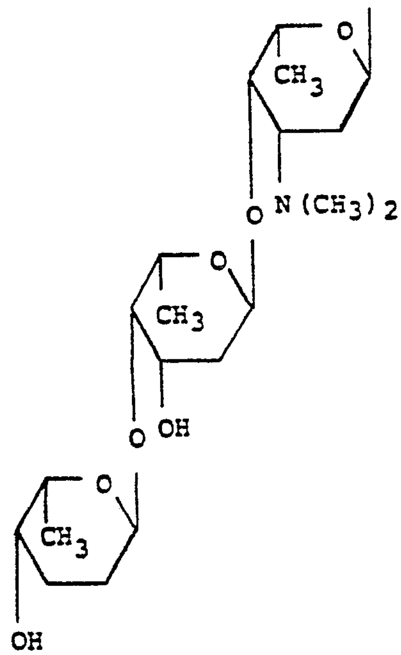

(IV) RN-Ro-Ac

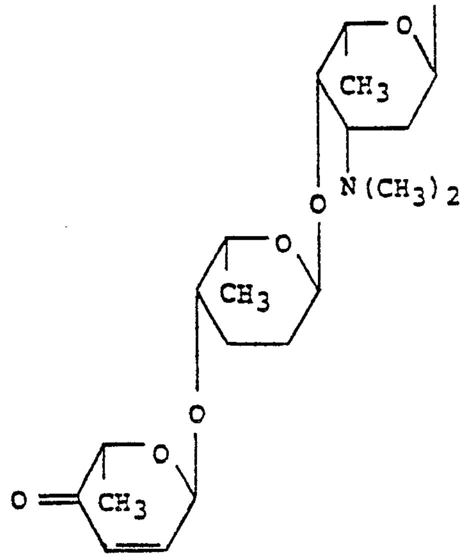

(V) RN-Ro-CA

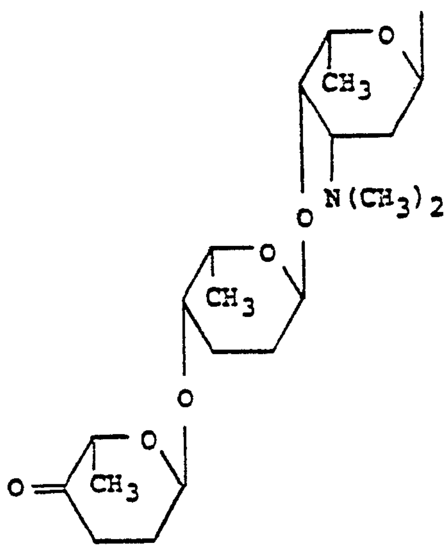

(VI) RN-Ro-Ro

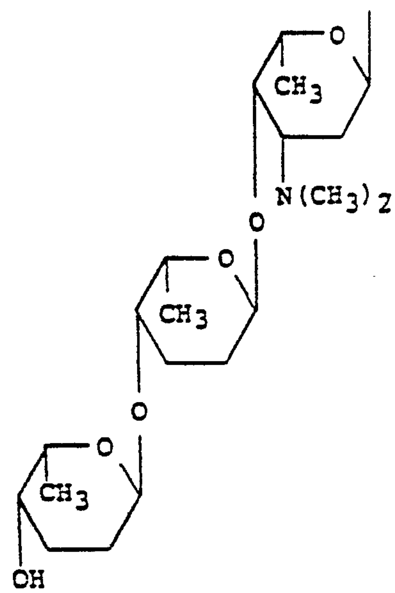

(VII) RN-dF

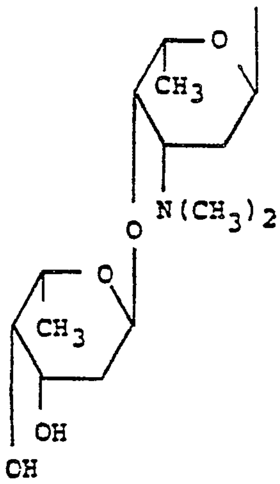

(RN: rhodosamine, dF: 2-désoxyfucose, CB: cinérulose B, CA: cinérulose A, Ac: aculose, Ro: rhodinose).

qui comprend la culture d'une souche du genre Streptomyces capable de produire les dérivés d'anthracycline de la présente invention dans un milieu de culture approprié en conditions aérobies, puis la récupération des composés de formule I à partir du milieu de culture.

3. Procédé selon la revendication 1, dans lequel le milieu de culture est un milieu de culture submergé liquide constitué de sources nutritives de carbone, de sources d'azote et facultativement de sels minéraux et/ou d'agents antimousses à une température de 20 à 35°C, les composés produits étant récupérés à partir du milieu de culture selon des procédés habituels d'extraction ou d'adsorption

4. Procédé selon l'une des revendications 1 et 2, dans lequel on utilise la souche *Streptomyces cyaneus* MG 344-hF (FERM BP—314).

FIG. 1

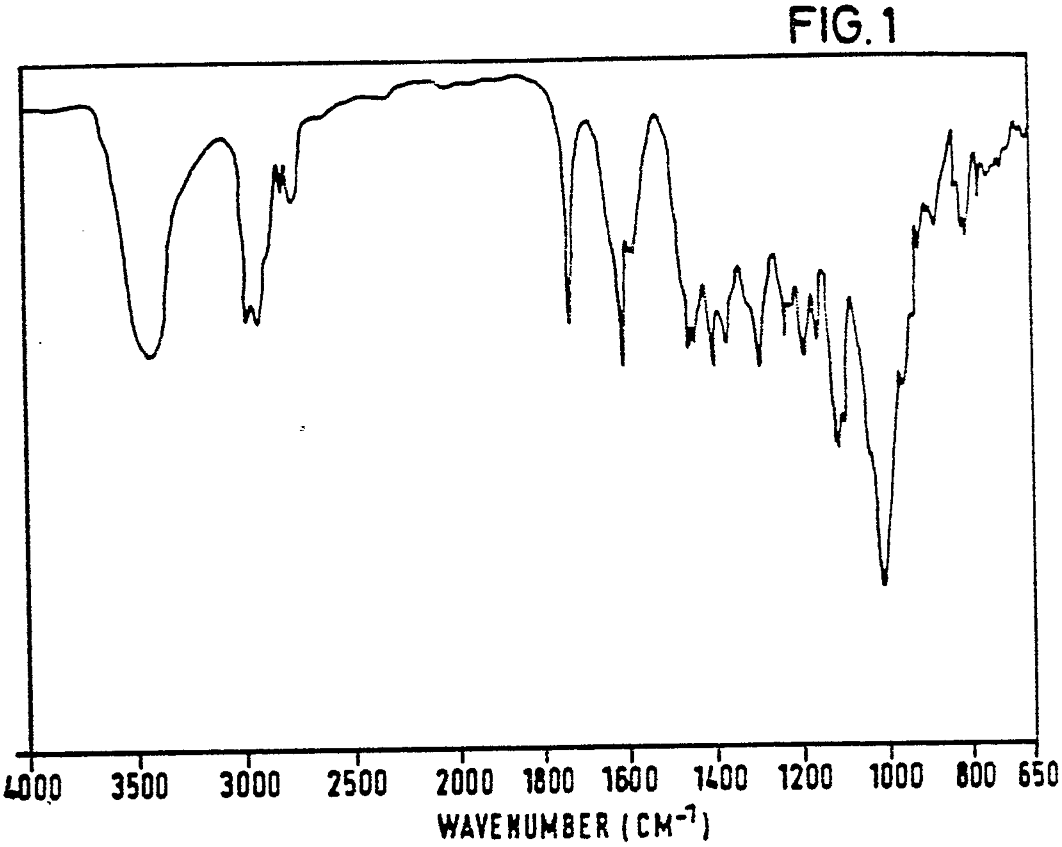
4000
3500
3000
2500
2000
1800
1600
1400
1200
1000
800
650
WAVENUMBER (CM⁻¹)

FIG. 2

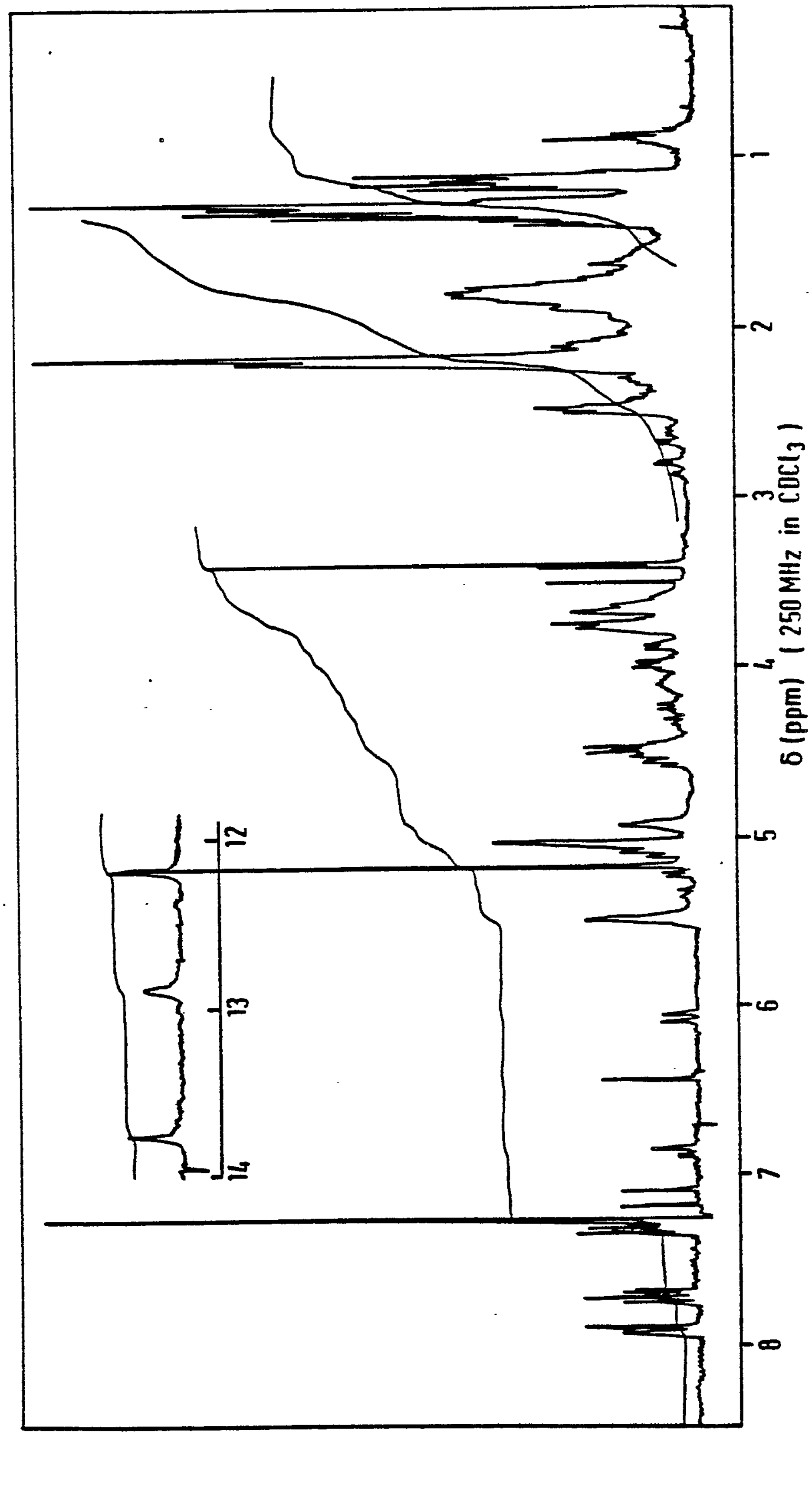

FIG. 3

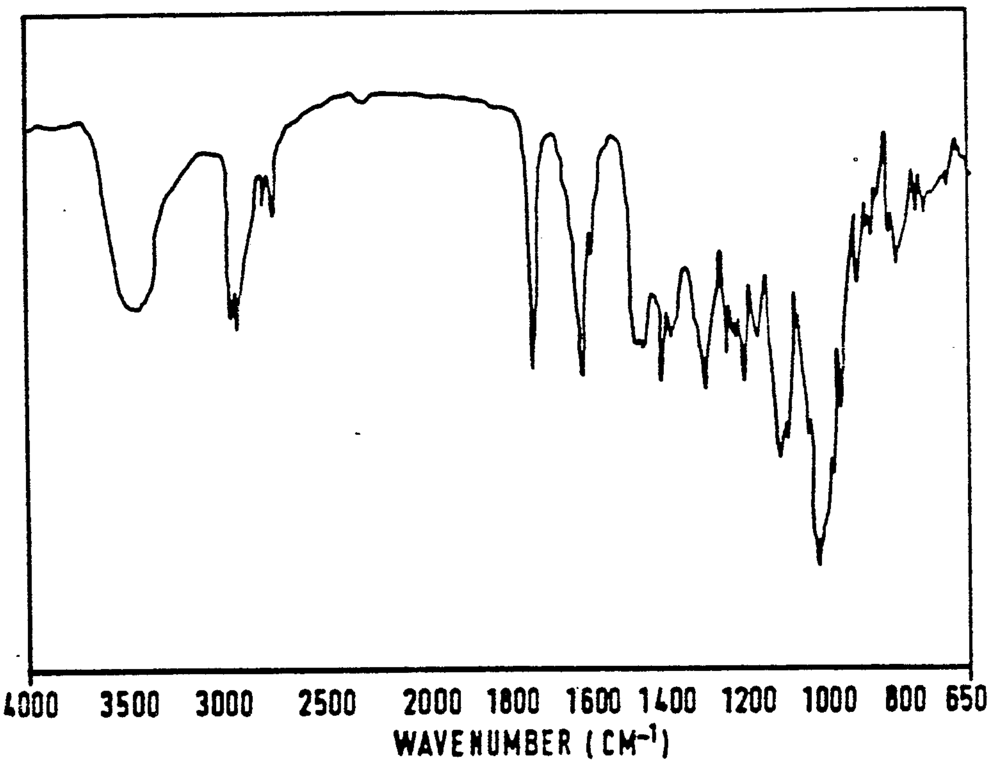
4000
3500
3000
2500
2000
1800
1600
1400
1200
1000
800
650
WAVENUMBER (CM$^{-1}$)

FIG. 4

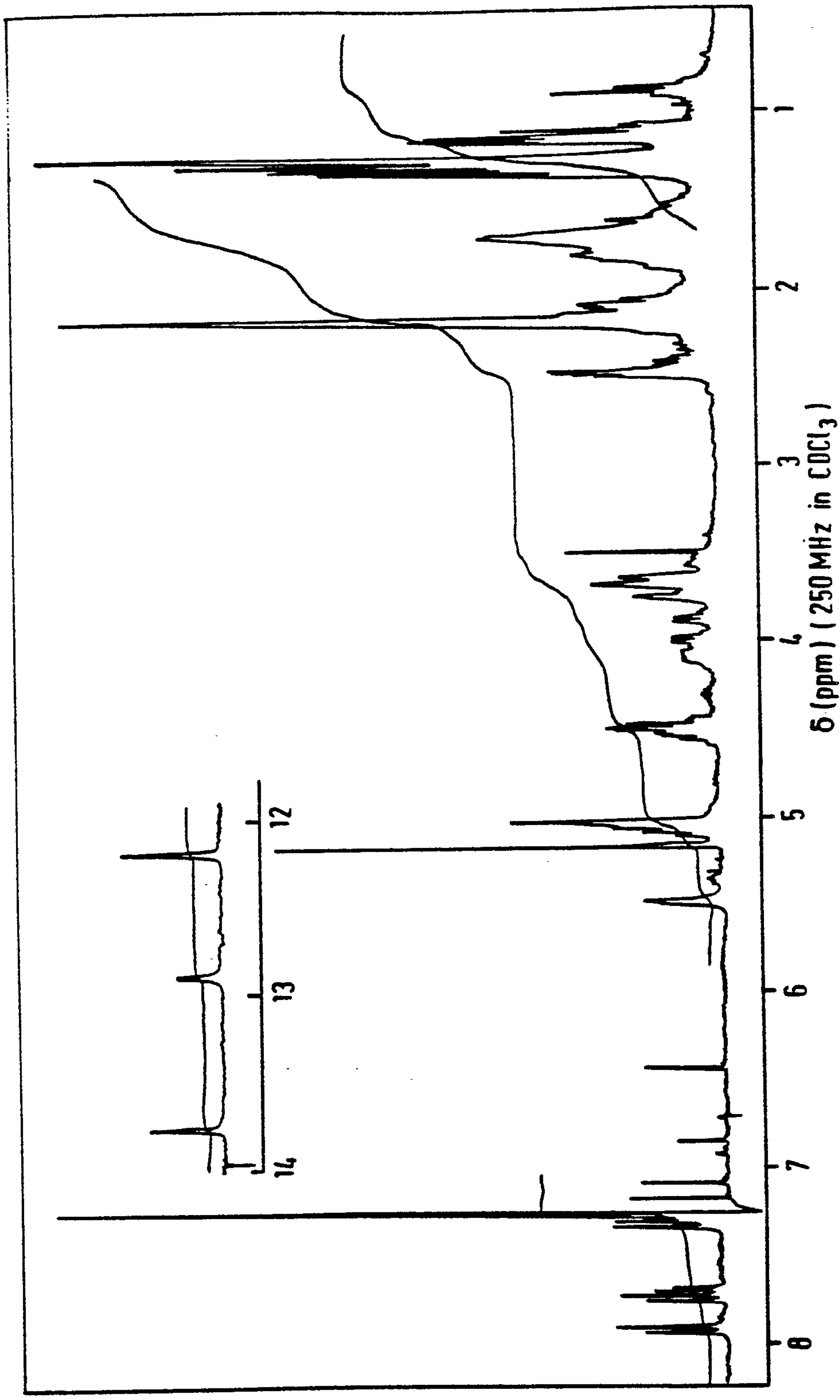


δ (ppm) (250 MHz in $CDCl_3$)

FIG. 5

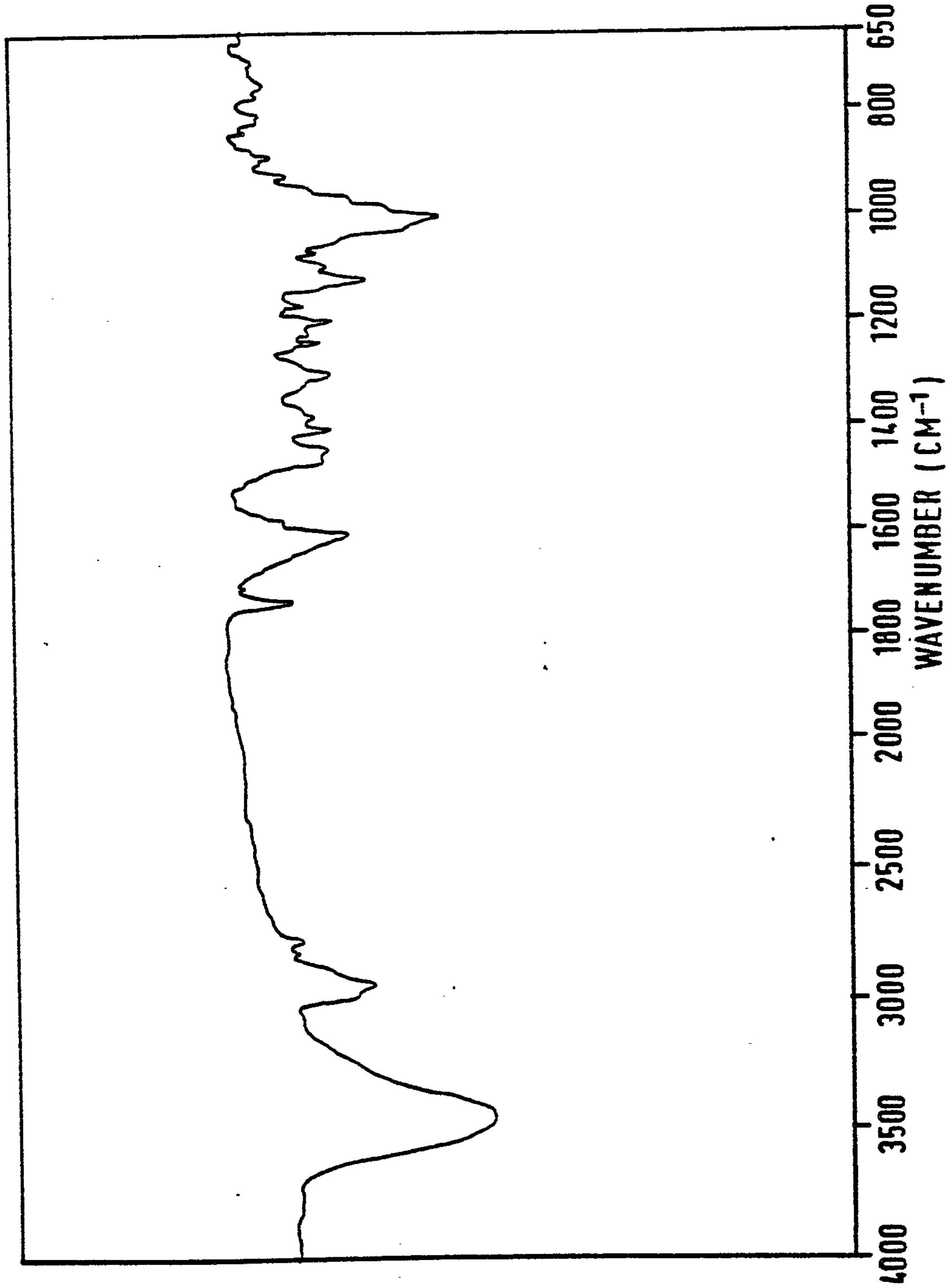
4000
3500
3000
2500
2000
1800
1600
1400
1200
1000
800
650
WAVENUMBER ( $CM^{-1}$ )

FIG. 6

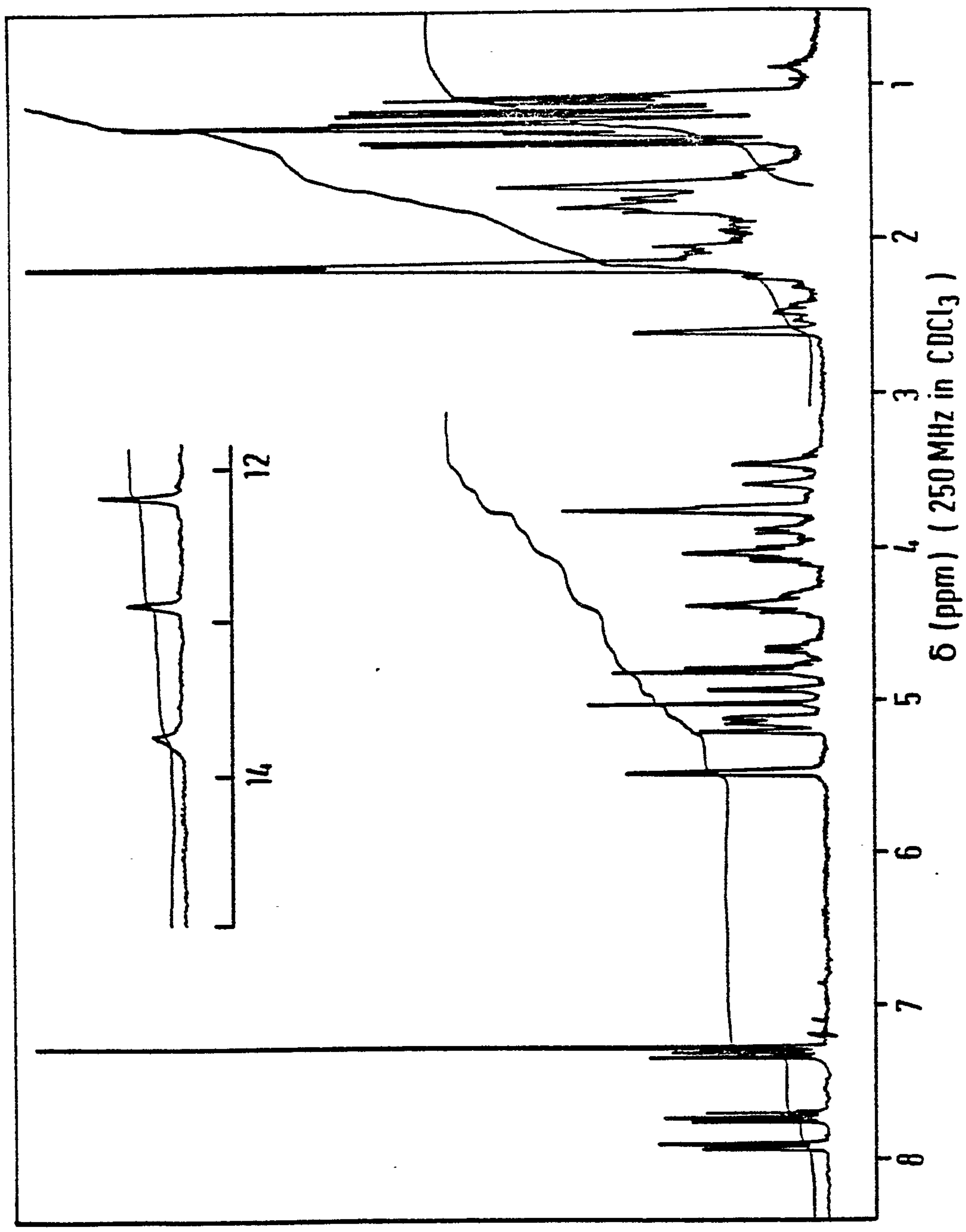

FIG. 7

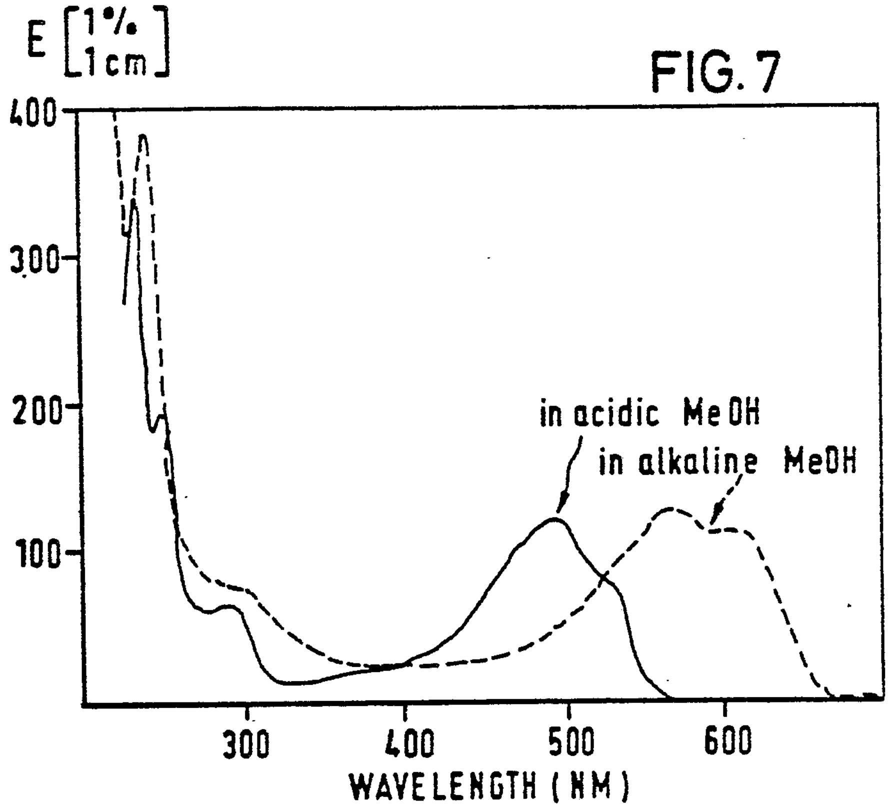
E [1% 1 cm]
400
300
200
100
in acidic MeOH
in alkaline MeOH
300
400
500
600
WAVELENGTH (NM)

FIG. 8

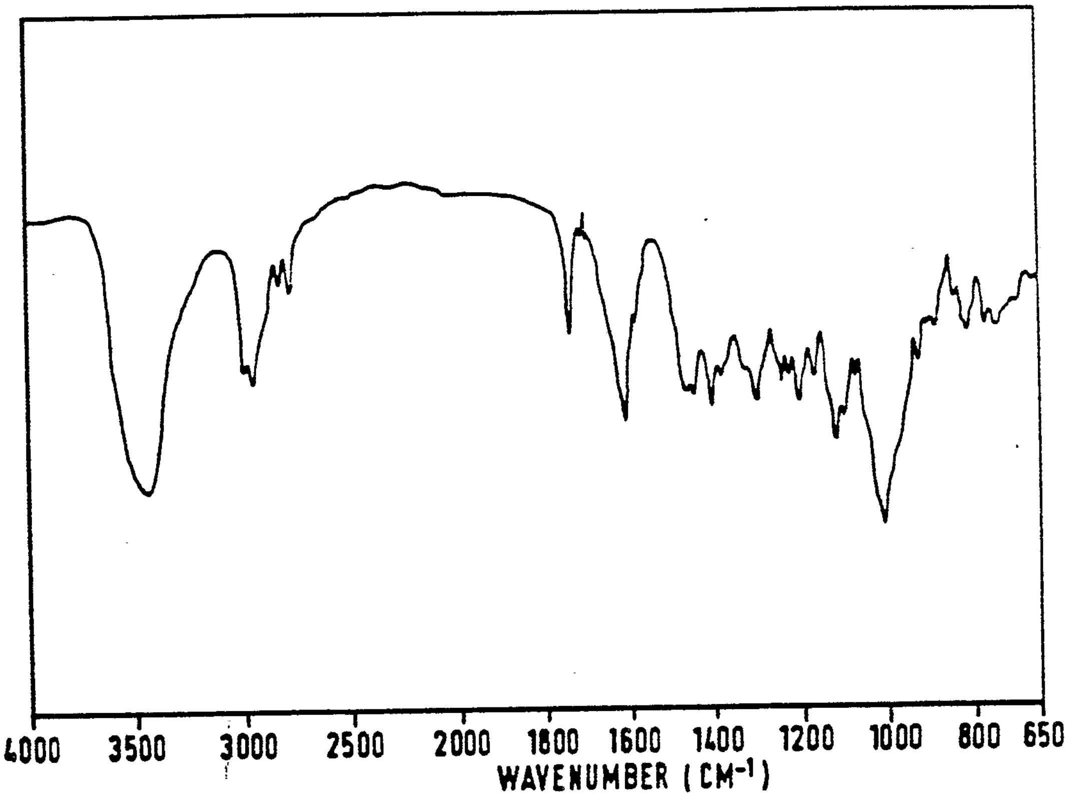
4000
3500
3000
2500
2000
1800
1600
1400
1200
1000
800
650
WAVENUMBER ( CM-1 )

FIG.9

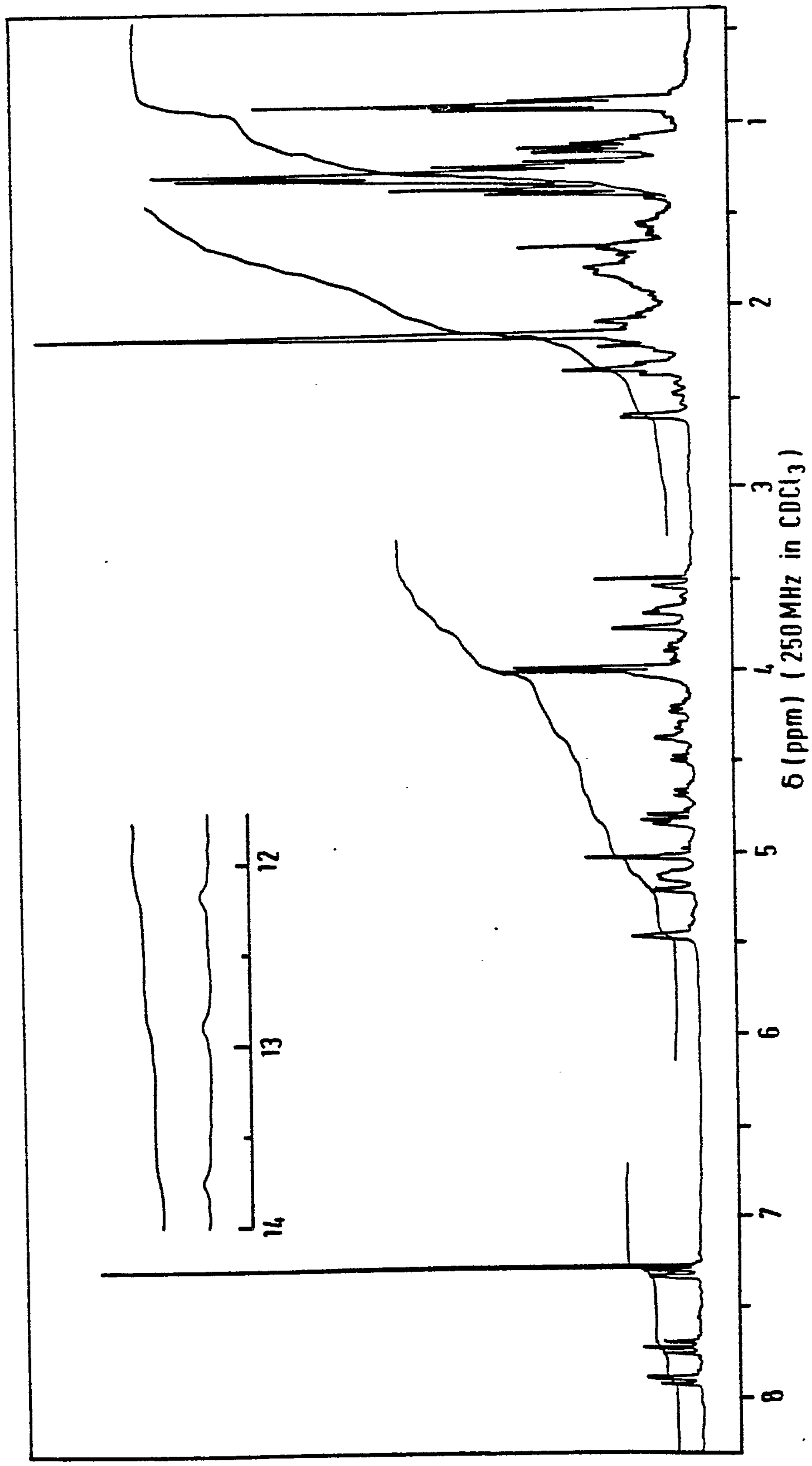

FIG. 10

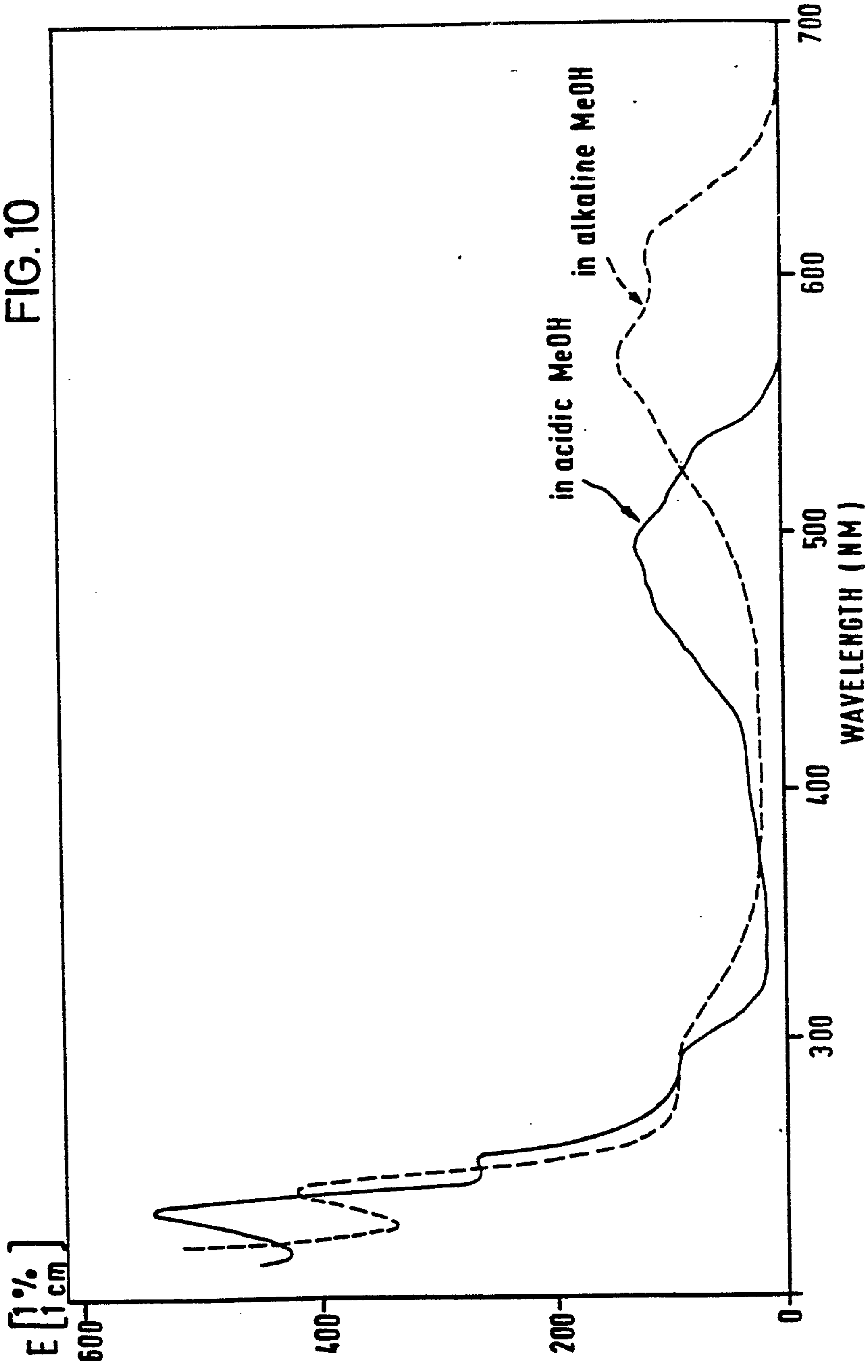
in acidic MeOH
in alkaline MeOH
WAVELENGTH (NM)
300
400
500
600
700
E [1% / 1 cm]
600
400
200
0

FIG. 11

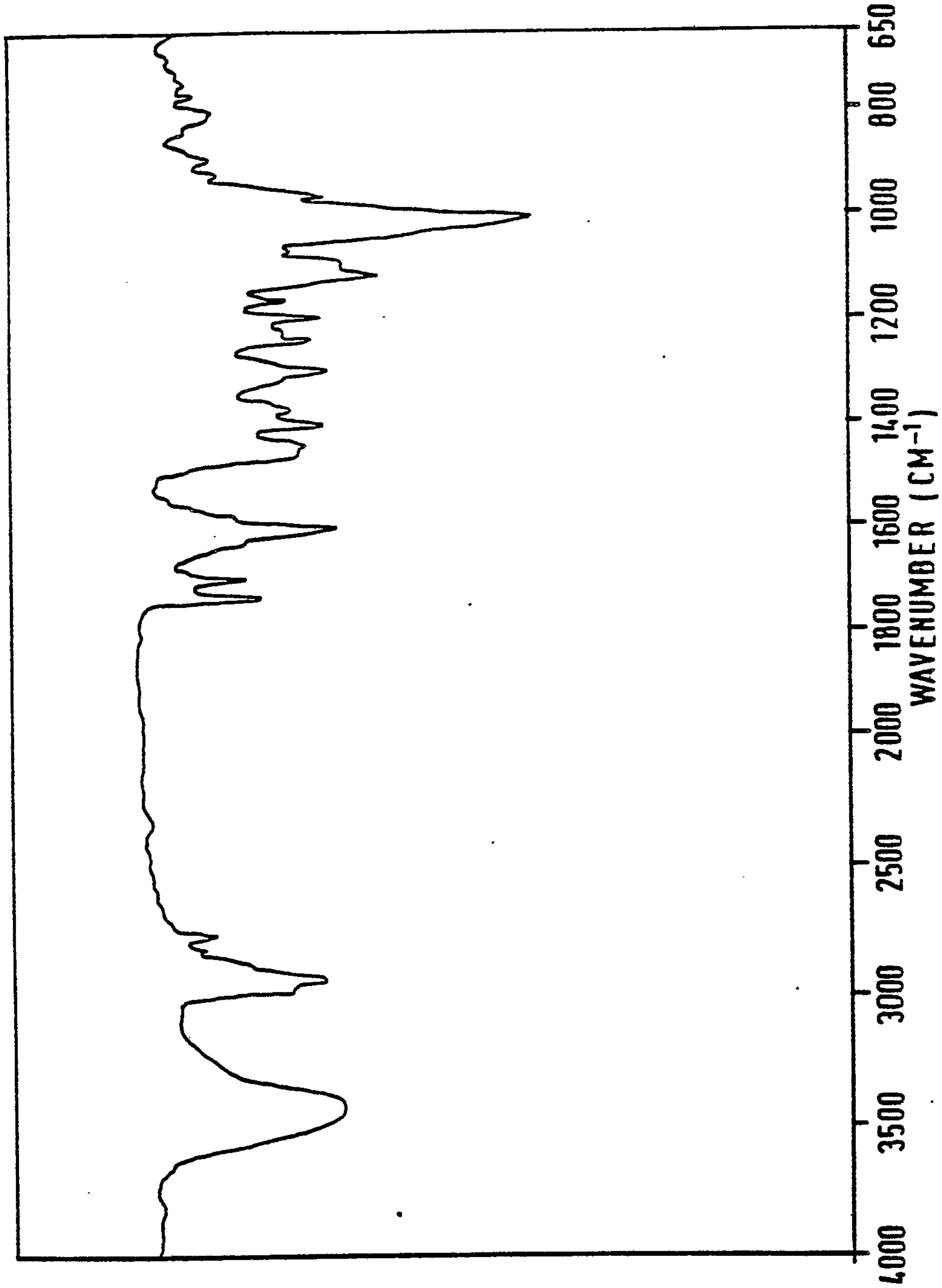
650
800
1000
1200
1400
1600
1800
2000
2500
3000
3500
4000
WAVENUMBER (CM⁻¹)

FIG. 12

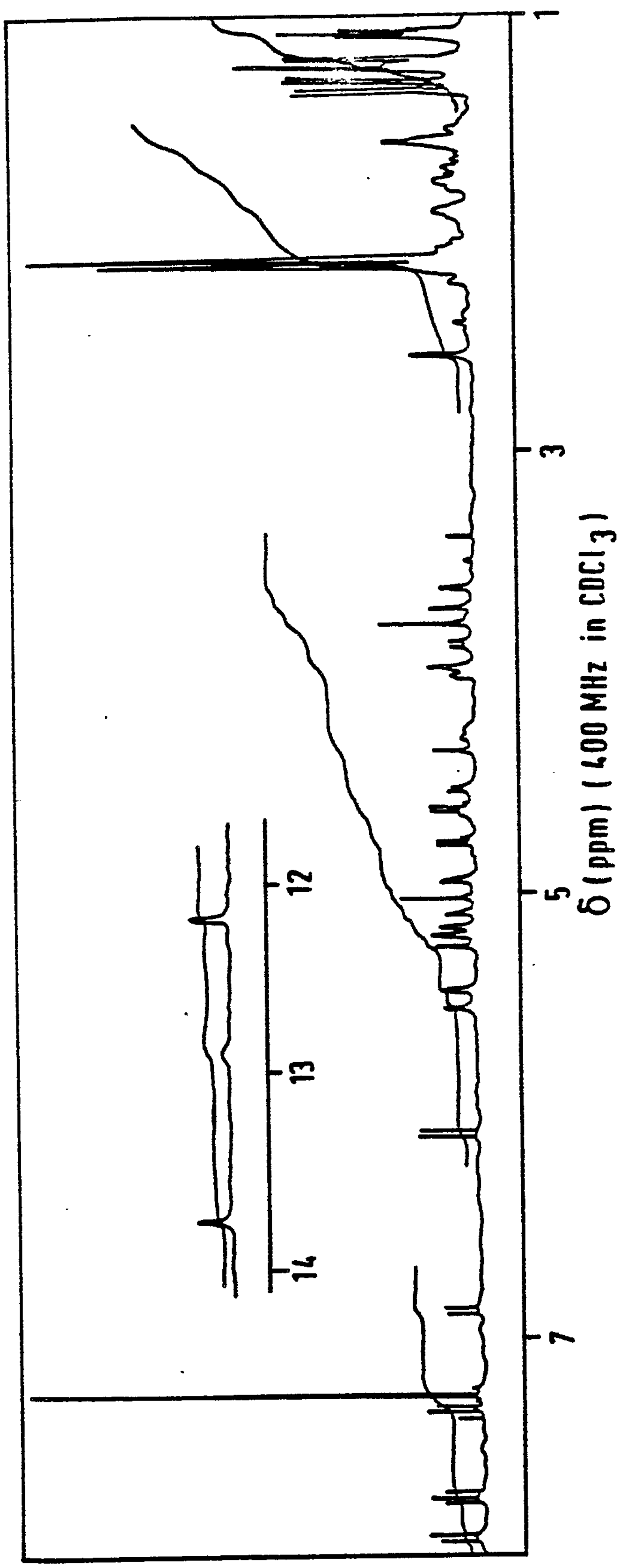

FIG. 13

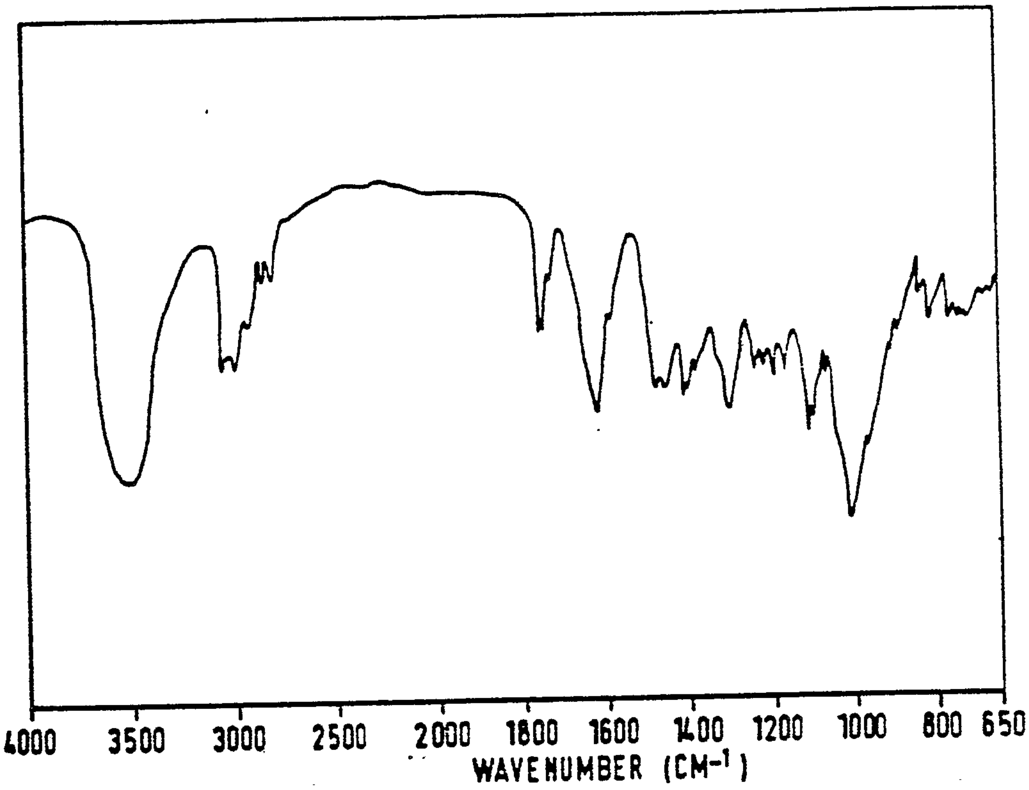
4000
3500
3000
2500
2000
1800
1600
1400
1200
1000
800
650
WAVENUMBER (CM$^{-1}$)

FIG. 14

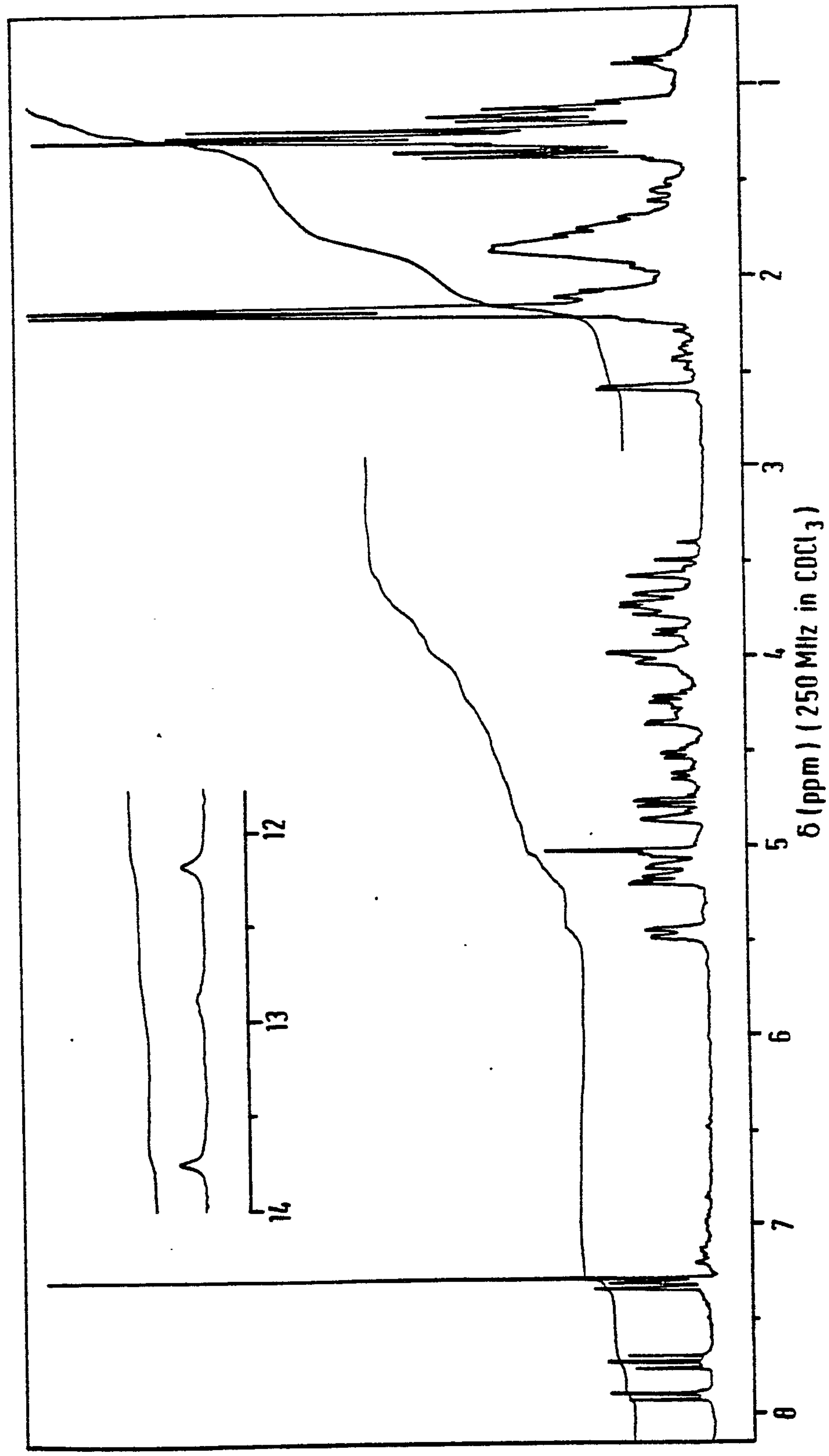

FIG.15

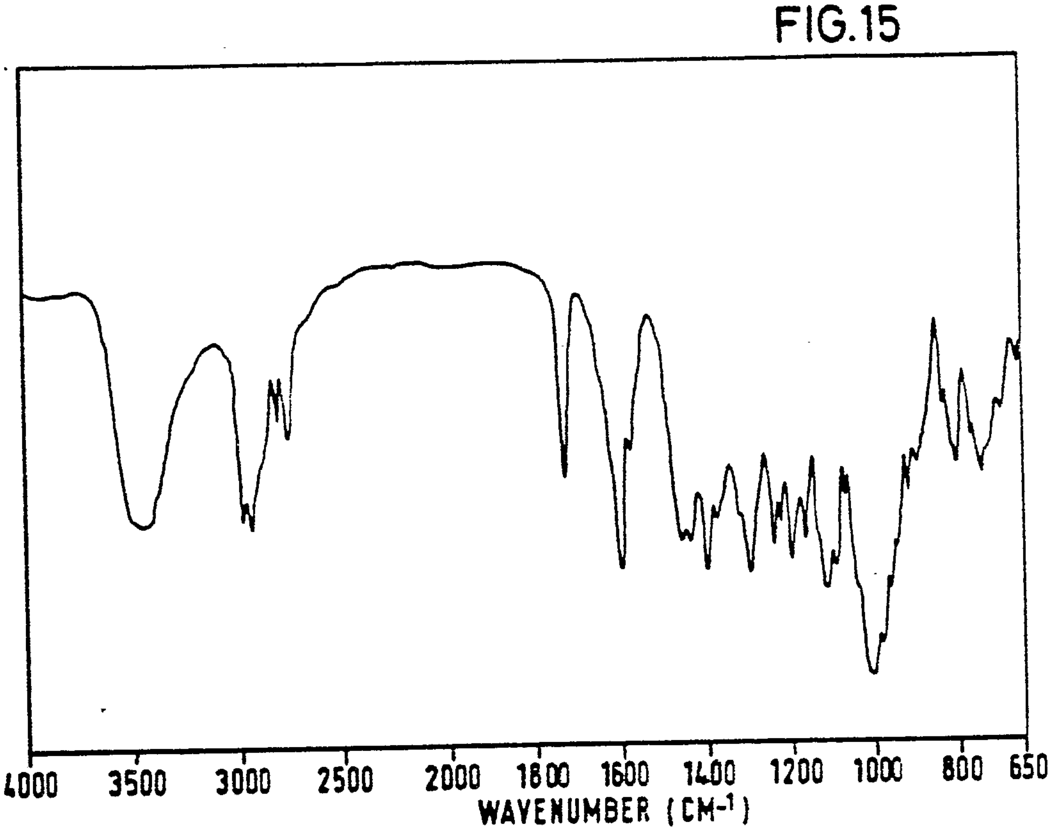
4000
3500
3000
2500
2000
1800
1600
1400
1200
1000
800
650
WAVENUMBER (CM-1)

FIG.16

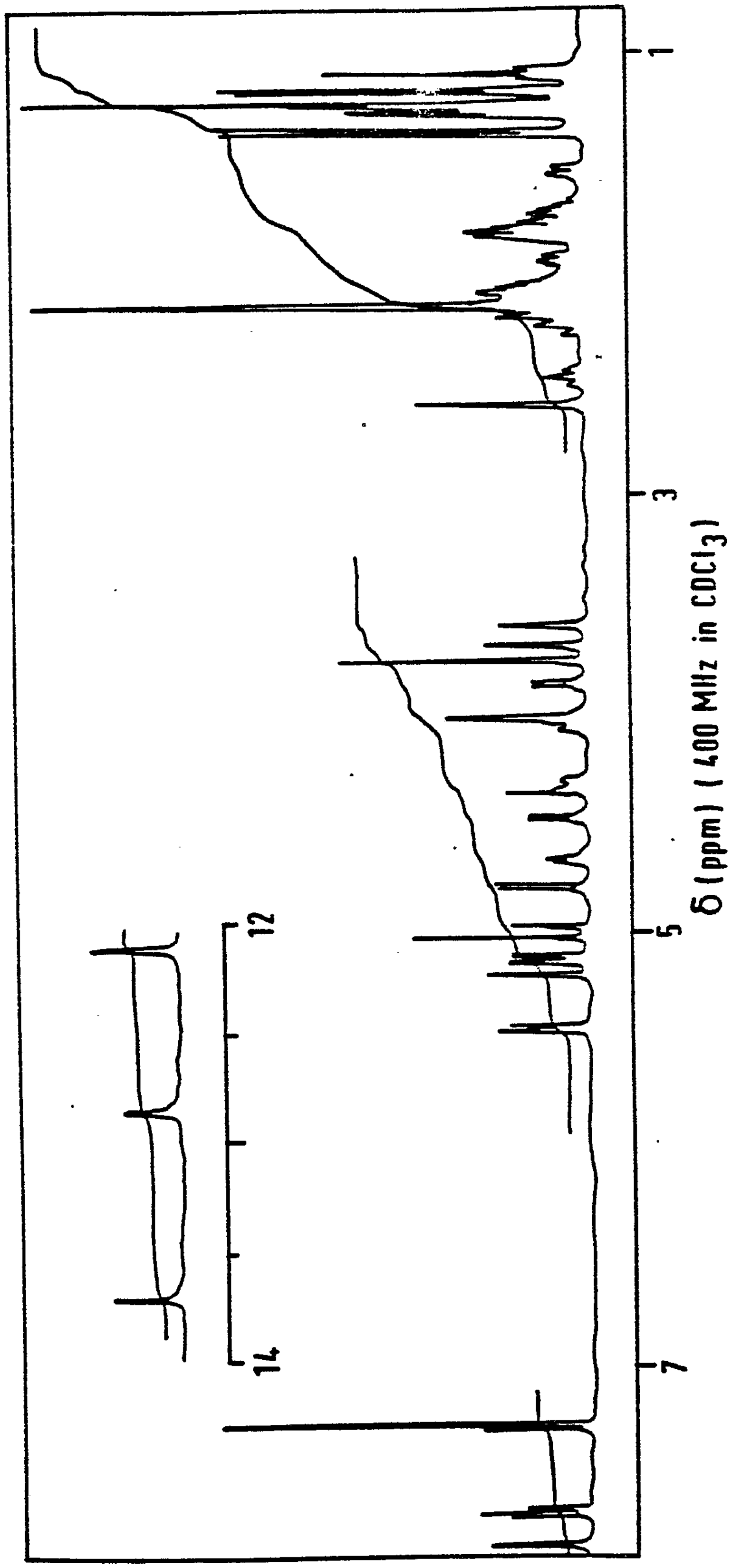

FIG.17

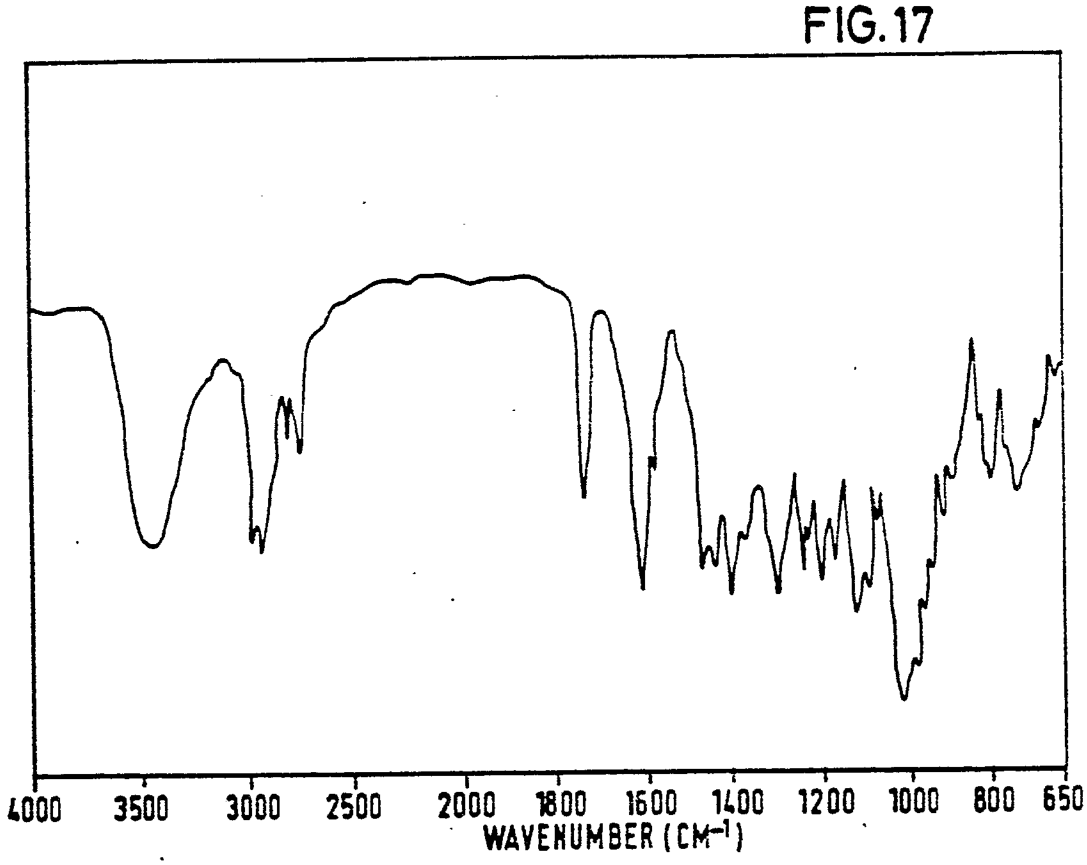
4000
3500
3000
2500
2000
1800
1600
1400
1200
1000
800
650
WAVENUMBER (CM-1)

FIG. 18

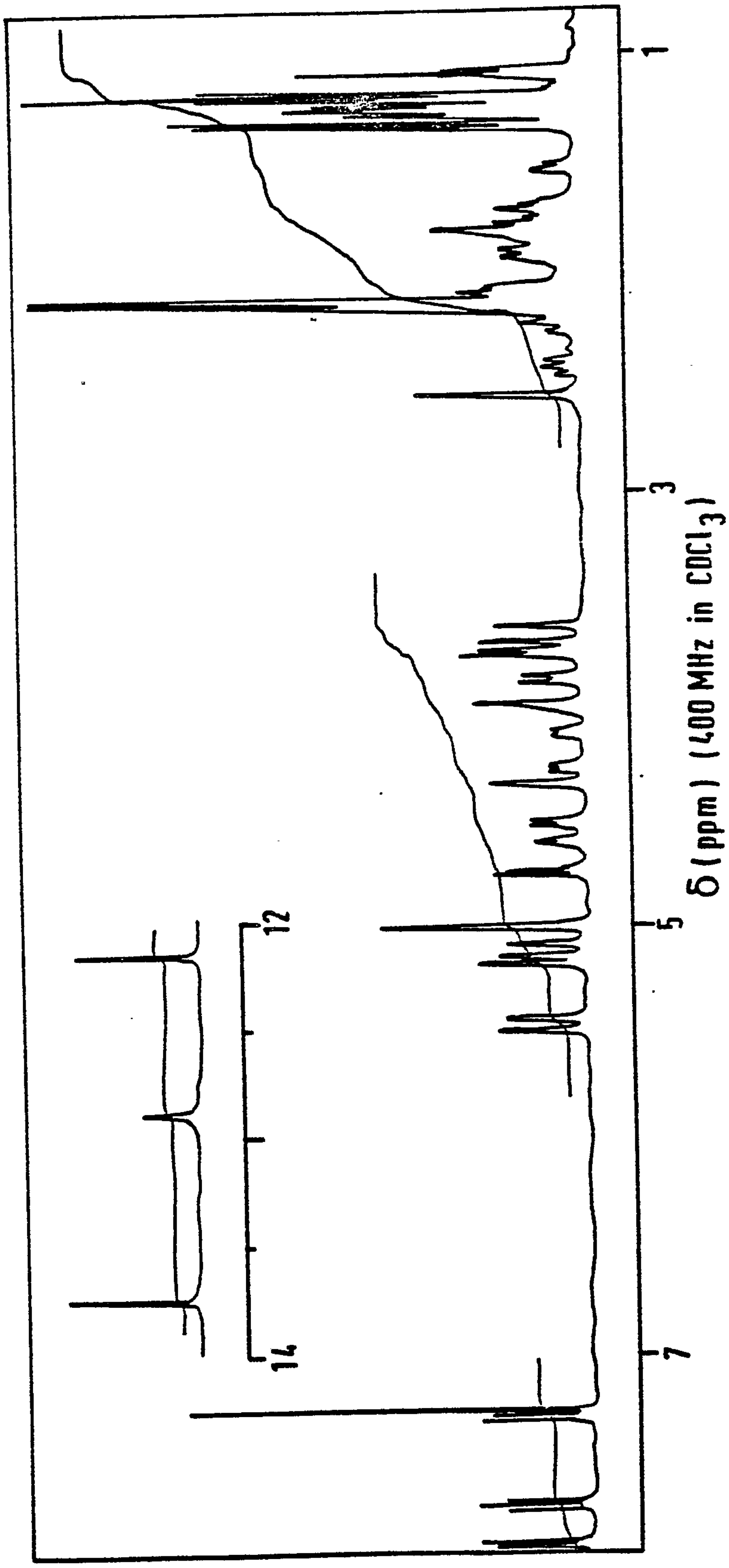

FIG. 19

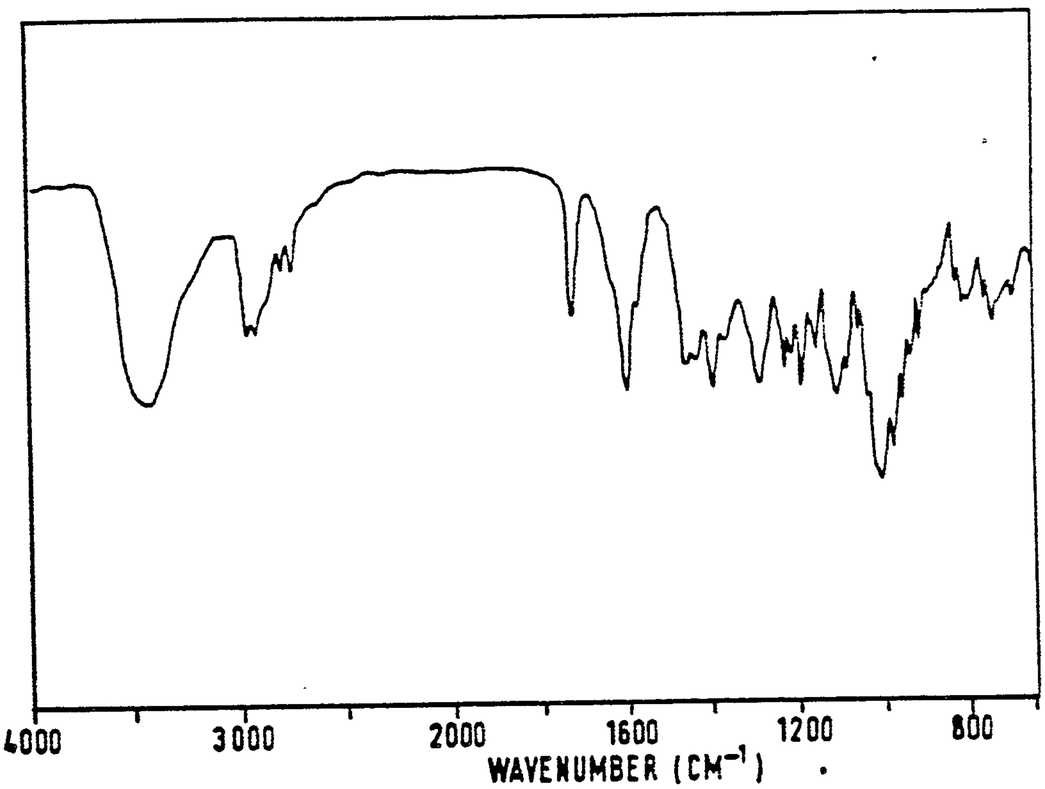

FIG. 20

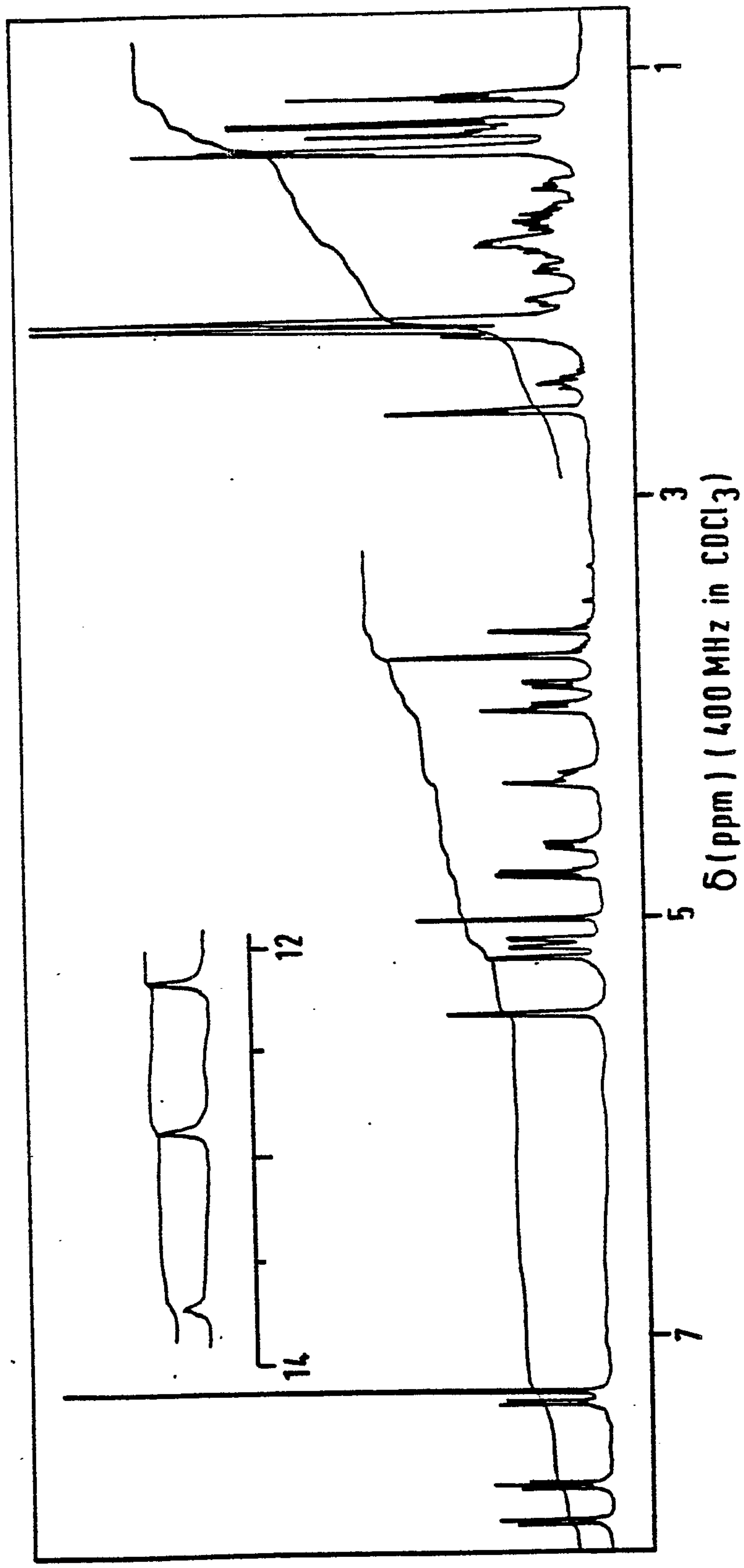

FIG. 21

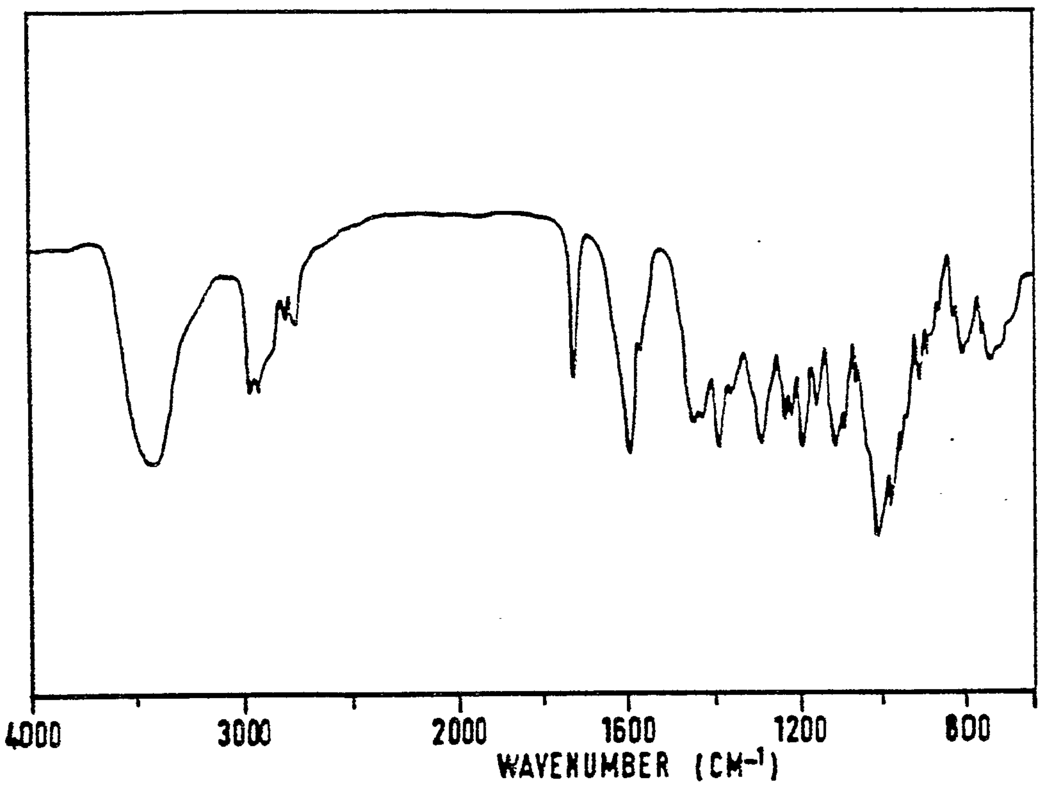

FIG. 22

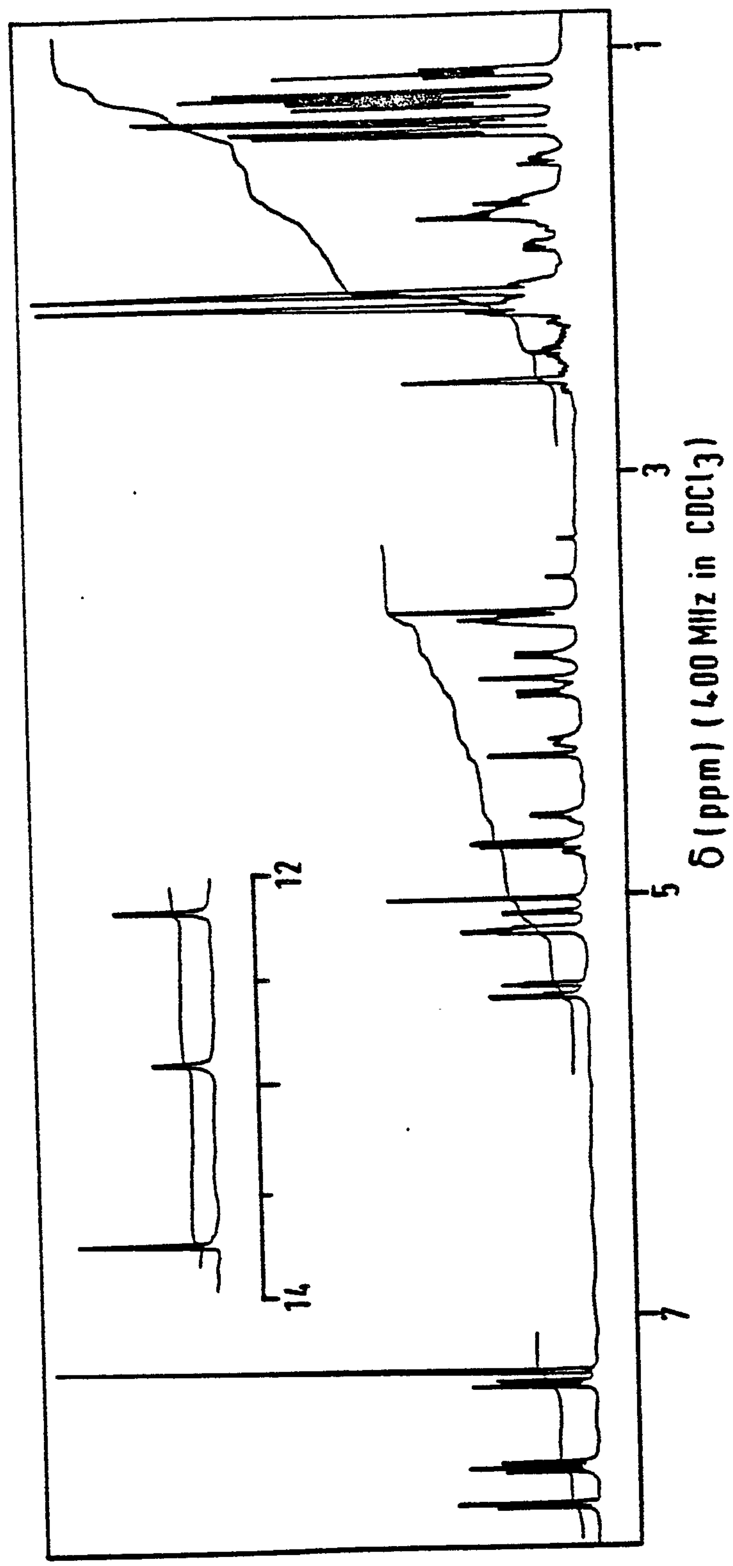

FIG. 23

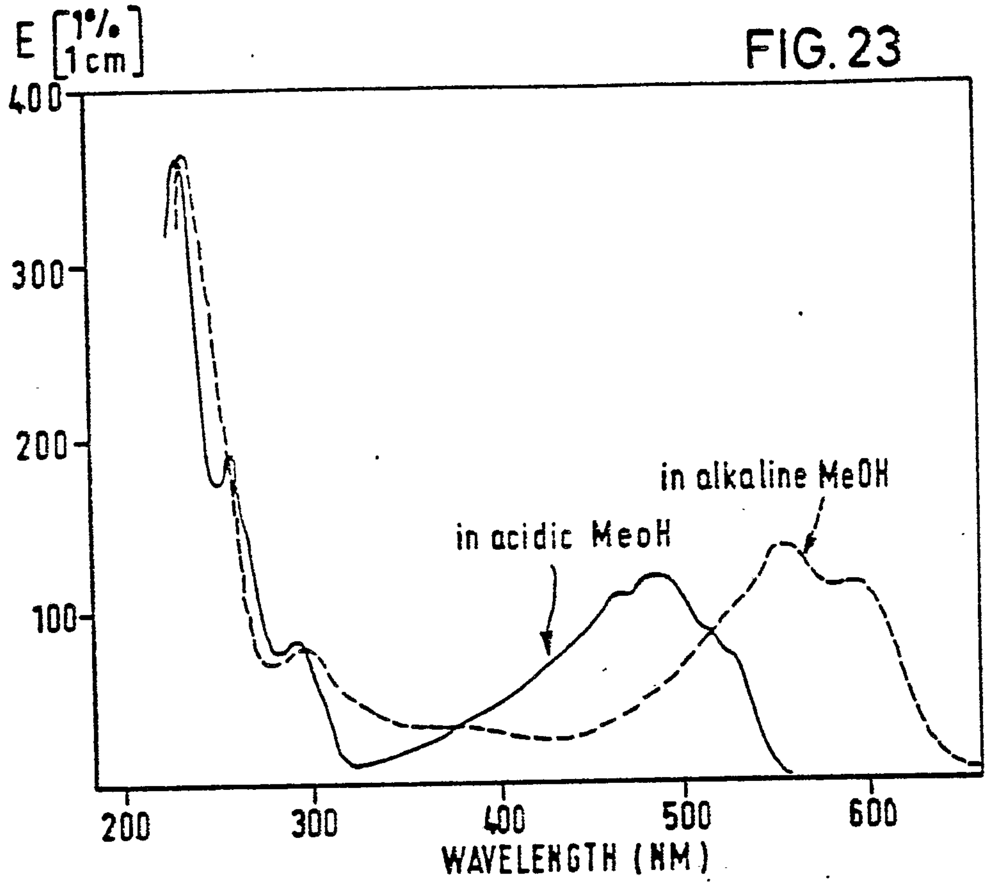
E [1% / 1 cm]
400
300
200
100
200
300
400
500
600
WAVELENGTH (NM)
in acidic MeoH
in alkaline MeOH

FIG. 24

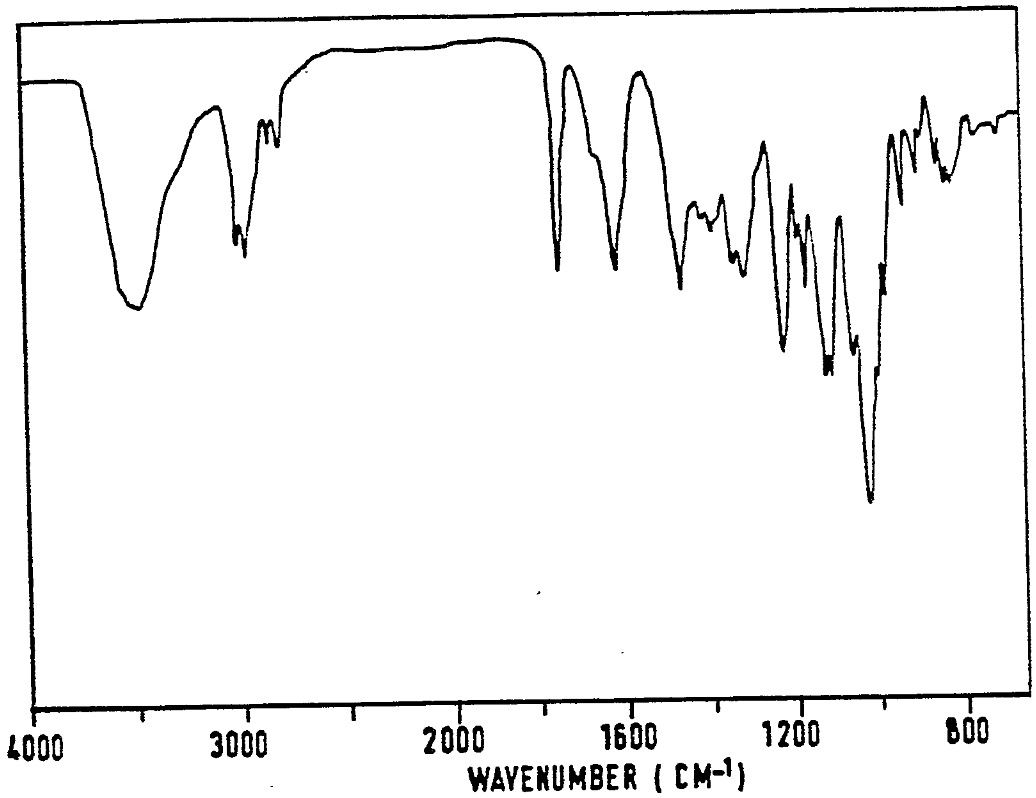

FIG. 25

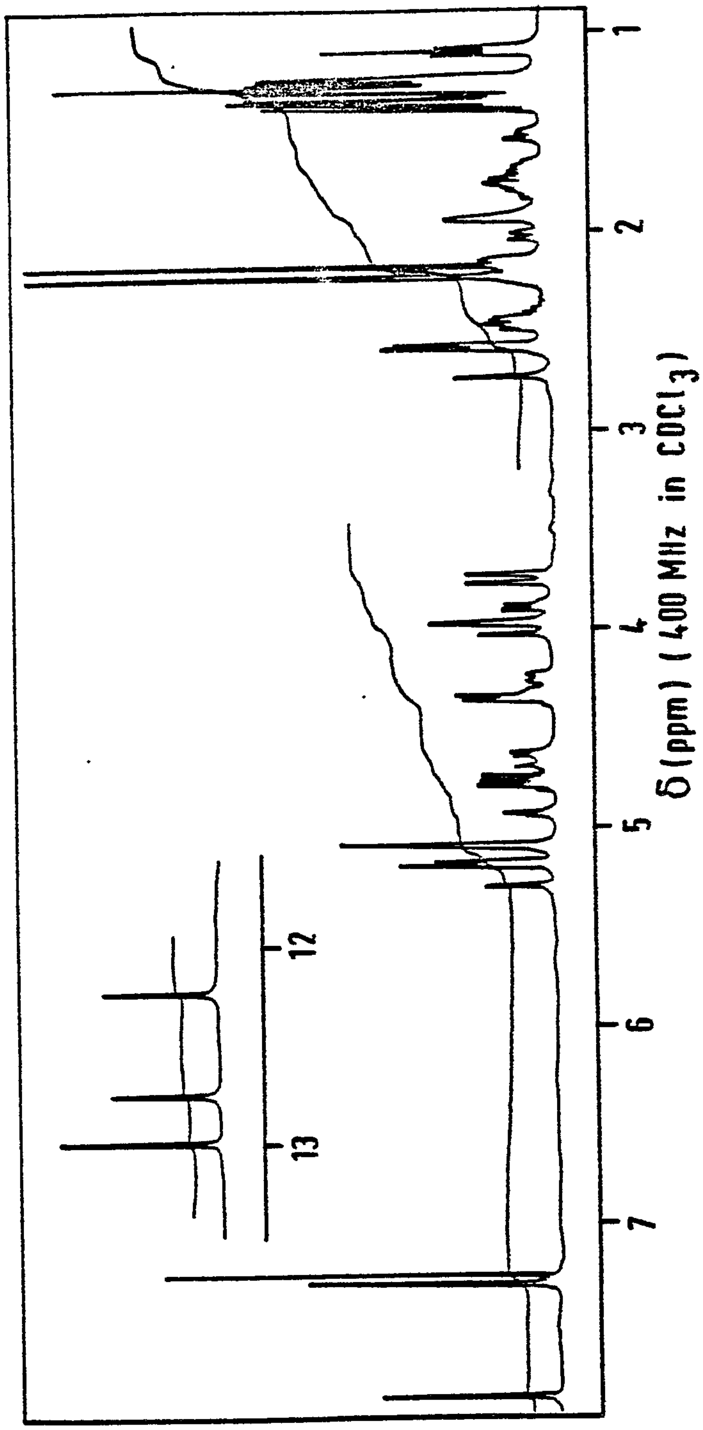

FIG. 26

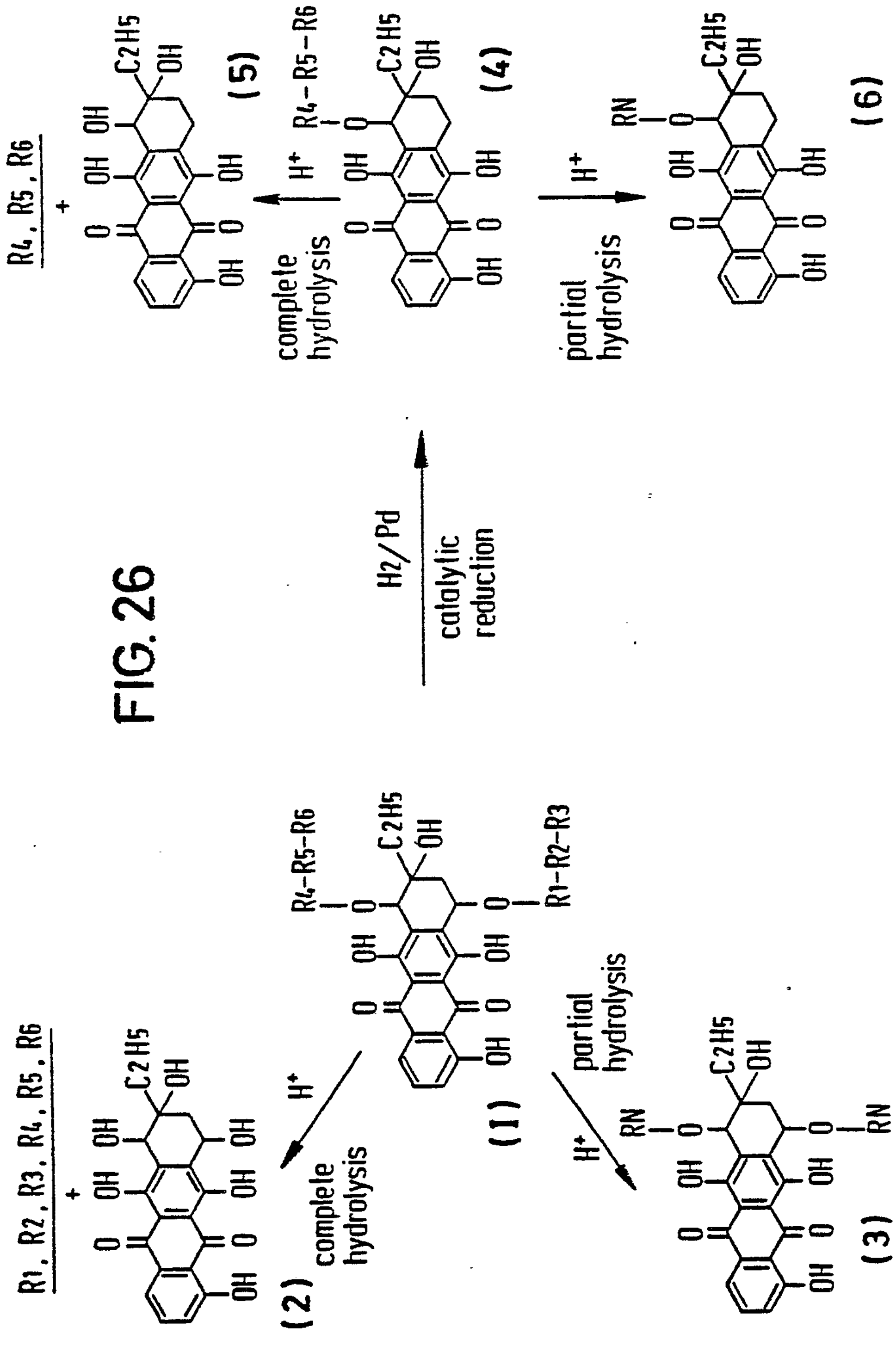


(1) (2) (3) (4) (5) (6)

FIG.27

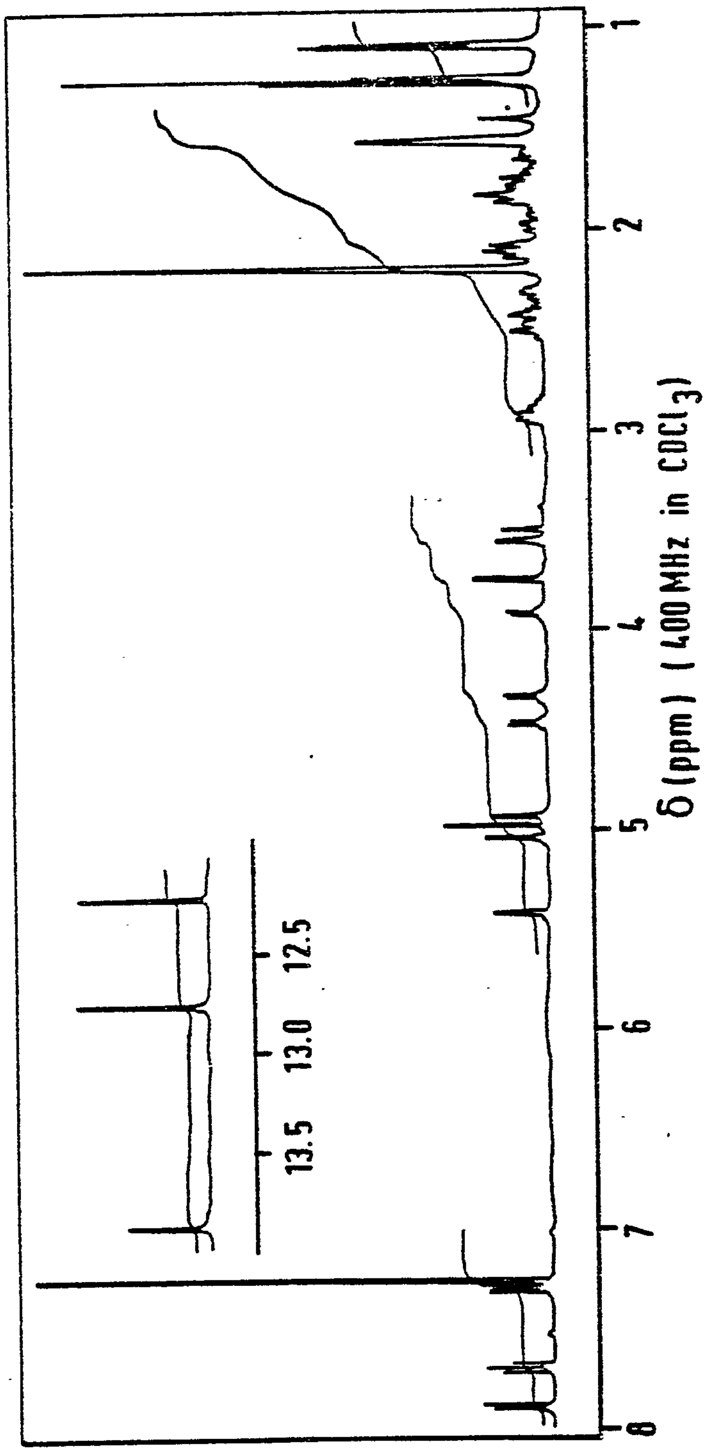

FIG. 28

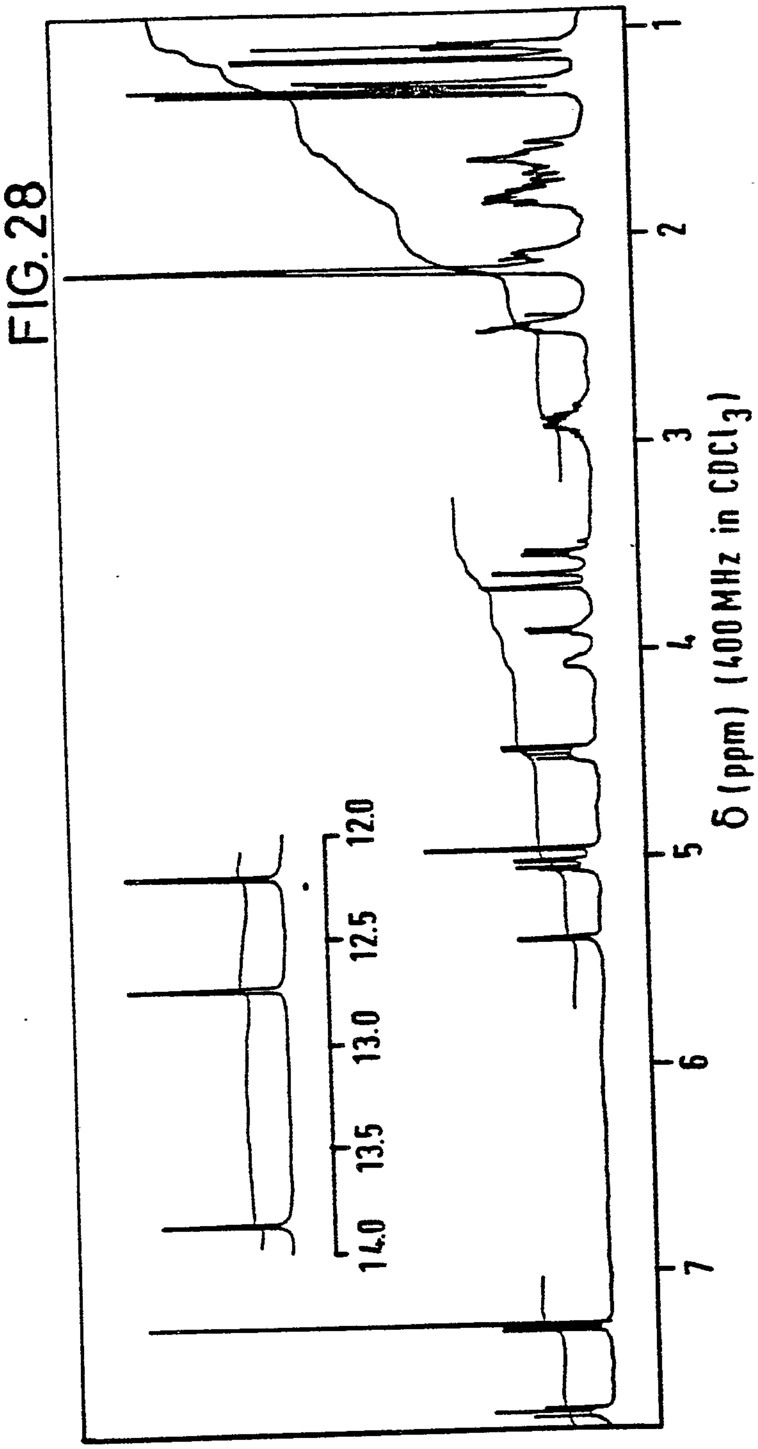

FIG. 29

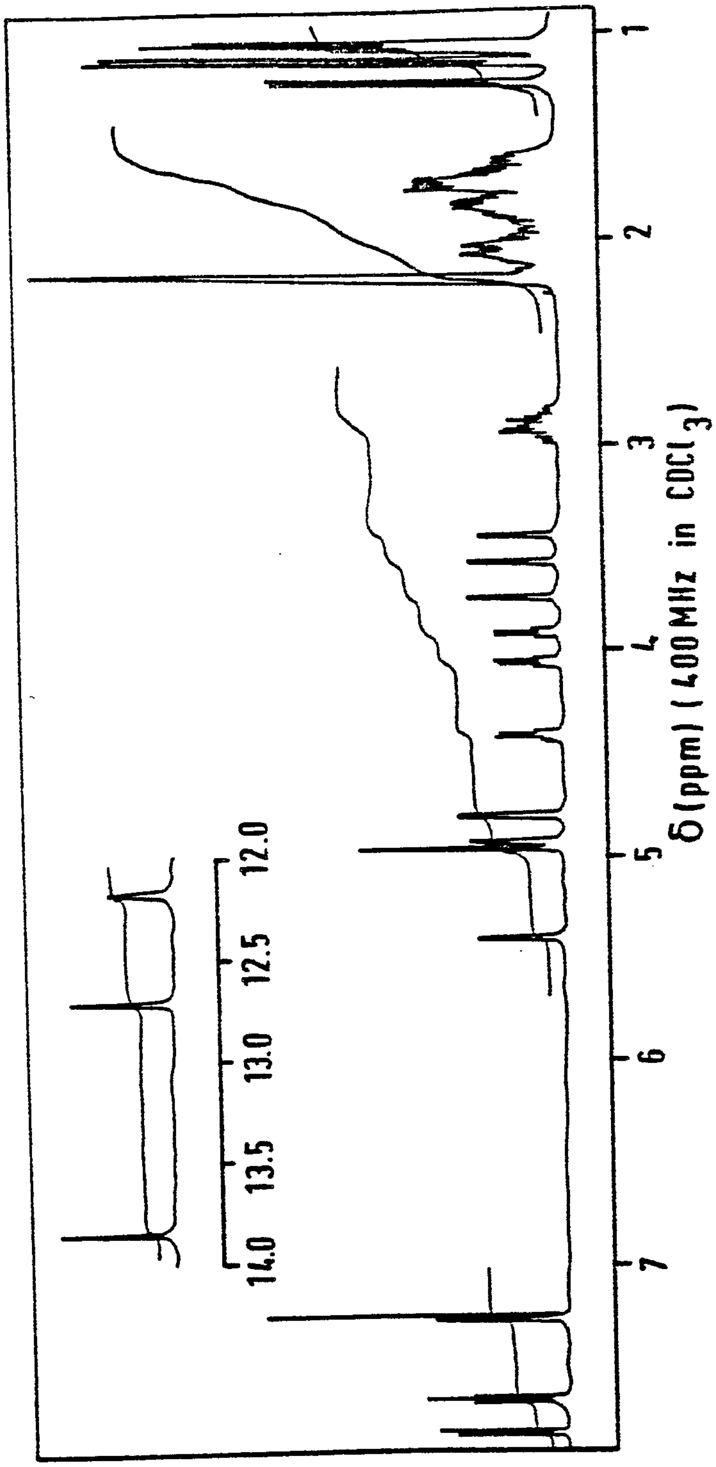

FIG. 30

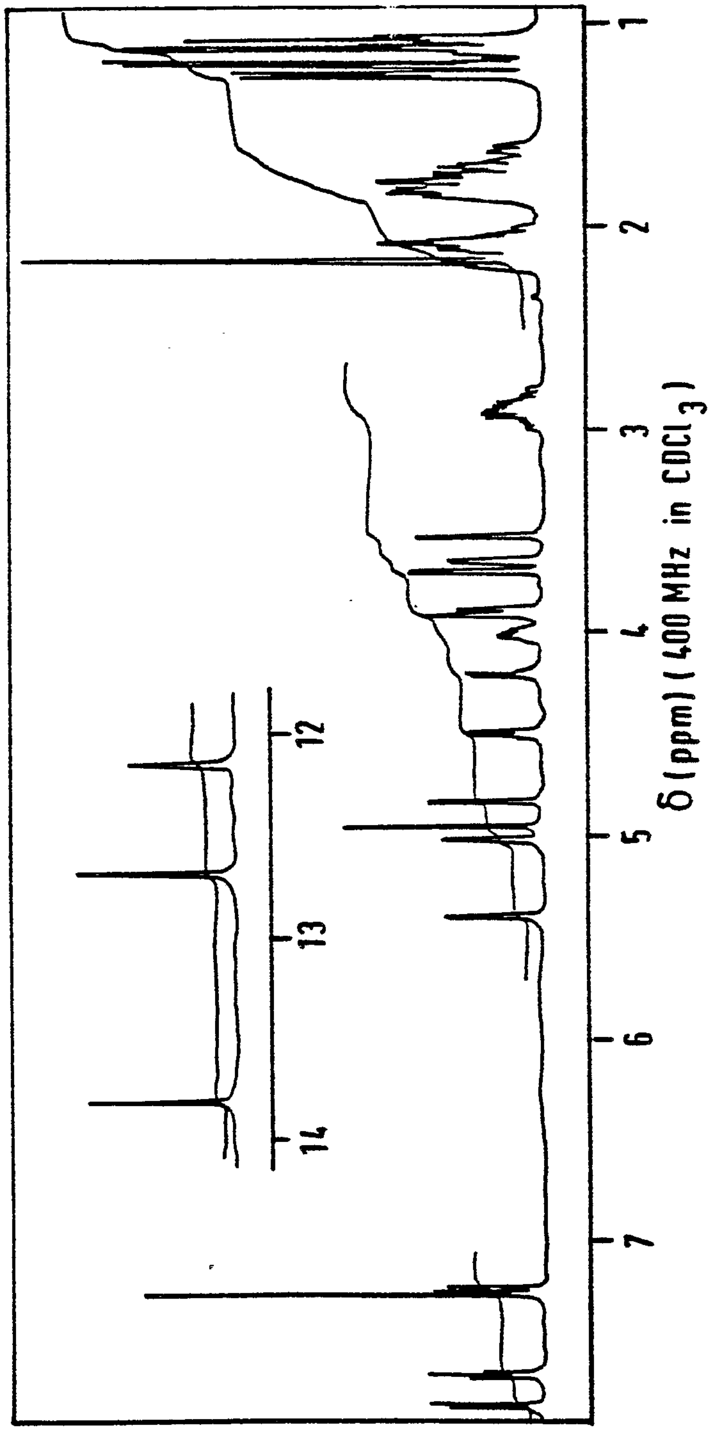

FIG. 31

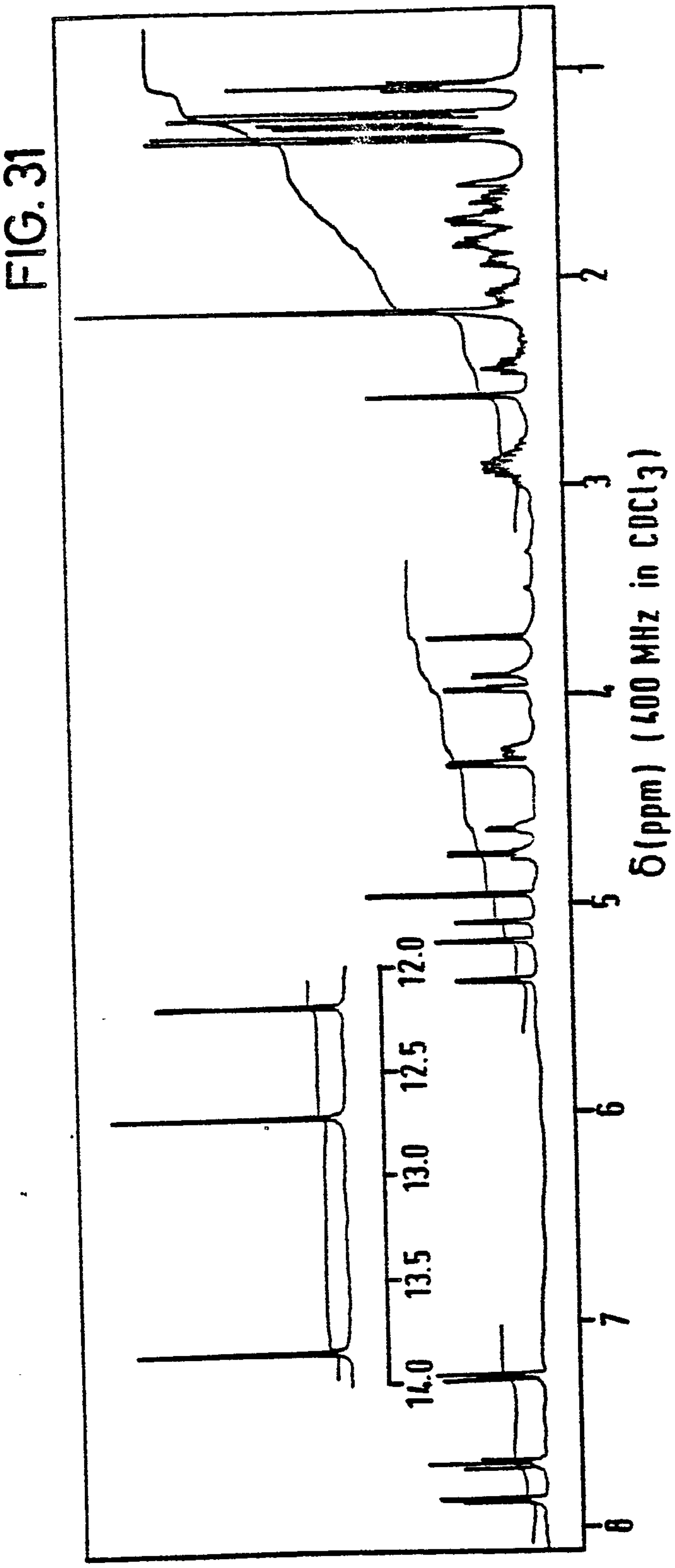

FIG.32

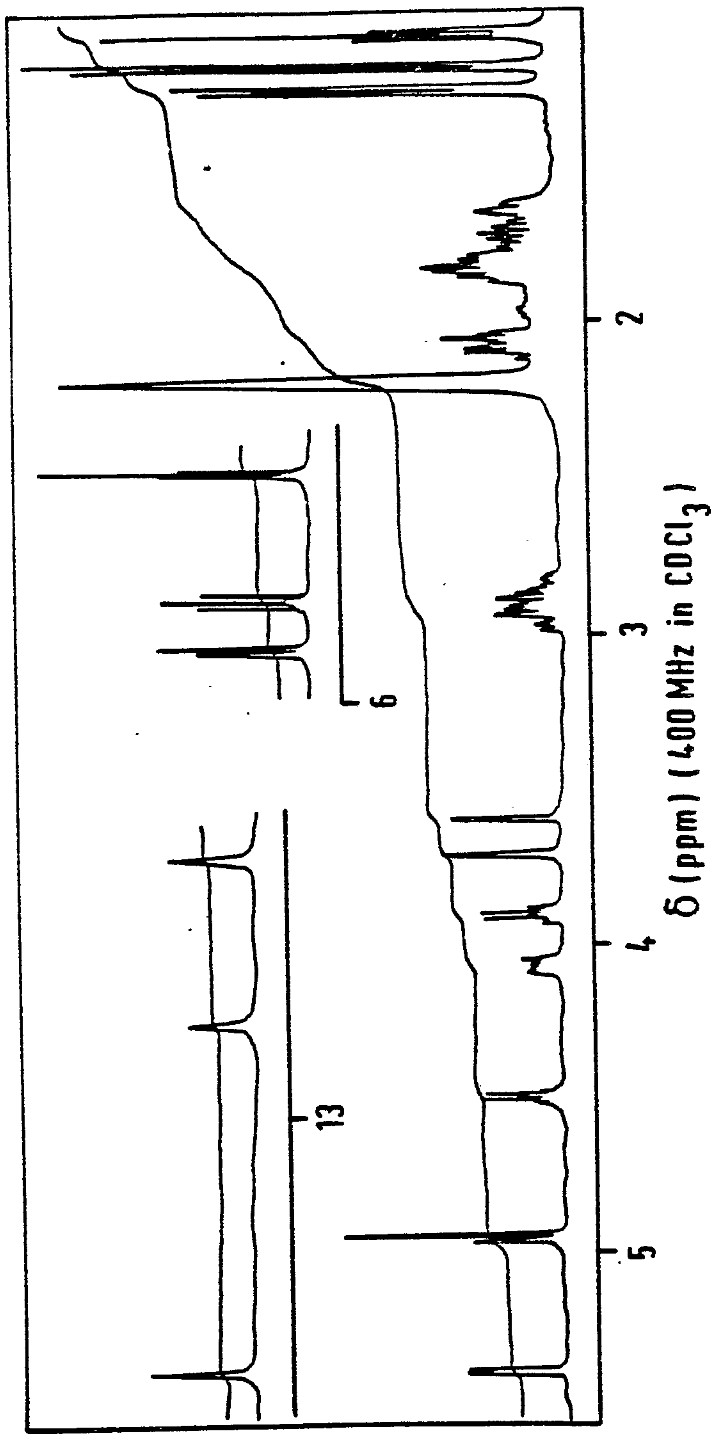

FIG. 33

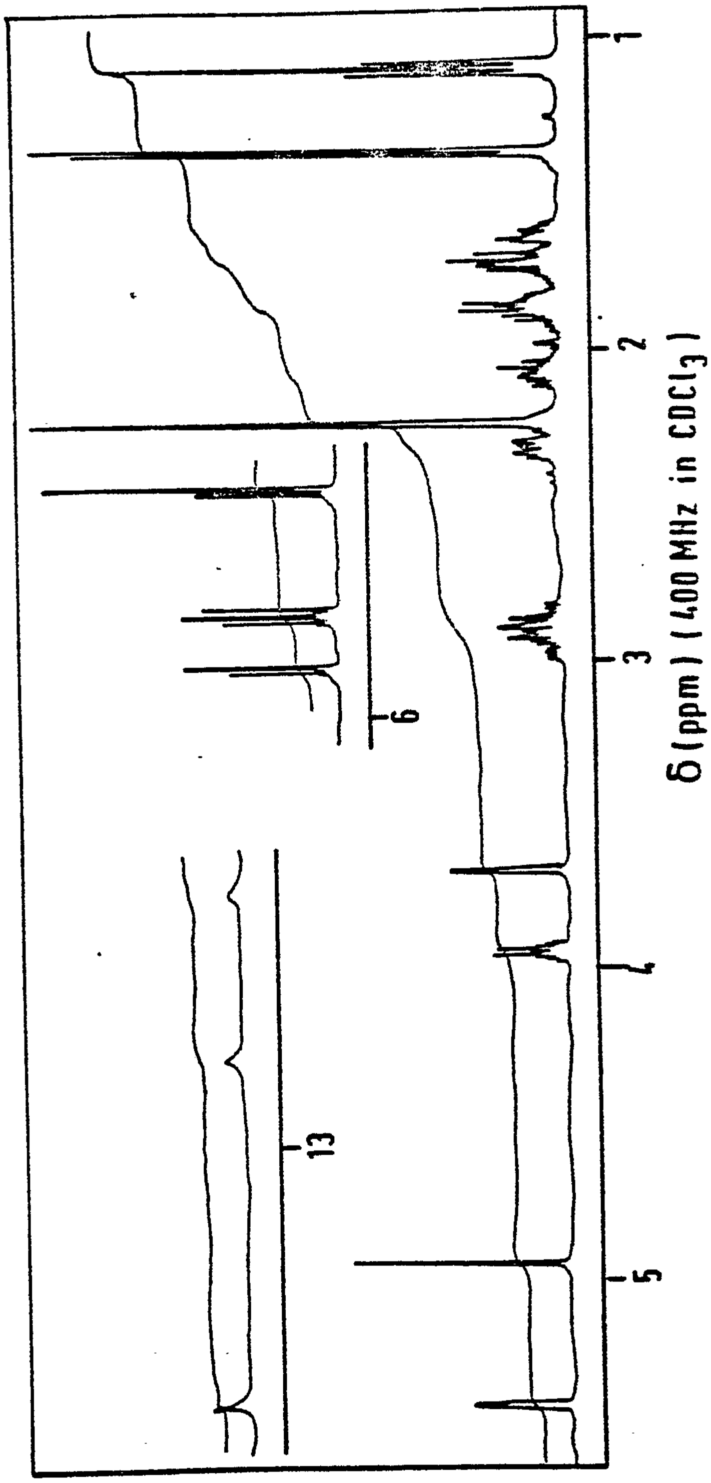

FIG.34

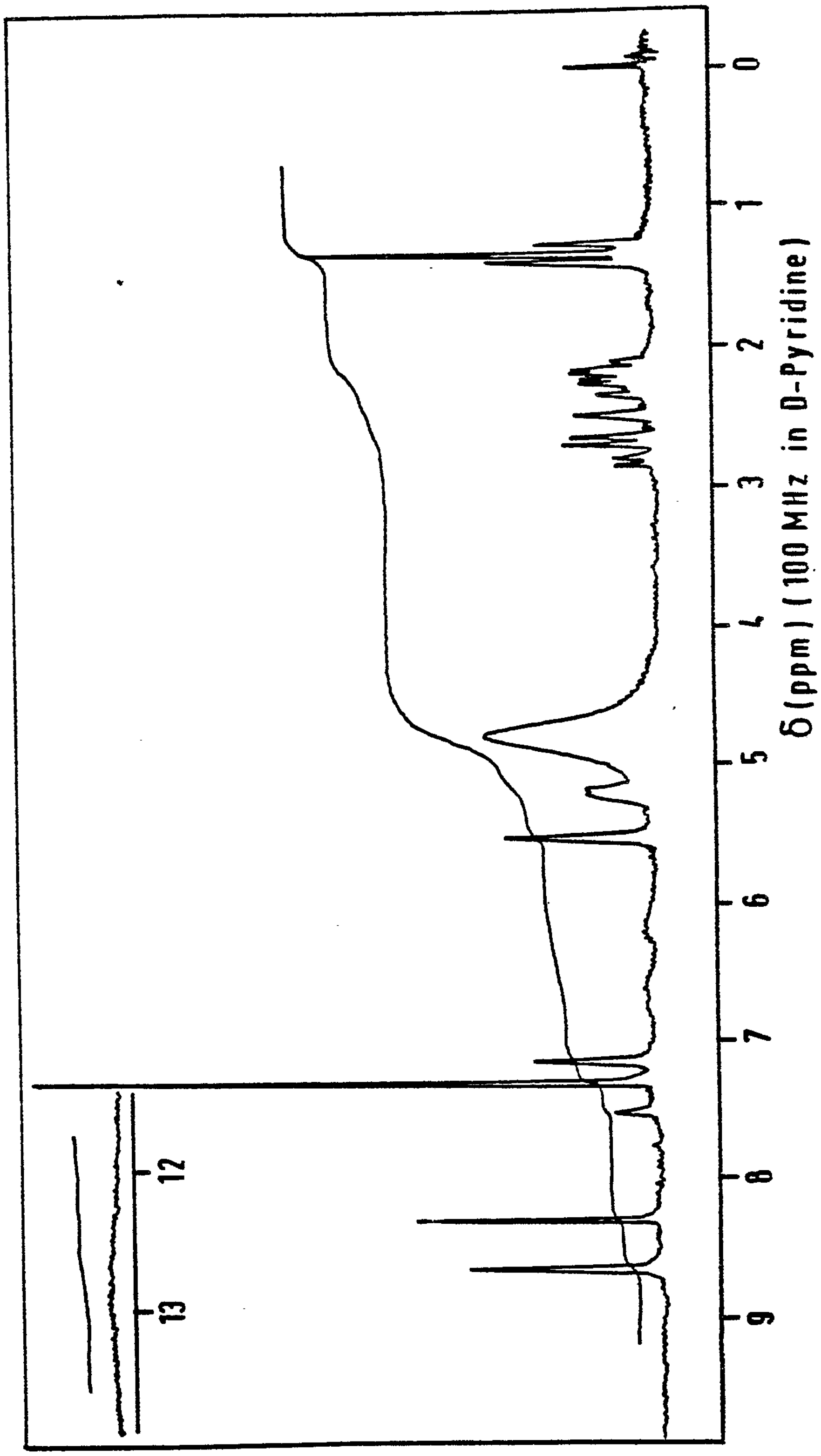

FIG.35

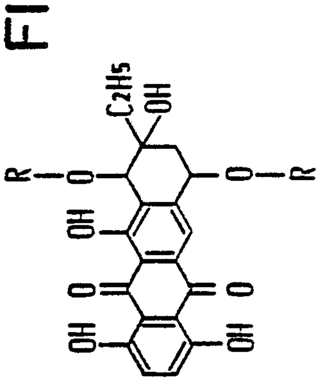

R:

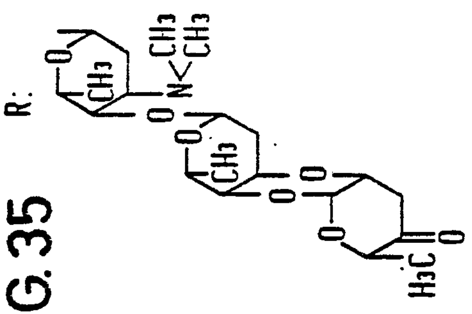

FIG.36

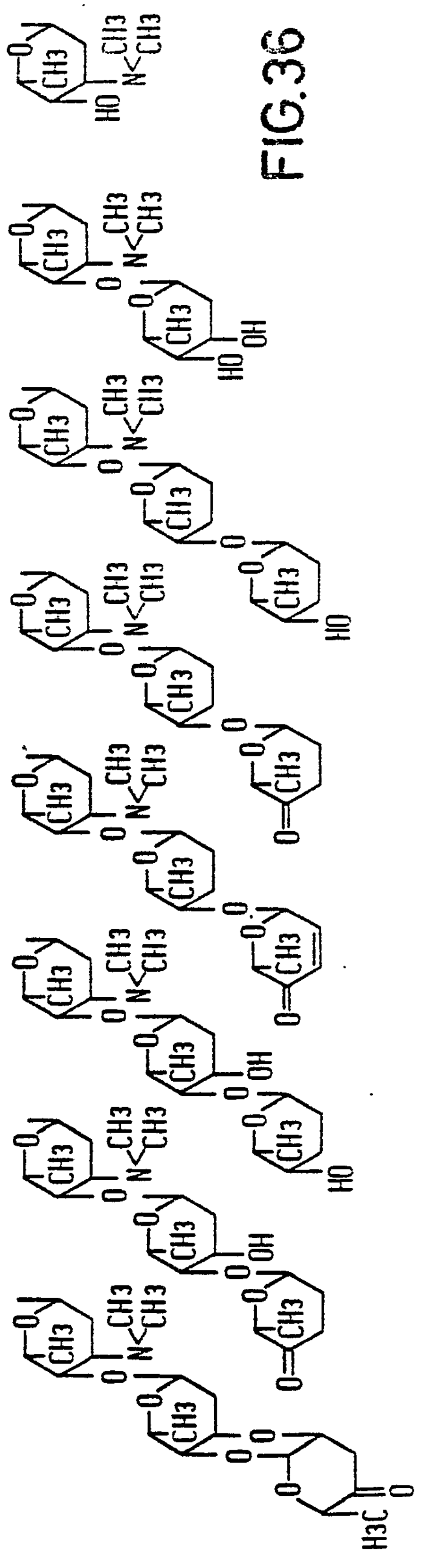

(I) (II) (III) (IV) (V) (VI) (VII) (VIII)